# EUROPEAN PATENT SPECIFICATION

(11) **EP 4 402 471 B1**
(45) Date of publication and mention of the grant of the patent: **25.06.2025**
(21) Application number: 22801292.8
(22) Date of filing: 16.09.2022
(51) Int. Cl.: C07K 1/107, G01N 33/532

(54) **A CHEMOSELECTIVE ELECTROCATALYTIC BIOCONJUGATION REACTION AND USES THEREOF**
CHEMOSELEKTIVE ELEKTROKATALYTISCHE BIOKONJUGATIONSREAKTION UND VERWENDUNGEN DAVON
RÉACTION DE BIOCONJUGAISON ÉLECTROCATALYTIQUE CHIMIOSÉLECTIVE ET SES UTILISATIONS

(30) Priority: 17.09.2021 US 202163245369 P
(43) Date of publication of application: 24.07.2024
(73) Proprietor: Trustees of Boston College, Chestnut Hill, MA 02467 (US)
(72) Inventor: CHATTERJEE, Abhishek, Lexington, Massachusetts 02420 (US); LOYND, Conor, Somerville, Massachusetts 02145 (US); ROY, Soumya Jyoti Singha, Brighton, Massachusetts 02135 (US)
(74) Representative: Marks & Clerk LLP
(86) International application number: PCT/US2022/076536
(87) International publication number: WO 2023/044413

(56) References cited:
- WO-A2-2021/072129
- US-A1- 2017 349 891
- US-A1- 2018 360 984
- SARATHI?ADDY PARTHA ET AL: "An Oxidative Bioconjugation Strategy Targeted to a Genetically Encoded 5-Hydroxytryptophan", CHEMBIOCHEM, vol. 19, no. 13, 1 June 2018 (2018-06-01), pages 1375 - 1378, XP093007729, ISSN: 1439-4227, Retrieved from the Internet <URL:https://api.wiley.com/onlinelibrary/tdm/v1/articles/10.1002%2Fcbic.201800111> DOI: 10.1002/cbic.201800111
- ADDY PARTHA SARATHI ET AL: "Supporting Information An Oxidative Bioconjugation Strategy Targeted to a Genetically Encoded 5-Hydroxytryptophan", CHEMBIOCHEM, vol. 19, 1 June 2018 (2018-06-01), pages 1 - 8, XP093007754, Retrieved from the Internet <URL:https://chemistry-europe.onlinelibrary.wiley.com/action/downloadSupplement?doi=10.1002%2Fcbic.201800111&file=cbic201800111-sup-0001-misc_information.pdf>
- TOYAMA EISHO ET AL: "Electrochemical Tryptophan-Selective Bioconjugation", CHEMRXIV, vol. 2019, 5 March 2019 (2019-03-05), pages 1 - 12, XP093007533, DOI: 10.26434/chemrxiv.7795484.v1
- SATO ET AL: "Protein Chemical Labeling Using Biomimetic Radical Chemistry", MOLECULES, vol. 24, no. 21, 3 November 2019 (2019-11-03), pages 3980, XP055711297, DOI: 10.3390/molecules24213980
- YOHEI SEKI ET AL: "Transition Metal-Free Tryptophan-Selective Bioconjugation of Proteins", JOURNAL OF THE AMERICAN CHEMICAL SOCIETY, vol. 138, no. 34, 31 August 2016 (2016-08-31), pages 10798 - 10801, XP055420898, ISSN: 0002-7863, DOI: 10.1021/jacs.6b06692
- ITALIA JAMES S ET AL: "An orthogonalized platform for genetic code expansion in both bacteria and eukaryotes", NATURE CHEMICAL BIOLOGY, vol. 13, no. 4, 13 February 2017 (2017-02-13), New York, pages 446 - 450, XP093007015, ISSN: 1552-4450, Retrieved from the Internet <URL:http://www.nature.com/articles/nchembio.2312> DOI: 10.1038/nchembio.2312

## Description

### FIELD OF THE INVENTION

The present invention is directed to a novel bioconjugation strategy using an electrochemical chemoselective bioconjugation reaction.

### BACKGROUND OF THE INVENTION

Electrochemistry has long played an important role in preparative chemical processes, with some early applications dating back to the 19^{th} century.¹⁻² It offers many unique advantages, including exquisite control over the fundamental parameters driving reaction outcome through facile manipulation of electrode potential, mild reaction conditions, innate scalability, and the ability to generate reactive intermediates *in situ*.³⁻⁶

The ability to chemoselectively modify proteins at predefined sites is a powerful technology to understand and engineer protein function.⁷⁻¹⁸ In order to achieve this goal, uniquely reactive unnatural chemical groups are incorporated into biomolecules through synthetic or biosynthetic routes, followed by their selective labeling using a bioorthogonal chemical reaction. Many such bioorthogonal reactions have been developed to date, including the conjugation of alkoxyamine/hydrazine to an aldehyde/ketone, the Staudinger ligation, the Cu(I)-catalyzed and strain promoted cycloaddition between alkynes and azides, the inverse electron demand Diels-Alder reactions between tetrazines and strained alkenes, etc. Many such functionalities can now be inserted into proteins site-specifically through the co-translational incorporation of genetically encoded non-canonical amitio acids (ncAAs). Such ncAA incorporation relies on engineered nonsense-suppressing aminoacyl-tRNA synthetase (aaRS)/tRNA pairs that selectively charge these into proteins expressed in living cells, in response to a repurposed nonsense codon. The incorporated ncAA handle can be subsequently used to precisely attach a variety of entities, from biophysical probes to therapeutic agents through the bioorthogonal conjugation reaction.

However, many of the available conjugation chemistries are limited by their slow kinetics, the need for toxic catalysts, scalability, cost of reagents, or a lack of compatibility with other chemistries to allow the simultaneous attachment of multiple distinct entities at different sites. There is continuing demand for new protein labeling methods that are efficient, use readily accessible reagents, provide excellent chemoselectivity, and are compatible with other well-established labeling strategies for introducing multiple, different modifications onto proteins in a controlled manner.¹⁹⁻²⁰

Specifically, there is a continued need for additional genetically encoded chemical functionalities that can be rapidly and chemoselectively labeled using catalyst-free conjugation reactions, and that are also compatible with other available bioorthogonal chemistries to allow concurrent labeling at multiple sites.

### SUMMARY OF THE INVENTION

The invention relates to methods of producing chemoselectively modified protein conjugates and electrochemically labeling of proteins as detailed in the appended claims.

Bioorthogonal chemical groups that enable selective labeling of complex biomolecules have become a cornerstone of modern chemical biology. Despite the exciting developments over the last two decades, the demand for new bioorthogonal reactions with unique abilities, and those compatible with existing chemistries for concurrent multisite-directed labeling, remains high. Electrochemistry has recently emerged as a powerful approach in small molecule synthesis owing to numerous attractive features, including precise control over fundamental reaction parameters, mild reaction conditions, and innate scalability. Even though these advantages also make it an attractive strategy for chemoselective modification of complex biomolecules such as proteins, such applications remain poorly developed.

Many of the unique advantages of electrochemistry, such as its tunability and mild reaction conditions, make it an attractive strategy for developing such chemoselective protein labeling methods.²¹ Indeed, recent reports have used electrochemistry to activate small-molecule labeling reagents - such as a phenothiazine radical,²² triazolinedioiones,²³ or luminols,²⁴⁻²⁵ generated through anodic oxidation - which label tyrosine residues on target proteins. Similarly, *in situ* generated oxoammonium reagents have also been used to label tryptophan residues. ²⁶⁻²⁷ Electrochemical strategies have also been employed to drive nitration²⁸ or iodination²⁹ of tyrosine residues on proteins. Although these examples corroborate the enormous untapped potential of using electrochemical strategies to develop protein labeling methods, their scope is restricted by the following limitations: Targeting canonical amino residues such as tyrosine and tryptophan intrinsically compromises the ability to precisely define the site and stoichiometry of protein labeling. Furthermore, reactive electrophilic reagents generated through such strategies may show off-target reactivity,³⁰⁻³¹ particularly in the context of proteins, where the reactivity of nucleophilic amino acid sidechains can be significantly enhanced by their microenvironment.

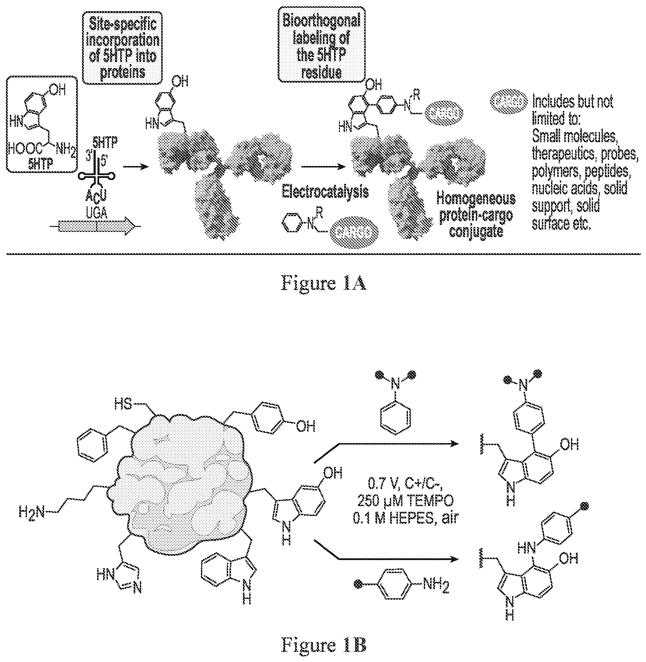

Described herein is a novel electrochemical strategy to label any recombinantly expressed protein at predefined sites (Figure 1A). The method is referred to herein as "eCLIC" and the basic mechanism is shown in Figure 1B. Unlike previous approaches that rely on electrochemically activating exogenous pro-electrophilic reagents, which are limited by suboptimal control over the site and stoichiometry of protein labeling (*vide supra*), eCLIC uses a two-pronged approach to establish exquisite site-control. First, the noncanonical amino acid (ncAA), i. e. 5-hydroxytryptophan (5HTP), which has a significantly lower oxidation potential relative to canonical aromatic amino acids, is introduced into a chosen site of the target protein through nonsense suppression using an engineered tryptophanyl-tRNA synthetase (EcTrpRS)/tRNA pair that we recently developed.³² Next, the electron-rich 5HTP residue is selectively oxidized at the anode to generate an electrophilic intermediate, which can be efficiently trapped using various aromatic amines to generate stable conjugates. This approach to chemoselectively convert the 5HTP residue into a reactive electrophile moiety enables its coupling with a nucleophilic group, such as aromatic amines, anilines, indoles and thiazoles, which are inherently unreactive toward proteins, making eCLIC highly chemoselective. The conditions for the eCLIC reaction were developed as described herein, and used to site-specifically modify several different proteins, including a full-length antibody, with excellent efficiency and selectivity.

It is also demonstrated herein that the eCLIC reaction is mutually compatible with the strain-promoted azide-alkyne cycloaddition (SPAAC),³⁴ as well as inverse-electron demand Diels-Alder (IEDDA) reaction between strained alkenes and tetrazines,^{16, 35} two of the most popular existing bioorthogonal conjugation reactions. Furthermore, as described herein, a protein containing both a 5HTP and an azide-ncAA residue at specific sites was expressed, thus demonstrating that eCLIC and SPAAC can be used sequentially to selectively label each side chain with a distinct cargo to generate a homogeneous dual-labeled conjugate. The eCLIC reaction is compatible with a broad range of nucleophilic groups, and in particular such as aromatic amine substrates, and these reagents, as well as 5HTP, are readily accessible and inexpensive. These advantages establish eCLIC as an attractive method for site-specific protein bioconjugation.

Specifically, the present invention involves the ability to: 1) chemoselectively functionalize a ncAA, i.e. 5HTP, a stable and inexpensive non-naturally occurring amino acid that can be site-specifically incorporated into proteins, using an electrochemical oxidative coupling reaction to many different coupling partners comprising a nucleophilic group (including aromatic amines) under physiological conditions, 2) systematically tune the coupling chemistry by rational substitution on the coupling partners comprising a nucleophilic group (such as aromatic amines), and 3) facilitate the reaction using additives such as TEMPO and HEPES that either facilitate electron transfer between protein and electrode or suppress unwanted side-reactions. The inexpensive nature of both 5HTP and its coupling partner (e.g., any nucleophilic molecule such as an aromatic amine), the intrinsically facile and scalable nature of electrochemical processes, and the compatibility of this reaction with established bioconjugation reactions, such as azide-alkyne or tetrazine-strained-alkene reactions, will make this bioconjugation strategy a useful new tool for industrial protein modification needs.

The technology of the present invention enables covalent attachment of any entity/molecule/moiety (drug, fluorophore, other biophysical probes, polyethylene glycol, other proteins, nucleic acids, a solid matrix like a solid surface), carrying a suitable reaction partner such as an aromatic amine, onto a specific site of any recombinant protein. The target protein can be expressed in any expression system (*E*. *coli*/eukaryotic cell) site-specifically incorporating the stable and inexpensive amino acid 5HTP. Reagents with suitable reaction partners, such as aromatic amine-containing molecules, can be coupled to this protein using anodic oxidation. Proteins encompassed by the present disclosure include, for example, a polypeptide, a peptide, an antibody, antibody fragment, viral protein, chemokine, cytokine, antigen, enzyme or growth factor.

Such conjugation reactions can be used to generate 1) antibody-drug conjugates, 2) antibody-fluorophore conjugates, 3) antibody-enzyme conjugates, 4) therapeutic protein-PEG conjugate, 5) protein-nucleic acid conjugates, 6) immobilize proteins on surfaces site-specifically, 6) generate kits for site-specific modification of proteins, etc. Synthesis of other biomolecules (e.g., DNA, RNA) with the 5-hydroxyindole will also allow their precise labeling through the electrochemical coupling reaction.

Thus, the present disclosure describes compositions of chemoselective, homogeneous protein-cargo conjugates, and specifically encompasses bioconjugates with biorthogonal labeling of 5-HTP residues genetically-engineered into the protein. Encompassed herein are bioconjugates comprising a biomolecule comprising one, or more unnatural amino acid residues incorporated in a site-specific manner into the biomolecule, wherein the one, or more, unnatural amino acid residues further comprise a biochemical or chemical cargo entity conjugated to the unnatural amino acid via a covalent electrochemical linkage. The bioconjugates comprise any suitable biomolecule, but specifically encompasses biomolecules selected from the group consisting of proteins, polypeptides, peptides or nucleic acids, including DNA or RNA nucleic acids.

Any suitable non-canonical amino acid (ncAA, also referred to herein as an unnatural amino acid residue) can be targeted for conjugation. In the present invention the unnatural amino acid is 5-hydroxytryptophan as incorporated into one, or more site-specific positions in the biomolecule.

The bioconjugates of the present disclosure are bioorthogonally-labeled with a suitable cargo entity. More specifically encompassed by the present disclosure are cargo entities selected from the group consisting of one, or more of the following: small molecule, therapeutic drug, detectable label, detectable probe, polymer such as PEG, peptide, or nucleic acid. In another aspect, the cargo entity comprises means (e.g., suitable chemical reaction partners or chemical linkages) to facilitate the immobilization of the bioconjugate to a suitable solid support such as a surface or bead, such as a polymer support or a glass bead.

Further encompassed by the present disclosure are methods of producing a bioconjugate comprising a biomolecule comprising one, or more unnatural amino acid residues incorporated in a site-specific manner into the biomolecule, wherein the one, or more, unnatural amino acid residues further comprise a biochemical or chemical cargo entity conjugated to the unnatural amino acid via a covalent electrochemical linkage.

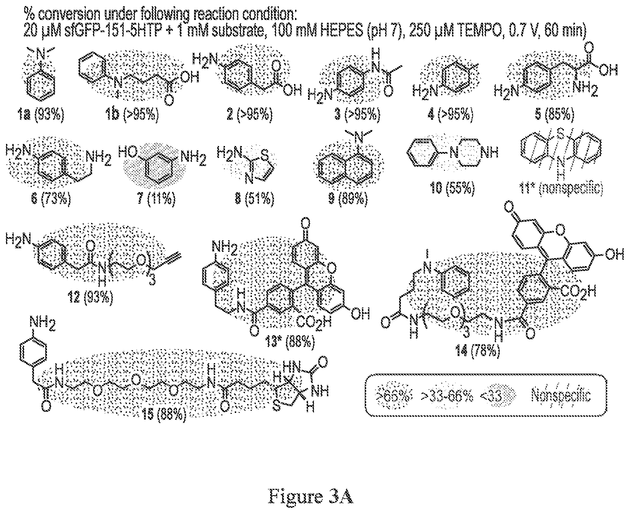

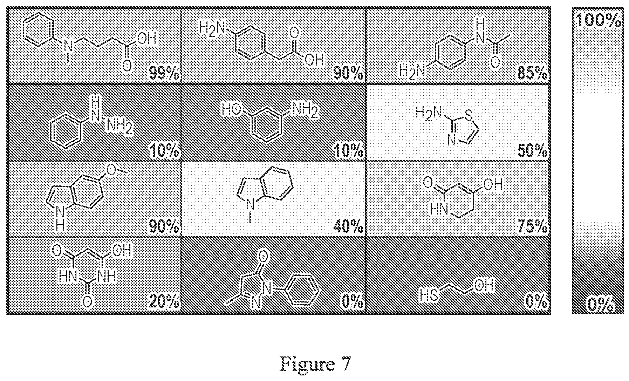

Specifically encompassed in the present invention are methods of producing a chemoselectively modified protein bioconjugate, comprising the steps of providing a recombinantly-expressed protein comprising one, or more non-canonical amino acid (ncAA) residues at specific sites of the protein, wherein one, or more. of the ncAA residues is a 5-hydroxytryptophan (5-HTPP); selectively oxidizing the one, or more, 5-HTP amino acid residues of the protein under suitable conditions and at low voltage, wherein the voltage is sufficient to generate an electrophilic 5-HTP intermediate; and reacting the 5-HTP intermediate with a coupling partner comprising a chemical moiety comprising a nucleophilic group such as shown in FIG. 3A or 7, under conditions suitable for the coupling of the nucleophilic group to the one, or more, 5-HTP residues in the protein, thereby producing a chemoselectively modified protein bioconjugate. As described herein typically the ncAA residues to be modified are exposed on the surface of the protein. In one embodiment of the present invention the protein is an antibody or an antibody fragment such as an Fab fragment.

The method uses an electrochemical reaction that results in a covalent linkage/bond between the targeted unnatural amino acid reside of the biomolecule and the cargo entity. More specifically, the electrochemical reaction is an electrocatalysis reaction. The electrocatalysis reaction comprises one, or more reaction partners (i.e., a nucleophilic group or a moiety comprising a nucleophilic group) selected from the group consisting of: aromatic amines, anilines, indoles, and thiazoles that link the cargo entity to the unnatural amino acid of the biomolecule.

The electron-rich 5-HTP has a significantly lower oxidation potential that canonical/naturally-occurring amino acids wherein oxidation voltage is between about 0.4 volts and about 1.0 volts. In particular, the oxidation voltage is between about 0.4 volts and about 0.8 volts; and in one embodiment the oxidation voltage is between about 0.7 and 0.8 volts. Importantly, under suitable oxidation conditions (e.g., mild oxidation conditions) as described herein, only the 5-HTP is oxidized, and in turn, only the 5-HTP residues of the protein react with aromatic amines to conjugate the cargo of interest-none of the other amino acid residues are reactive.

In some embodiments of the present invention, the oxidation step comprises the addition of an electron transfer mediator, such as 2,2,6,6-tetramethylpiperidine-1-oxyl (TEMPO). Additionally, as described herein, a suitable buffer for the oxidation reaction is HEPES.

The methods described herein couple an aromatic amine group to the 5-HTP residue. The aromatic amines can be any of the following: anilines, indoles, and thiazoles, and particularly suitable aromatic amines are shown in shown in FIG. 3A and FIG. 7.

Also as described herein, the aromatic amine group can further comprise a biochemical of chemical cargo entity. In particular, the cargo entity is selected from the group consisting of one, or more of the following: small molecule, therapeutic drug, detectable label, detectable probe, polymer such as PEG, peptide, or nucleic acid. In one embodiment, the cargo entity comprises means to immobilize the bioconjugate to a solid support.

In some embodiments of the present invention, the 5-HTP further comprises a bulky protecting group, such as a tert-Butoxycarbonyl (Boc) protecting group.

In one aspect, the one, or more, non-canonical amino acid residues is an azide ncAA such as a cyclopropane-lysine amino acid (CpK) or an azido-lysine amino acid (CpK) or an azido-lysine amino acid (AzK) and the method further comprises coupling the azide ncAA residue with a coupling partner under conditions suitable for the coupling of the coupling partner to the azide ncAA, thereby producing a chemoselectively modified protein bioconjugate modified at two specific sites of the protein. In some embodiments the coupling partner further comprises a biochemical or chemical cargo entity as described herein.

The electrochemical conjugation chemistry of the present inventions is compatible with existing bioconjugation strategies, such as azide-alkyne cycloaddition and tetrazine-strained alkene reactions and can be used together to simultaneously attach multiple different entities onto distinct sites of a target protein. Technology to site-specifically incorporate 5HTP as well as another non-canonical amino acid (ncAA, also referred to herein as an unnatural amino acid, or non-naturally occurring amino acid) incorporating azide/cyclopropene into any two sites of a target protein has already been developed and shown that the two sites can be concurrently labeled with two distinct entities (e.g., two different fluorophores or therapeutic agents).

The 5HTP-incorporating aminoacyl-tRNA synthetase/tRNA can also be used to substitute all tryptophan residues with 5HTP (partially) within the proteome of any cell. Subsequent chemoselective labeling of these 5HTP-residues using suitable reagents, such as aromatic amines, will enable their facile identification (e.g., through fluorophore labeling)/isolation (e.g., through biotin labeling). This would provide a unique way to identify newly synthesized proteins in a cell.

### BRIEF DESCRIPTION OF THE DRAWINGS

FIG. 1A-B. FIG 1A shows the scheme for chemoselective conjugation of a wide range of cargo onto a 5HTP residue, incorporated site-specifically into a protein, using an electrochemical oxidative coupling reaction. FIG. 1B shows the mechanism of eCLIC that directly oxidizes a site-specifically incorporated 5HTP residue (structure of the sidechain shown in red), which then reacts with aromatic amines to generate stable conjugates.

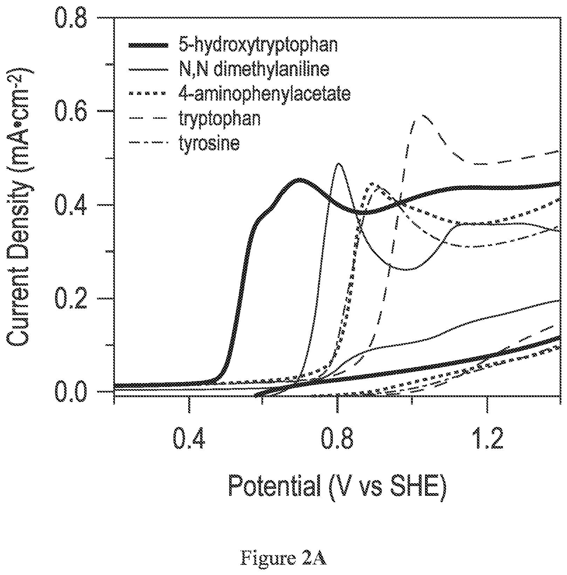

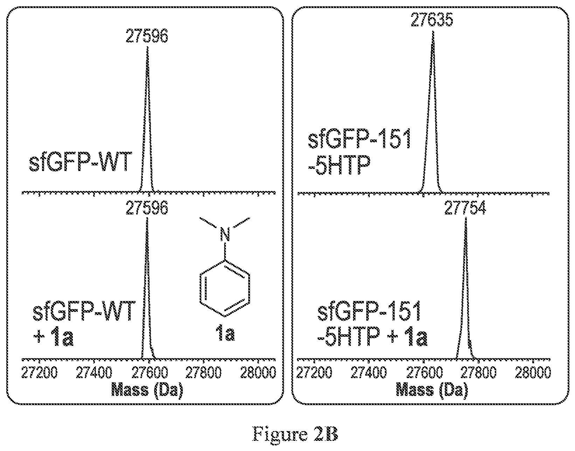

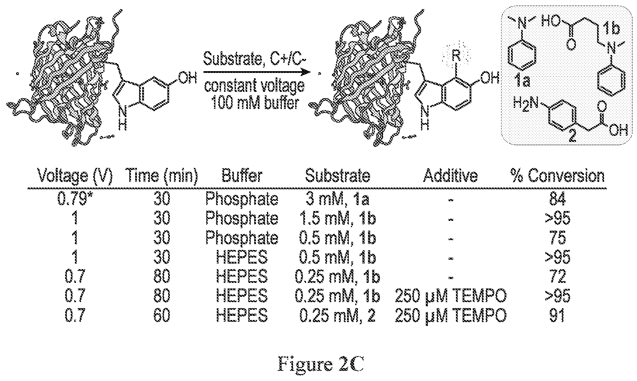

FIG. 2A-C depict the optimization of the eCLIC reaction. FIG. 2A. CV analysis shows 5HTP is oxidized at a significantly lower potential relative to other canonical aromatic amino acids such as tyrosine and tryptophan, as well as the nucleophilic coupling partners used for eCLIC conjugation. (*vs SHE; all others are ElectraSyn cell voltages). FIG. 2B. N,N-dimethylaniline **1a** can be electrochemically attached to sfGFP-151-5HTP to generate a single homogeneous conjugate, whereas the corresponding wild-type sfGFP protein does not react under identical condition. FIG. 2C. Optimization of eCLIC reaction revealed beneficial effect of HEPES buffer and the inclusion of TEMPO.

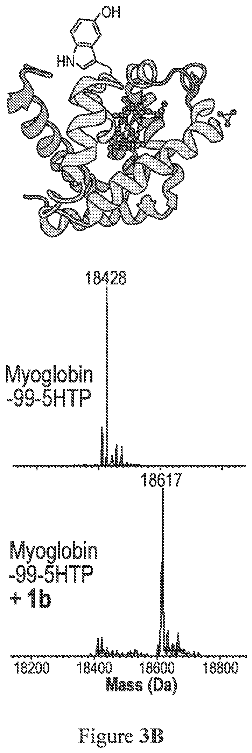

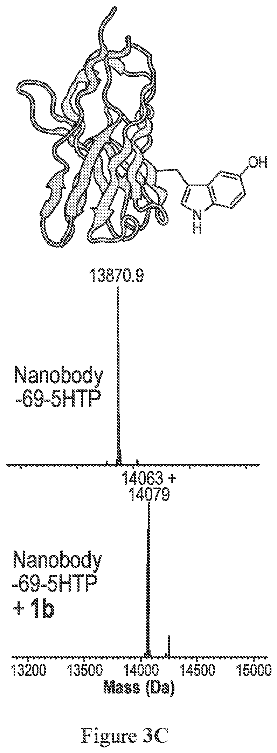

FIG. 3A-C shows the scope of the electrochemical labeling reaction (eCLIC) targeting ncAAs with chemoselectivity. FIG. 3A. Various aromatic nucleophilic substrates that would successfully participate in the eCLIC reaction were used at 0.5 mM. Except for phenothiazine, which is known to react with tyrosine residues, none of the other substrates labeled wild-type sfGFP lacking the 5HTP residue Myoglobin-99-5HTP (FIG. 3B) and anti-HER2 nanobody-69-5HTP (FIG. 3C) can also be efficiently labeled using the optimized eCLIC condition. The corresponding wild-type proteins, lacking the 5HTP residue, show no labeling under identical conditions.

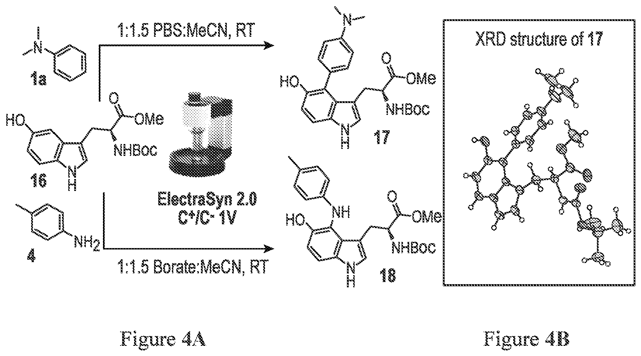

FIG. 4A-B. Structural characterization of the eCLIC product. FIG. 4A. A boc-protected 5HTP (**17**) enabled the suppression of the interfering homocoupling reaction and formation of significant quantities of the aniline coupled product. These products were characterized by NMR and MS analysis. The coupling product (**18**) with N,N-dimethylaniline (**1a**) was further characterized using X-ray crystallography as shown (FIG. 4B).

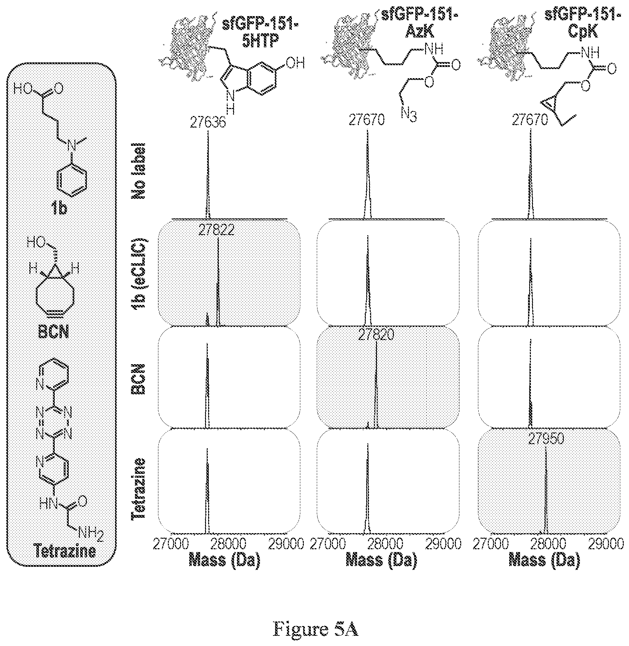

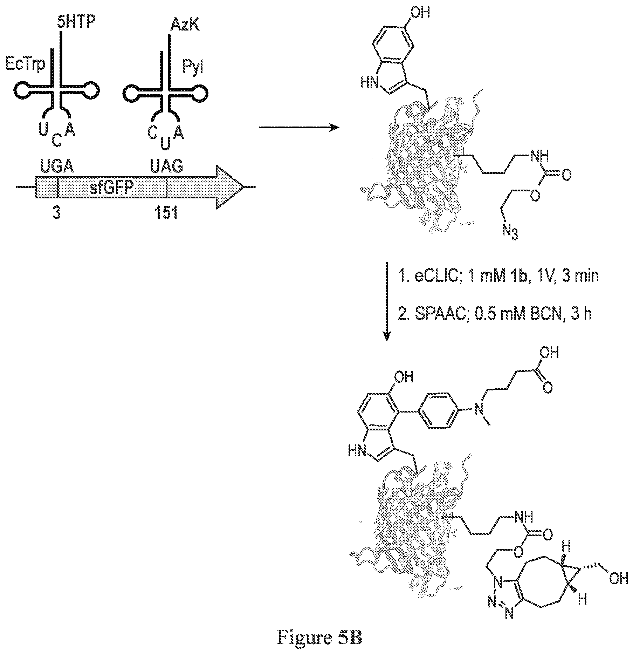

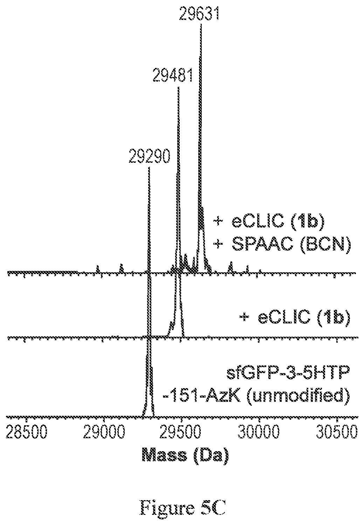

FIG. 5A-C. The eCLIC reaction is compatible with SPAAC and IEDDA. FIG. 5A shows sfGFP reporters harboring either 5HTP, AzK, or CpK (the structure of each amino acid sidechain is shown above the panel) that were separately subjected to eCLIC conditions (with **1b**), BCN, or tetrazine, which shows that each reagent selectively labels its cognate substrate, but not the others. FIG. 5B. A sfGFP reporter harboring a 5HTP at site 3 and an AzK at site 151 was expressed using engineered EcTrp and pyrrolysyl tRNA/synthetase pairs, respectively. Sequentially subjecting this protein to eCLIC (with **1b**) and SPAAC (with BCN) resulted in efficient site-specific dual-labeling. The resulting protein was characterized by mass spec. (FIG. 5C).

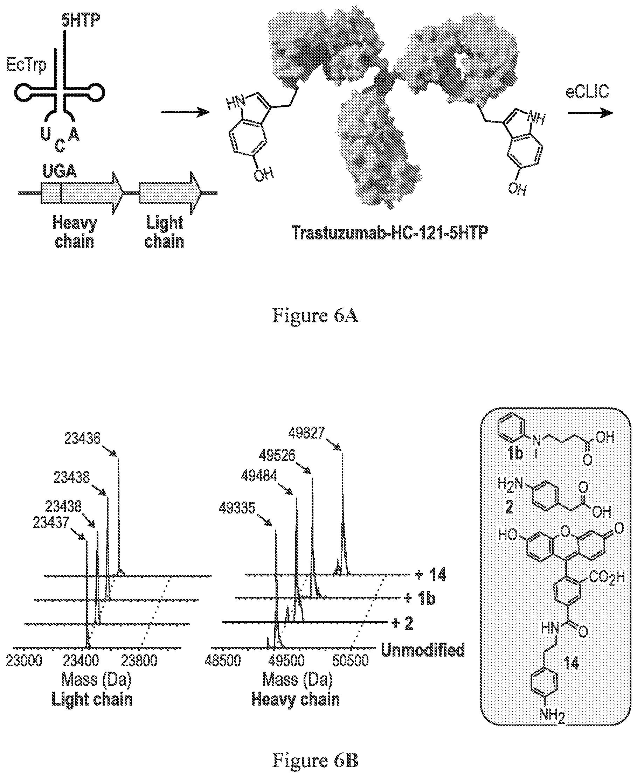

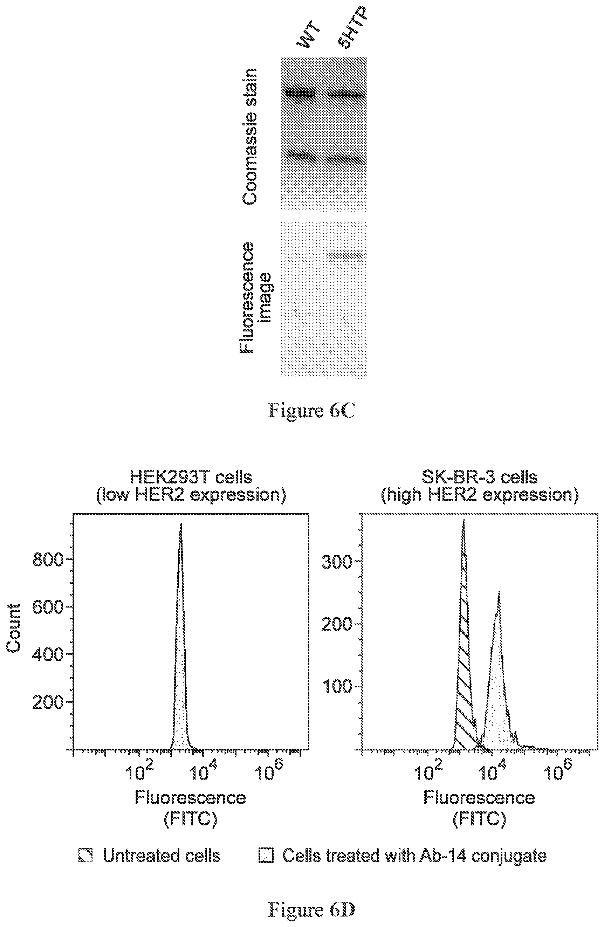

FIG. 6A-D. The eCLIC reaction can be used to generate functional conjugates of a full-length antibody. FIG. 6A. Using an engineered EcTrpRS/tRNA pair, 5HTP was site-specifically incorporated into the 121 site of the heavy chain of Trastuzumab. FIG. 6B. Subjecting Trastuzumab-HC-121-5HTP to eCLIC reaction using three different coupling partners (**1b, 2, 14**) led to efficient labeling of the heavy chain in each case, while the light chain remained unmodified, as shown by MS analysis. **c**FIG. 6C. Analysis of the eCLIC labeling reaction of wild-type Trastuzumab and Trastuzumab-HC-121-5HTP with the fluorescent substrate **14** by SDS-PAGE followed by fluorescence imaging revealed selective labeling of the 5HTP-containing heavy chain. FIG. 6D. Incubating HER2-expressing SK-BR-3 cells with **14**-labeled Trastuzumab (prepared by eCLIC) results in efficient cell staining, as shown by FACS analysis, while it failed to stain HERZ-negative HEK293T cells.
FIG. 7 shows the substrate scope for the 5HTP-targeted electrochemical conjugation reaction. Each of the potential coupling partners were reacted with GFP-5HTP under electrochemical coupling condition and the extent of conversion in each case, as observed by MS analysis is shown in each case.

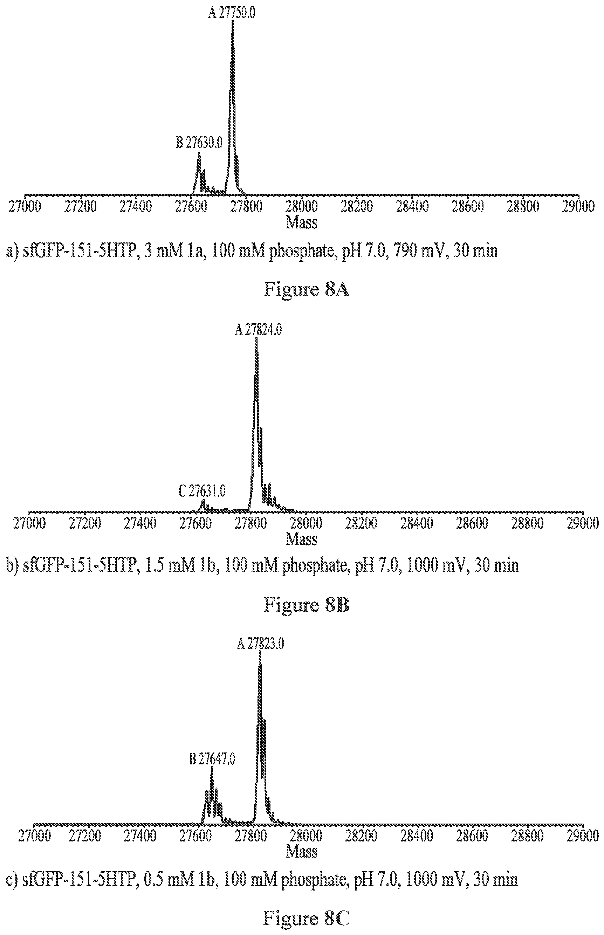

FIG. 8A-G. MS analysis of eCLIC reactions associated with FIG. 2C. Expected molecular weights: sfGFP-151-5HTP (27636 Da), conjugate with **1a** (27755 Da), conjugate with 1b (27827 Da), conjugate with 2 (27785 Da).

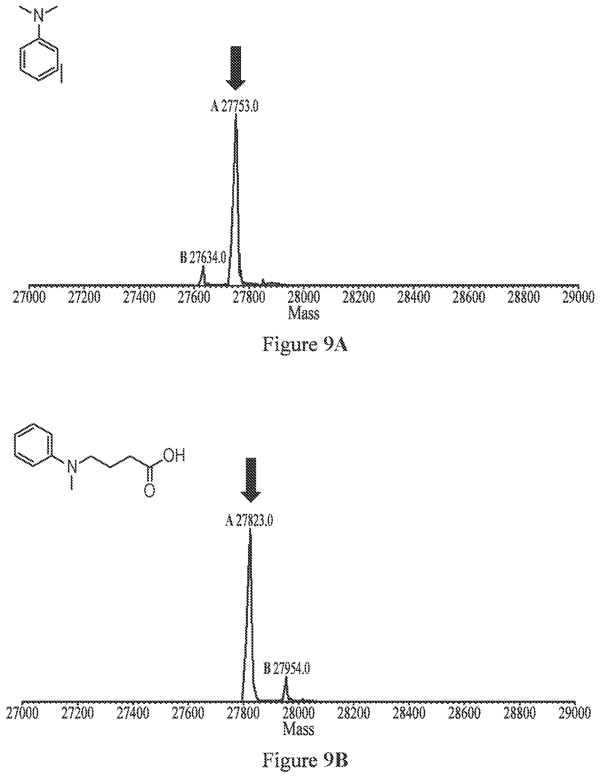

FIG. 9A-P. MS analysis of eCLIC reactions of sfGFP-151-5HTP) with various substrates associated with FIG. 3A. Expected molecular weight of unmodified sfGFP-151-5HTP is 27636 Da. Expected eCLIC adduct is highlighted with a green arrow in each case.

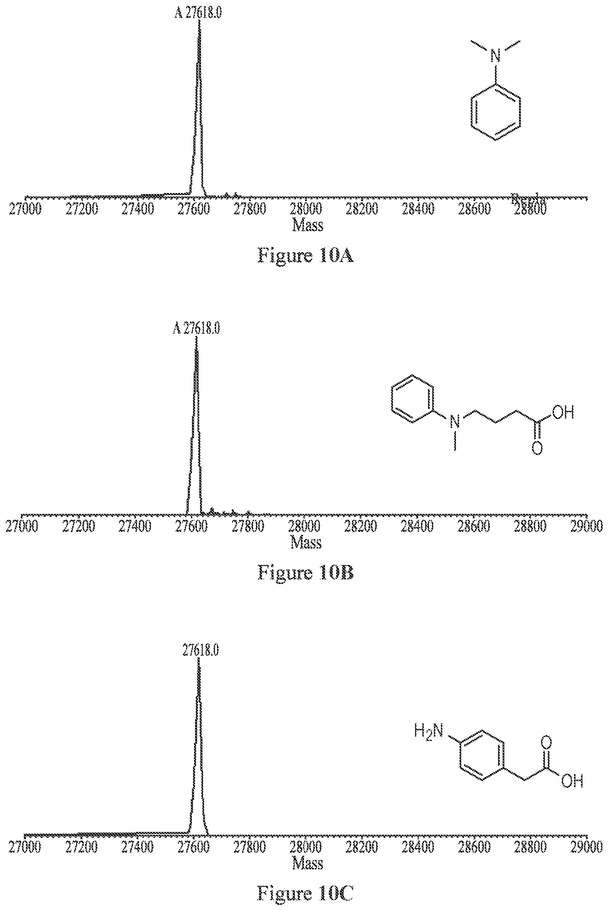

FIG. 10A-E. Representative examples of MS analysis of eCLIC reactions of wild-type sfGFP with various substrates shown in FIG. 3. None of the substrates show detectable labeling, except for phenothiazine (**11**), which is known to label tyrosine residues. Expected molecular weight of unmodified wild-type sfGFP is 27618 Da.

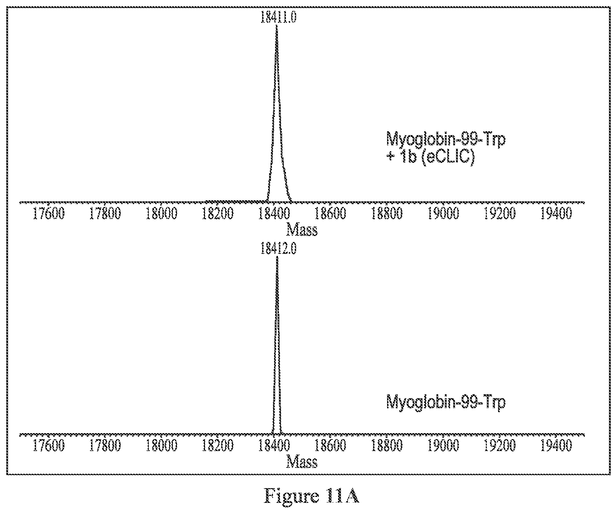

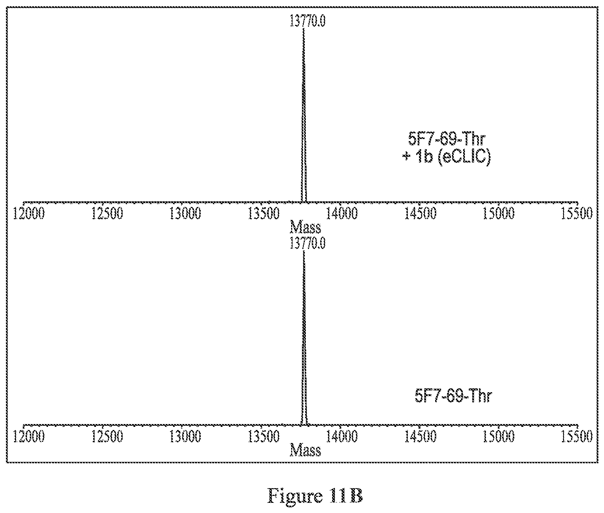

FIG. 11A-B. Myoglobin and 5F7 anti-HER2 nanobody control proteins lacking a 5HTP residue do not undergo eCLIC labeling with the 1b aniline, even though the 5HTP mutants of these proteins can be efficiently labeled (FIG. 3B-C).

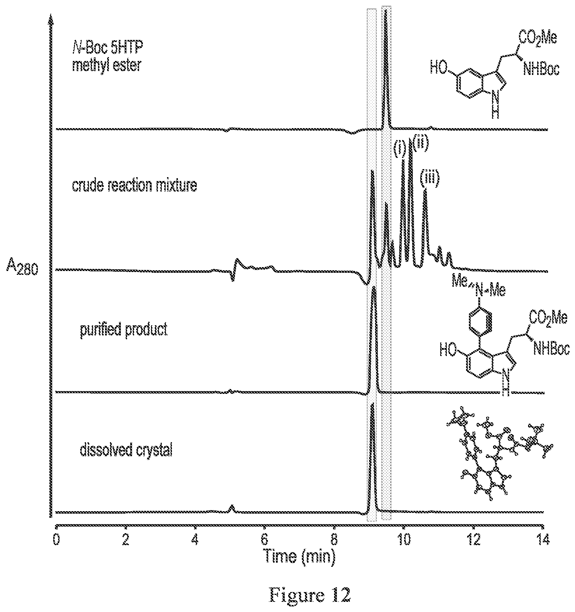

FIG. 12. HPLC-MS analysis of the eCLIC reaction between N-Boc methyl ester of 5-hydroxytryptophan **17.** HPLC-MS analysis of the eCLIC reaction between N-Boc methyl ester of 5-hydroxytryptophan **17** (exact mass: 334.15) and N,N-dimethylaniline (exact mass: 121.09); A₂₈₀ represents absorbance at 280 nm in an arbitrary unit; N,N-dimethylaniline has little absorbance at 280 nm (elutes around 4.5 min); there was a single peak having the expected product mass (at 9.3 min, see below for the mass spec) in the crude reaction mixture, other peaks correspond to unreacted starting material (5HTP derivative), higher order homo- and hetero-coupling reactions between the starting materials having following m/z (protonated forms): peak (i) 786.35 (2A+B-4+H⁺), peak (ii) 1118.48 (3A+B-6+H⁺ peak (iii) 667.28 (2A-2+H⁺) etc. [A and B refers to **17** and **1a,** respectively]; expected product (A+B-2) having m/z 454.21 was structurally characterized by mass spectrometry, NMR and XRD analysis; dissolved crystal refers to a solution of the crystal of the product, the crystal having been retrieved from the same sample pool from which diffraction quality single crystal was picked for XRD structure analysis

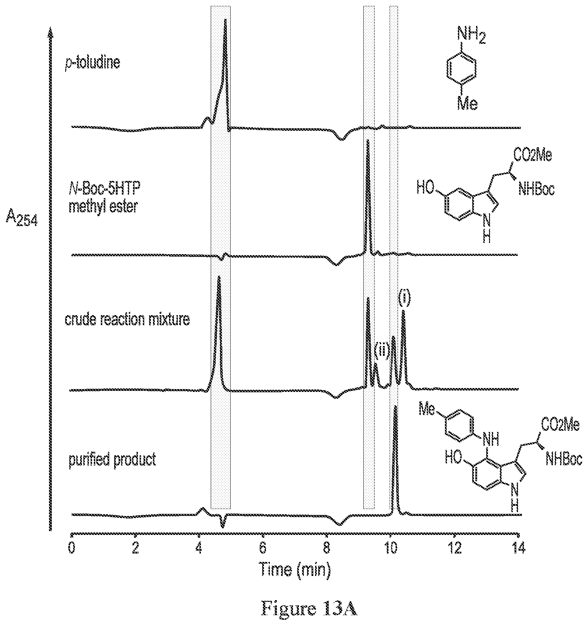

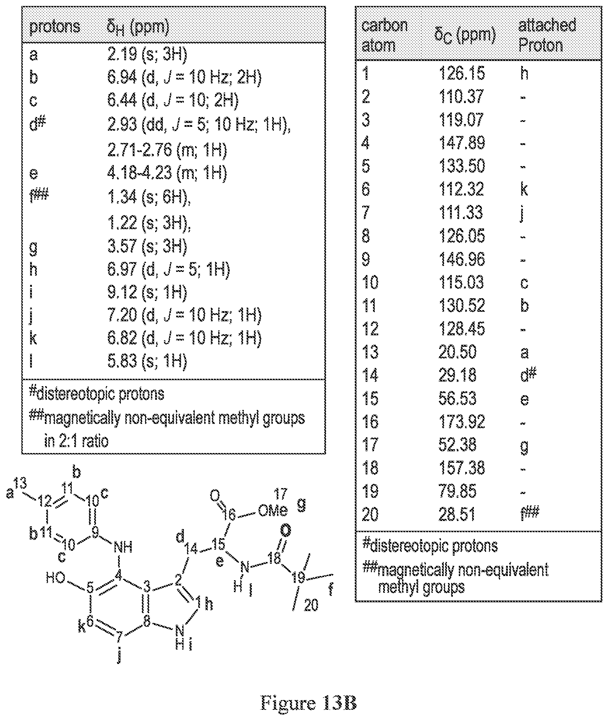

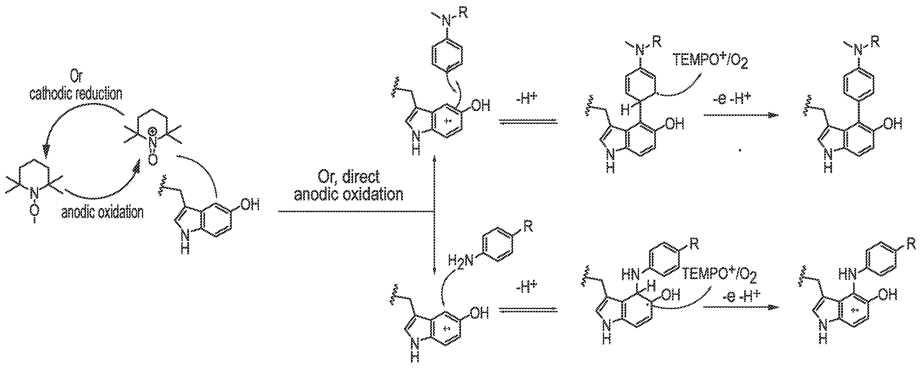

FIG. 13A-B. HPLC-MS analysis of the eCLIC reaction modelling between *N-*Boc methyl ester of 5-hydroxytryptophan **17.** HPLC-MS analysis of the eCLIC reaction modelling between *N*-Boc methyl ester of 5-hydroxytryptophan **1**7 (exact mass: 334.15) and *p*-toludine **4** (exact mass: 107.15); A₂₅₄ represents absorbance at 254 nm in an arbitrary unit; expected product (A+C-2) having m/z 440.21 (protonated form) was structurally characterized by mass spectrometry and co-relation NMR analysis; peak (i) had m/z (protonated form) of 438.21 (A+C-4+H⁺) is likely generated by over-oxidation of the expected product (A+C-2); peak (ii) had m/z (protonated forms) of 454.20 (A+C-4+16+H⁺) is suggestive of a further oxidized product, [A and C refers to the **17** and **4,** respectively] **(B)** chemical shifts of ¹H and ¹³C assigned through 1-D and 2-D NMR analysis (*vide infra*); carbons are numbered numerically and protons are designated alphabetically.
FIG. 14. A proposed mechanism of eCLIC.

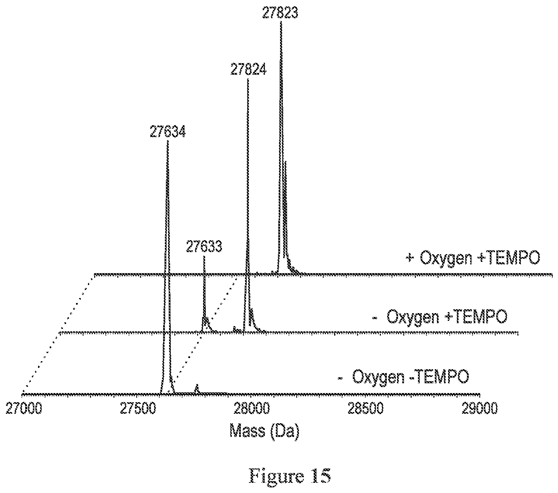

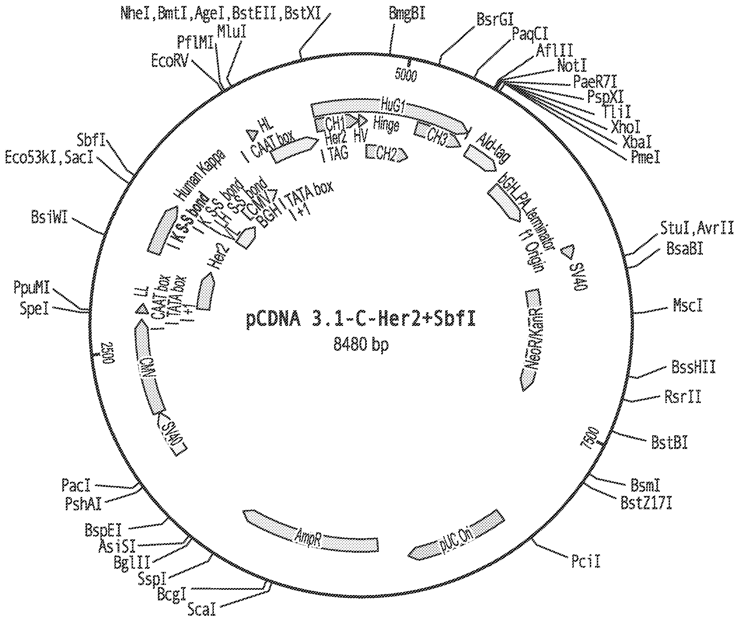

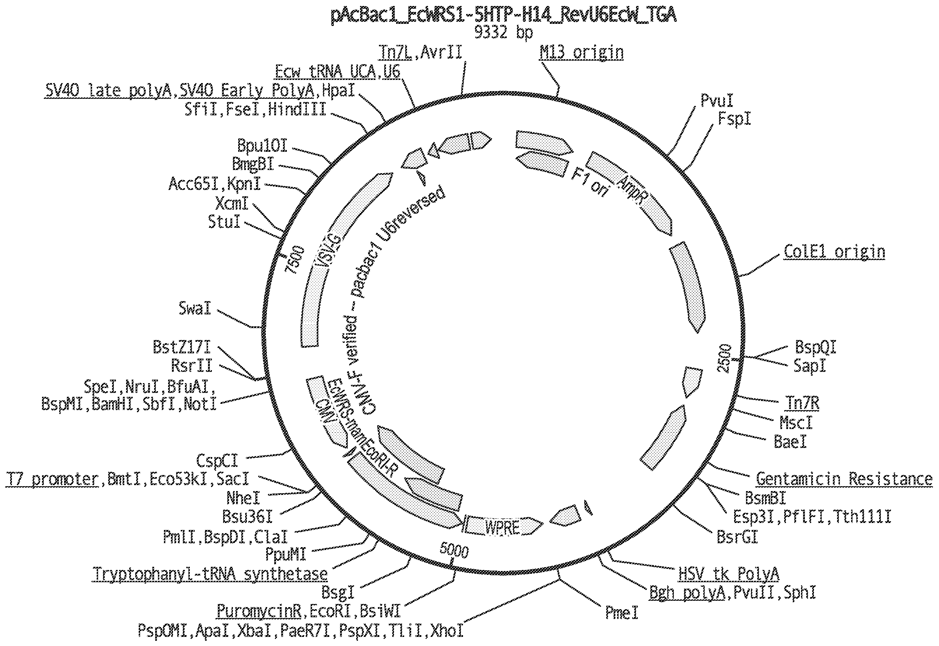

FIG. 15. The interchangeable roles of TEMPO and oxygen in the eCLIC reaction. sfGFP-151-5HTP was reacted with **1b** in 100 mM HEPES, pH 7.0 at 700 mV for 1 hour under oxygen-depleted conditions in the absence of TEMPO, oxygen-depleted conditions in the absence of TEMPO (250 µM), and optimized eCLIC conditions (aerobic + TEMPO). Expected molecular weight for sfGFP-151-TAG is 27636 Da, and its conjugation product with **1b** is 27827 Da.
FIG. 16. Plasma map of pCDNA3.1-C-Her2+SbfI (containing Trastuzumab light chain and heavy chain fragments). Sequences of the plasmid are found in SEQ ID. NO:1.
FIG. 17. Plasma map of pAcBac1_EcWRS1-5HTP-H14_RevU6EcW_TGA, Sequences of the plasmid are found in SEQ ID NO:2.

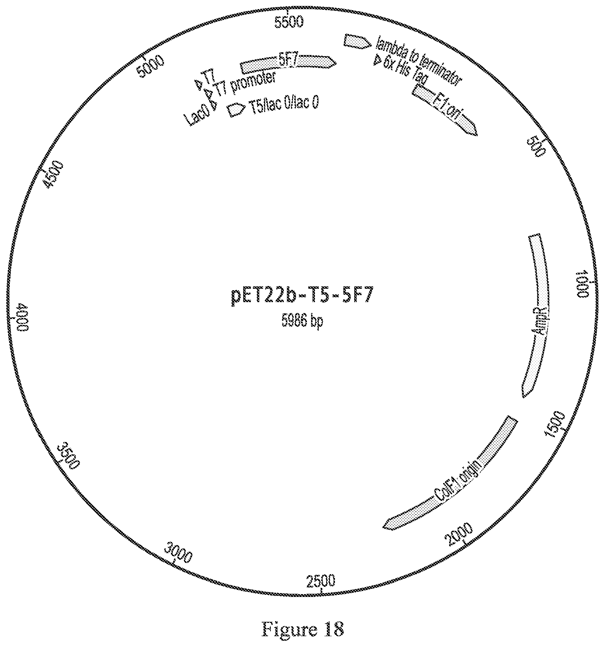

FIG. 18. Plasmid map of pET22b-T5-5F7. Sequences of the plasmid are found in SEQ ID NO:3.

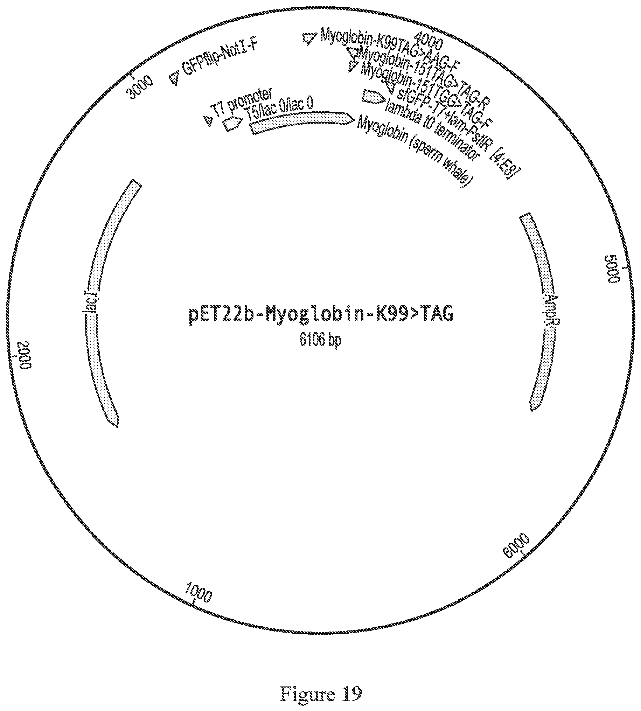

FIG. 19. Plasmid map of pET22b-Myoglobin-K99>TAG. Sequences of the plasmid are found in SEQ ID NO:4.

### DETAILED DESCRIPTION OF THE PREFERRED EMBODIMENTS

As used herein, the term "and/or" includes any and all combinations of one or more of the associated listed items. Also, all conjunctions used are to be understood in the inost inclusive sense possible. Thus, the word "or" should be understood as having the definition of a logical "or" rather than that of a logical "exclusive or" unless the context clearly necessitates otherwise. Further, the singular forms and the articles "a", "an" and "the" are intended to include the plural forms as well, unless expressly stated otherwise. It will be further understood that the terms: includes, comprises, including and/or comprising, when used in this specification, specify the presence of stated features, integers, steps, operations, elements, and/or components, but do not preclude the presence or addition of one or more other features, integers, steps, operations, elements, components, and/or groups thereof. Further, it will be understood that when an element, including component or subsystem, is referred to and/or shown as being connected or coupled to another element, it can be directly connected or coupled to the other element or intervening elements may be present.

It will be understood that although terms such as "first" and "second" are used herein to describe various elements, these elements should not be limited by these terms. These terms are only used to distinguish one element from another element. Thus, an element discussed below could be termed a second element, and similarly, a second element may be termed a first element without departing from the teachings of the present invention.

Unless otherwise defined, all terms (including technical and scientific terms) used herein have the same meaning as commonly understood by one of ordinary skill in the art to which this invention belongs. It will be further understood that terms, such as those defined in commonly used dictionaries, should be interpreted as having a meaning that is consistent with their meaning in the context of the relevant art and will not be interpreted in an idealized or overly formal sense unless expressly so defined herein.

As described herein, the present invention establishes a general strategy to label recombinant proteins using 5HTP-directed electrochemical conjugation reaction. The ability to 1) site-specifically incorporate 5HTP, a stable and accessible ncAA, into any protein of interest expressed in both *E. coli* and eukaryotic cells, 2) chemoselectively functionalize this non-natural residue with readily available coupling partners such as aromatic amines, 3) systematically tune the coupling chemistry by rational substitution on the aromatic amine and other coupling partners, and 4) the innate scalability and other advantages of electrochemical reaction will make this bioconjugation strategy a useful new tool in chemical biology.

The genetic code expansion (GCE) technology enables site-specific incorporation of noncanonical amino acids (ticAAs) using engineered aminoacyl-tRNA synthetase/tRNA pairs.³⁶⁻³⁸ This technology provides an attractive way to precisely introduce a unique chemical functionality into predefined sites of proteins, which can be subsequently activated using electrochemistry with high chemoselectivity for conjugation with suitable bioorthogonal reaction partners. The *E. coli* tryptophanyl-tRNA synthetase (EcTrpRS)/tRNA^{EcTrp} pair was engineered for the site-specific incorporation of 5-hydroxytryptophan (5HTP) into proteins expressed in both *E. coli* and eukaryotic cells with excellent fidelity and efficiency.^{32, 39-43} The electron-rich 5-hydroxyindole functionality is otherwise stable, but under mild oxidative conditions, it is known to readily undergo coupling with itself or other electron-rich aromatic compounds.⁴³⁻⁴⁷ The ability to selectively trigger an electrochemical oxidation of the 5HTP residue on a protein provides a plausible path for chemoselective protein labeling. The behavior of 5HTP was analyzed using cyclic voltammetry (CV) relative to other redox-active aromatic amino acids such as tyrosine and tryptophan (FIG.2A). 5HTP was found to oxidize at a significantly lower voltage than canonical aromatic amino acids, revealing room for its selective oxidative modification.

Using the engineered EcTrpRS/tRNA^{EcTrp} pair, a 5HTP residue was incorporated at the surface-exposed 151 site of superfolder GFP (sfGFP), and the resulting protein was used to explore if the unique 5-hydroxyindole functionality could be chemoselectively modified using electrochemistry. An otherwise identical wild-type sfGFP protein, with a tyrosine residue at the 151 site instead of 5HTP, was used as a control. A variety of reaction conditions and potential coupling partners were screened using an IKA ElectraSyn 2.0 Microvial instrument to find that sfGFP-151-5HTP forms an adduct with N,N-dimethylaniline **la,** as observed by whole-protein mass-spectrometry (FIG. 2B). Using graphite electrodes at a controlled potential (0.79 V), stirring 20 µM sfGFP-151-5HTP and 10 mM N,N-dimethylaniline **1a** in 100 mM phosphate buffer resulted in complete protein modification within 30 minutes (FIG. 2B). Treating the wild-type sfGFP protein - which harbors all twenty canonical amino acids - under the same condition resulted in no detectable protein labeling, which confirms the chemoselectivity of the eCLIC reaction.

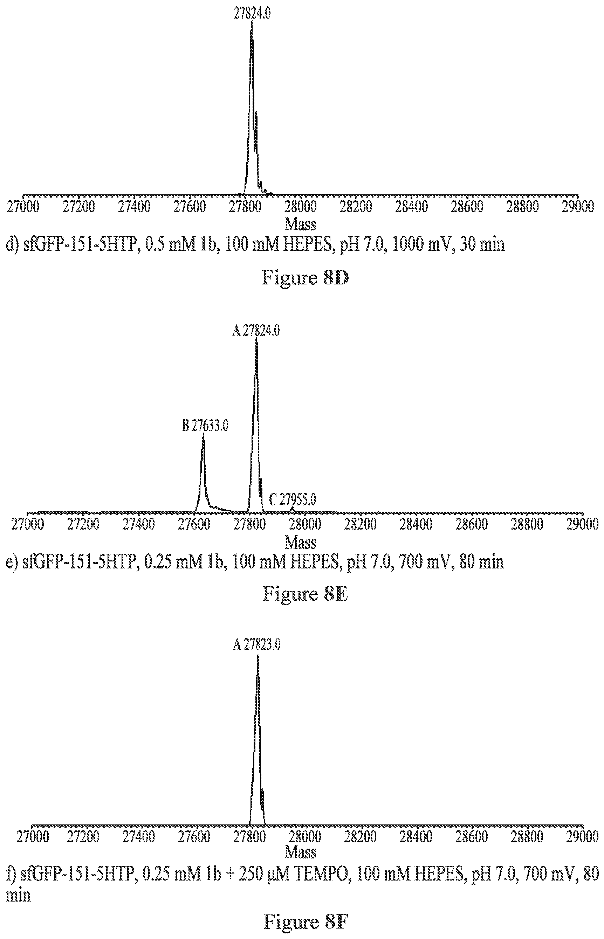

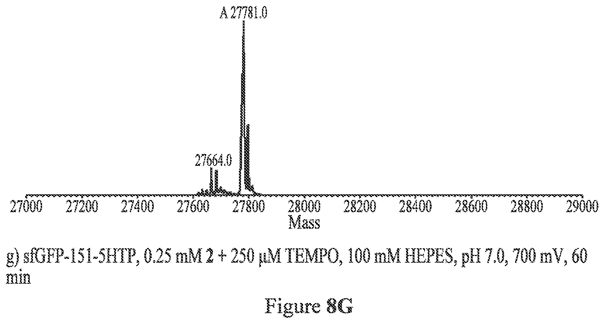

Next, the eCLIC reaction conditions were optimized in an attempt to reduce the concentration of the aniline substrate needed for efficient conjugation. These optimizations were performed using a synthetic dialkylaniline **1b** (FIG. 2C; see also FIG. 8A-G), which has a carboxylic acid group that improves solubility in aqueous buffers and serves as an attachment handle for other cargo. Near-complete labeling of sfGFP-151-SHTP using only 1.5 mM of **1b** was achieved, but an elevated electrode potential of 1V was necessary. Further reducing the concentration of **1b** under these conditions resulted in incomplete conversions, as well as protein overoxidation (FIG. 2C; FIG. 8). Even at this elevated potential, the chemoselectivity of the eCLIC reaction was not compromised, as evidenced from the absence of protein modification in corresponding control reactions with wild-type sfGFP. Further optimization identified HEPES (N-2-hydroxyethylpiperazine-N'-2-ethanesulfonicacid)⁴⁸ buffer to be beneficial for eCLIC; the use of 100 mM HEPES (pH 7) instead of phosphate buffer facilitated near-complete protein labeling using only 0.5 mM **1b,** and also suppressed protein overoxidation, likely due to its known ability to scavenge reactive oxygen species.⁴⁹ Finally, the inclusion of TEMPO,⁵⁰ a known electron-transfer mediator, further improved the efficiency of the reaction, enabling near-complete labeling of sfGFP-151-5HTP using only 0.25 mM dialkylaniline 1b, at 0.7 V.

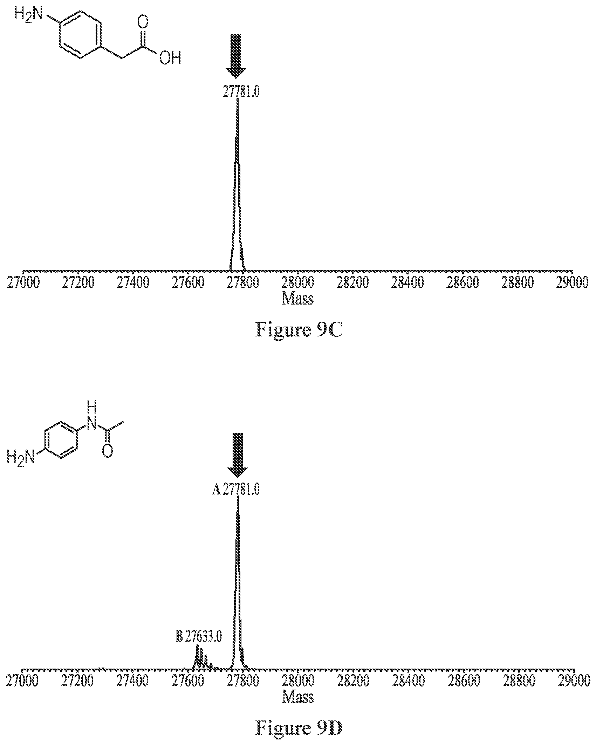

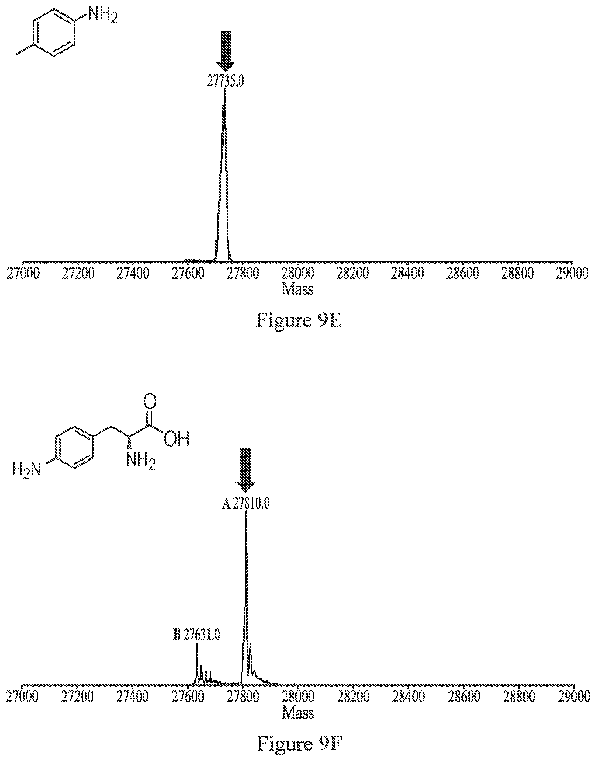

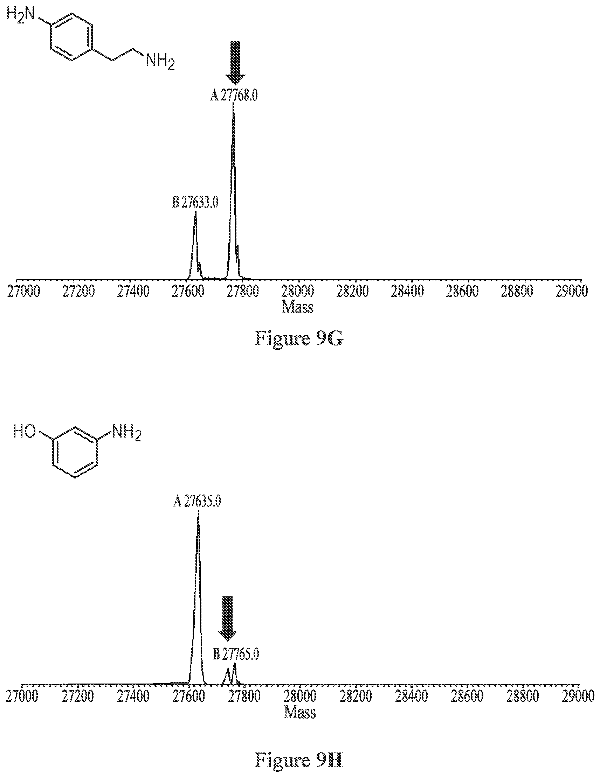

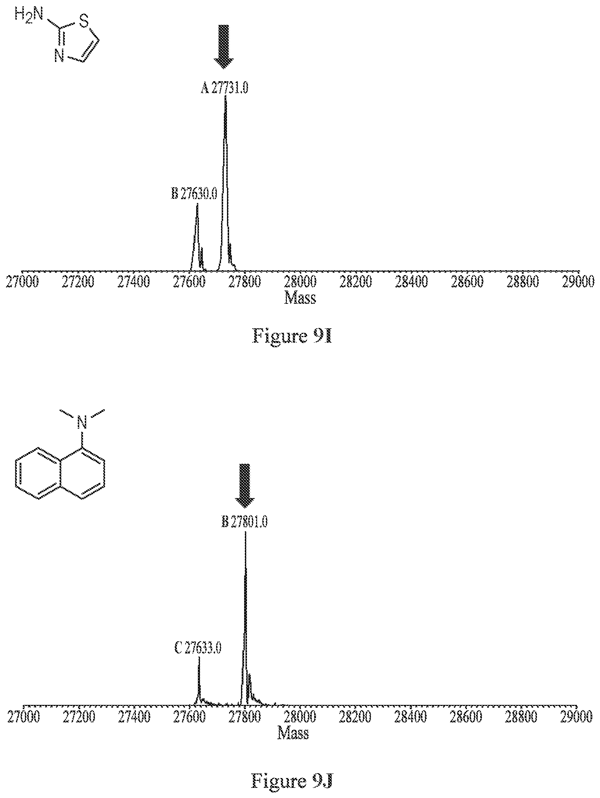

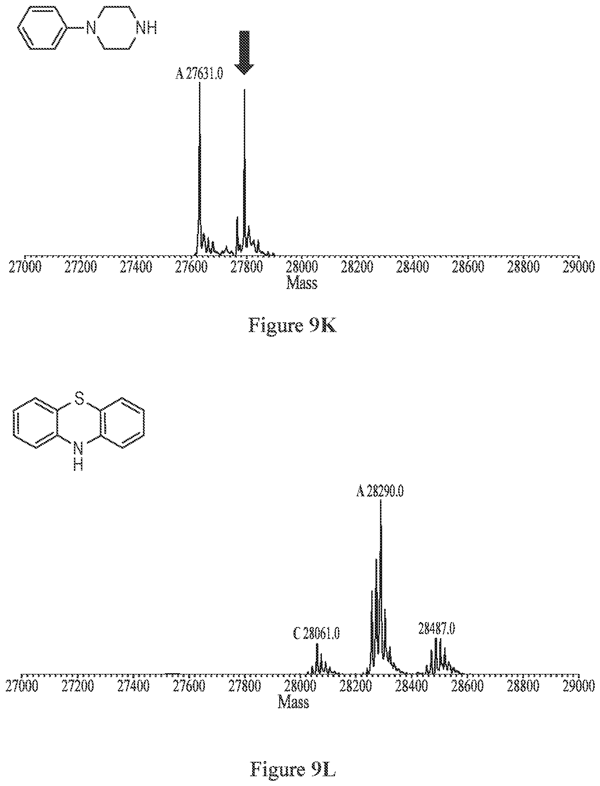

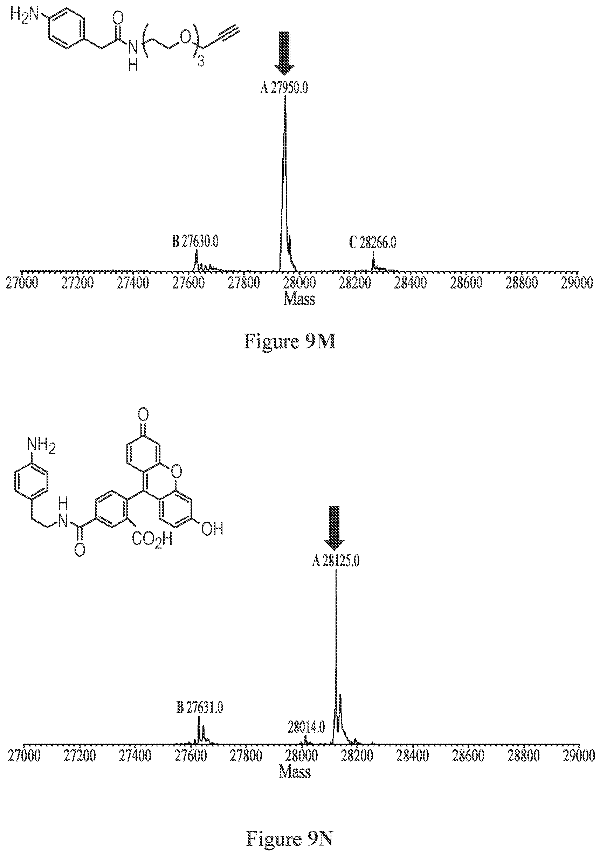

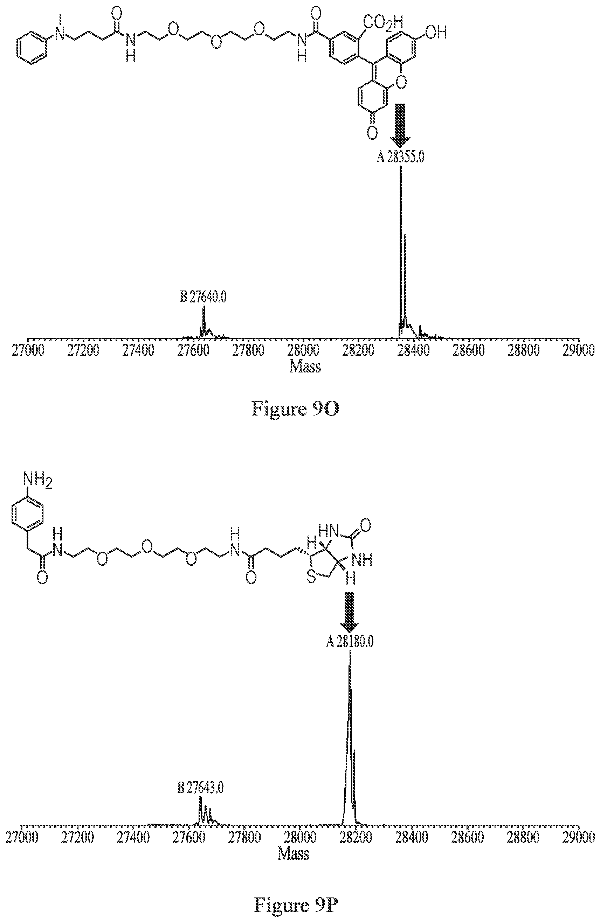

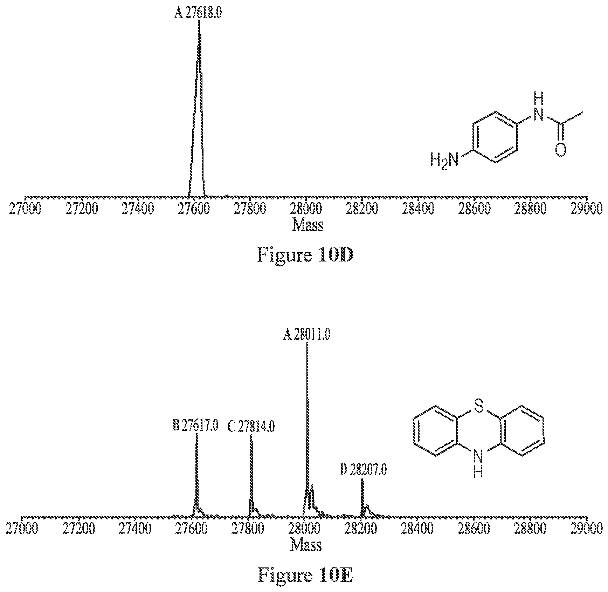

Using these reaction conditions, sfGFP-151-5HTP was functionalized with several other aromatic amines with diverse structures (FIG. 3A; see also FIG. 9A-P). Wild-type sfGFP was not labeled by these reagents under the same eCLIC condition (FIG. 10A-E). Anilines functionalized with additional cargo were also synthesized, such as a terminal alkyne, a biotin, and a fluorophore, attached through a short PEG linker, and demonstrated their successful conjugation to sfGFP-151-TAG using eCLIC (FIG. 3A; FIG. 9). Most of the structurally diverse anilines tested demonstrated efficient protein labeling without further optimization of reaction conditions tailored to individual substrates. Not surprisingly, a labeling reaction with phenothiazine, which is known to facilitate the labeling of tyrosine residues, led to multiple labeling of both the wild-type and 5HTP-mutant of sfGFP (FIG. 3A; FIG. 10). In addition to sfGFP, 5-HTP was also site-specifically incorporated into myoglobin (at site 99) and the 5F7 nanobody that binds the HER2 receptor (at site 69).⁵¹ In each case, the target protein was efficiently modified using eCLIC (FIG. 3A-B), while the corresponding wild-type proteins lacking the 5HTP residue showed no conversion under identical reaction conditions (FIG. 11A-B). These observations underscore the generality of eCLIC for site-specific protein bioconjugation. Its compatibility with a wide range of aromatic amine coupling partners - which are highly accessible and inexpensive - significantly enhances the flexibility and utility of this bioconjugation strategy.

The whole-protein mass spectrometry analyses of eCLIC labeling reactions show product masses that are consistent with the attachment of the aromatic amine to the protein substrate and a concomitant loss of 2 Da, which is indicative of an oxidative coupling reaction. The reaction using free 5HTP and N,N-dimethylaniline **1a** was modeled. However, this was complicated by the high propensity of 5-hydroxyindoles to form complex oligomeric products under mild oxidative condition through homocoupling.^{43-45, 47} Subjecting a mixture of 5HTP and **1a** to eCLIC conditions led to complex product mixtures, even when the latter was used in large excess. This is in stark contrast to the clean eCLIC labeling observed with 5HTP-containing proteins, which likely stems from the site-isolated nature of the protein-bound 5HTP residue that thwarts such homocoupling reactions, and promotes heterocoupling with exogenous aromatic amine coupling partners. Interestingly, the presence of a bulky protection group, such as tert-butyloxycarbonyl (Boc), on the amine functionality of 5HTP suppresses the homocoupling reaction, perhaps mimicking the behavior of a protein-associated 5HTP residue. Using the Boc-protected 5HTP, significantly promoted the heterocoupling reaction with 1a under eCLIC conditions and isolate the resulting coupling product using HPLC (FIG. 12). A thorough structural characterization using NMR, X-ray crystallography, and mass-spectrometry revealed a C-C coupled product where 5HTP and the N.N-dimethylaniline **1a** are connected through the 4 positions of both aromatic rings (FIG. 4). The coupling product formed between Boc-protected 5HTP (17) and a primary aniline substrate **18** was structurally analyzed. Intriguingly, the eCLIC product in this case resulted in a different connectivity, where the aromatic amine is attached to the 4 position of 5HTP, as confirmed by NMR analyses (FIG. 4; FIG. 13A-B).

CV of both N,N-dimethylaniline **1a** and the primary aniline substrate **2** show a significantly higher potential needed for their oxidation relative to 5HTP (FIG. 2A). Consequently, it is reasonable to propose a mechanism where the reaction is initiated through anodic oxidation of 5HTP side chain to create a radical cation. The aromatic amines react with this electrophilic radical either through a radical coupling or a nucleophilic attachment mechanism (FIG. 14). A further single-electron oxidation of this intermediate is necessary to rearomatize the coupled product and complete the reaction. It is reasonably believed that this terminal electron transfer is facilitated by dissolved oxygen or oxidized TEMPO. Indeed, no protein labeling was observed when eCLIC was performed under oxygen-depleted condition and in the absence of TEMPO (FIG. 15). Including TEMPO under oxygen depleted condition partially restored protein labeling. These observations suggest that, unlike established electrochemical protein modification strategies that activate a pro-electrophilic small-molecule labeling reagent, eCLIC proceeds through direct chemoselective activation of the uniquely reactive 5HTP residue on the protein.

Due to its distinctive reaction mechanism, the eCLIC labeling strategy is mutually compatible with the two other widely used bioorthogonal reactions: the strain-promoted azide-alkyne cycloaddition (SPAAC),³⁴. and the inverse-electron demand Diels-Alder (IEDDA) reaction between tetrazines and strained alkenes. ^{16,35} To test this hypothesis, the sfGFP protein was expressed separately incorporating three different ncAAs at the surface-exposed 151 site: 5HTP, AzK, and CpK (FIG. 5A). The pyrrolysyl pair was used to incorporate AzK and CpK in response to a UAG codon, as described previously.²⁰ In each case, incorporation of the desired ncAA was confirmed by the MS analysis, and each sfGFP mutant was separately subjected to three different labeling reactions (FIG. 5A): SPAAC (with BCN), IEDDA (with tetrazine), and eCLIC (with 1b). Modification of each ncAA residue was observed only upon treatment with its cognate reaction partner, confirming the mutual orthogonality of the three labeling reactions (FIG. 5A).

Next, it was demonstrated that eCLIC and SPAAC can be used together to selectively label a protein at two different sites. To this end, we generated an sfGFP protein incorporating 5HTP and AzK at sites 3 and 151, respectively (FIG. 5B).²⁰ This was achieved by co-expressing the sfGFP-3-TGA-151-TAG reporter in ATMW-BL21 *E*. *coli*⁴¹ with a TGA-suppressing EcTrpRS/tRNA pair that charges 5HTP, and a TAG-suppressing pyrrolysyl pair that charges AzK. The resulting protein was purified by immobilized metal-ion chromatography using a C-terminal polyhistidine tag, and was characterized by mass-spectrometry (FIG. 5C). This sfGFP double mutant was first labeled at the 5HTP residue through eCLIC using **1b,** followed by SPAAC modification of the AzK side chain with a cyclooctyne. MS analysis of these reactions confirmed efficient sequential labeling at intended sites to yield the dual-labeled product (FIG. 5C). These results confirm that eCLIC is compatible with other established bioconjugation reactions, and can be used together to facilitate precise multi-site protein labeling.

In recent decades, monoclonal antibodies have been exploited for countless applications, from enabling reagents for biological discovery to powerful new biologics that offer novel therapeutic modalities. Such applications frequently rely on the ability to functionalize the antibody with additional entities. For example, antibodies labeled with fluorescent probes are widely used for numerous assays both for biological research and disease diagnosis. Additionally, antibody-drug conjugates,⁵²⁻⁵³ where a toxic drug molecule is covalently attached to a monoclonal antibody, provide a powerful strategy to deliver such payloads into target cells with high precision, reducing both the effective therapeutic dose, as well as undesirable side-effects. Traditionally, antibody modification has relied on functionalizing canonical nucleophilic side chains such as lysine or cysteine, but such strategies are limited by poor control over the site and stoichiometry of antibody modification. Using the GCE technology, it has been possible to incorporate ncAAs with bioconjugation handles into full-length antibodies, which allows subsequent attachment of cargo with full site control.⁵⁴⁻⁵⁵ Such site-specific labeling strategy provides significant advantage for developing both antibody-based reagents and therapeutics.^{15, 54, 56-57}

To explore the feasibility of using eCLIC for modifying full-length antibodies, the anti-HER2 antibody Trastuzumab was recombinantly expressed site-specifically incorporating 5HTP at the 121 position of the heavy chain. Expi293 suspension cells were transiently transfected with two plasmids, one encoding the mutant heavy chain (HC-121-TGA) and the light chain of Trastuzumab, and the other a TGA-suppressing EcTrpRS/tRNA pair selective for 5HTP (FIG. 6A), which yielded Trastuzumab-HC-121-5HTP with good yield (8 mg/L) after purification. In comparison, the same expression/purification conditions yielded 12 mg/L wild-type Trastuzumab. we were able Trastuzumab-HC-121-5HTPwas homogenously labeled with aniline probes **2** or **1b** using eCLIC selectively on the heavy chain (FIG. 6B.). Next, a primary aniline warhead coupled to a fluorescein (14; FIG. 6B) and conjugated it to Trastuzumab-HC-121-5HTP using the optimized eCLIC condition. Fluorescence imaging following SDS-PAGE (FIG. 6C) and mass-spectrometry analyses (FIG. 6B) confirmed selective and efficient labeling of the heavy chain with the fluorophore, whereas the light chain and the corresponding wild-type antibody was not modified. When this antibody-fluorophore conjugate was incubated with the SK-BR-3 cell line that overexpresses the HER2 receptor, robust cell-binding was observed using fluorescence-activated cell sorting (FACS) analyses, while the same conjugate failed to stain HEK293T cells that do not overexpress this receptor (FIG. 6D). These observations confirm that eCLIC can be used to efficiently generate functional conjugates of full-length antibodies for various applications.

It is now demonstrated herein that electrochemistry can be used to efficiently modify proteins at predefined sites using the unique reactivity of the 5HTP residue that can be introduced into proteins through the GCE technology. Previously reported electrochemical protein bioconjugation strategies rely on activating exogenous electrophilic reagents that functionalize a canonical amino acid residue, which intrinsically lack precise control over the site and stoichiometry of protein labeling. In contrast, eCLIC proceeds through chernoselective anodic oxidation of the protein-associated 5HTP residue, followed by its efficient capture by a wide array of aromatic amines. Notably, the reagents used in eCLIC are inexpensive and readily accessible. For example, 5HTP (<$20/g) is significantly less expensive relative to other commonly used ncAAs for bioconjugation ($250-$1000/g), whereas simple anilines such as 4-aminophenylacetic acid 2 (<$3/g) are orders of magnitude cheaper than labeling agents such as cyclooctynes, and trans-cyclooctenes (>$1000/g). Moreover, it has recently been shown that 5HTP can also be biosynthetically generated in cells,⁵⁸ obviating its exogenous supply altogether. A variety of aromatic amines can be used as coupling partners in eCLIC, which enhances the utility of this bioconjugation strategy. Even though eCLIChas demonstrated broad substrate scope with several different aromatic amines (FIG. 3A), it is likely that the full scope of potential coupling partners is much larger. It is reasonably expected that other electron rich aromatic amino acids (such as amino-tryptophan, amino-tyrosine, hydroxy-tyrosine, histidine-imidazole with electron donating substitutions) can also react selectively in a similar manner under electrochemical conditions. The GCE technology has been used to express ncAA-containing relevant proteins, such as full-length antibodies, in industrial scale (grams/L),^{57, 59} providing an established path to express conjugation-ready proteins for eCLIC labeling in large scale. Finally, the compatibility of eCLIC with established bioconjugation strategies such as SPAAC and IEDDA will enable its use for site-specific labeling of proteins with distinct entities with precise site control. Owing to these advantages, eCLIC will find numerous applications for generating useful conjugates of recombinant proteins.

The ability to precisely chemically label complex biomolecules (protein, DNA, RNA, etc.) is highly useful to understand and engineer their properties. To achieve this, the present invention has developed a new highly efficient "biorthogonal" chemical reaction between an ncAA and different coupling partners, such as aromatic amines, both of which are non-natural chemical groups not found in biomacromolecules.

### Examples

### Material and methods

### Cyclic Voltammogram Measurements

Experiments were conducted in a home-made glass three electrode cell containing a carbon rod counter electrode (99.995% trace metals; Sigma Aldrich), a Ag/AgCl reference electrode (3 M NaCl; Basi Inc.; West Lafayette, IN), and a glassy carbon working electrode (3.0 mM OD; Pine Research; Durham, NC). Prior to each experiment, the glassy carbon electrode was polished for 5 minutes with a 1 µM polycrystalline diamond slurry (Ted Pella; Redding, CA). The electrode potential was controlled with a Versastat 3 potentiostat (AMETEK; Berwyn, PA).

The CV experiments were conducted with 5 mL of electrolyte solution containing a pH 7 PBS supporting electrolyte (20 mM phosphate; 150 mM sodium chloride) and 1 mM of the analyte. The nominal turning potentials for the CV were -0.3 V and +1.7 V vs SHE with a scan rate of 100 mV/s.

The CV experiments with TEMPO and N-Boc-Orvle-5HTP were carried out in 5 mL of electrolyte solution containing a 1:1 mixture of pH 7 PBS supporting electrolyte (20 mM phosphate; 150 mM sodium chloride) and acetonitrile. Mixed into the electrolyte was 250 µM of the analyte of interest. The nominal turning potentials for the CVs were +0.2 V and +1.0 V vs SHE with a scan rate of 10 mV/s.

### Various eCLIC bioconjugation reactions

### General optimized condition for eCLIC

To a 1 mL ElectraSyn reaction vial was added 40 µL of 1 M HEPES, pH 7, 1 µL of 100 mM TEMPO, 8 µL of 1 mM sfGFP-151-5HTP (20 µM final concentration), a sufficient volume of substrate in DMSO for a 250 µM - 1000 µM final concentration, and molecular biology grade water to a final volume of 400 µL. Reactions were run under "Constant Voltage" settings at 0.7 V for 60 min at 1 mM substrate (time and concentration may be optimized), using SK-50 microelectrodes as both the anode and cathode with no reference electrode. Due to the low current generated in the reaction, Vial Detection setting was turned off. The reaction mixture was buffer-exchanged using a Bio-Spin^{®} P-30 column (Bio-Rad 7326006) pre-equilibrated with PBS to remove excess small-molecule substrate and HEPES buffer.

### Reaction under oxygen-depleted conditions

Oxygen-free deionized water was prepared by sparging with nitrogen for 1 hour. The sparged water, solid HEPES powder, concentrated 151-5HTP-sfGFP stock (1.1 mM), 100 mM TEMPO in DMSO, and 100 mM dialkylaniline carboxylate (1b) were transferred to a N₂ glovebox after 3 purge/refill cycles and left for 24 hours. The water was used to prepare fresh 1 M HEPES buffer, pH 7. All components were mixed as in standard reaction conditions (20 µM 151-5HTP-sfGFP, 100 mM HEPES, 1 mM 1b) in the presence or absence of 250 µM TEMPO and sealed in an Electrasyn 2.0 microvial. Reactions were carried out at 700 mV for 80 minutes and processed as described above for mass spectrometry analysis outside of the glove box.

### Testing orthogonality of eCLIC, SPAAC, and IEDDA

stGFP-151-5HTP, sfGFP-151-AzK, and sfGFP-151-CpK were each diluted to 20 µM in 100 mM HEPES, pH 7 for all reactions. eCLIC was conducted on each protein with 250 µM dialkylaniline carboxylate **1b** and 250 µM TEMPO for 1 hour. Tetrazine cycloadditions were carried out with a final concentration of 200 µM tetrazineaminc,⁶¹⁻⁶² and allowed to react for 3 h. Strain promoted azide-alkyne cycloadditions were carried out with a final concentration of 500 µM BCN-OH (Sigma-Aldrich), and allowed to react for 3 h. All labeling reactions were buffer exchanged into PBS by PD-10 spin columns and analyzed by ESI-MS.

### Site-specific dual labelling of sfGFP-3-SHTP-151-AzK

A stock of sfGFP-3-5HTP-151-AzK was diluted to a 20 µM in 100 mM HEPES, pH 7, and reacted with 1 mM **1b** for 30 minutes at 1 V. The sample was buffer exchanged into PBS using a PD-10 spin column, then reacted with BCN-OH was added to a final concentration of 1 mM and was allowed to incubate for 1 hour at room temperature. The protein was analyzed after both steps by ESI-MS.

### Protein expression and purification

### Expression of sfGFP, myoglobin, and 5F7 nanobody in ATMW-BL21

Expression and protein purification of sfGFP, myoglobin, and 5F7 nanobody were performed as previously described, in the *E. coli* ATMW-BL21 strain.⁶³ All proteins were expressed from a pET22b plasmid, driven by a IPTG-inducible T5-lac promoter. In brief, ATMW-BL21 cells were co-transformed with the appropriate pET22b plasmid and a pEVOL-tacI EcTrpRS-h13 leuV³ suppressor plasmid. For wild-type protein expression, the cells were transformed with only the appropriate pET22b plasmid. A single transformant was used to start a 5 mL overnight culture containing the appropriate antibiotics (100 µg/mL ampicillin, 20 µg/mL chloramphenicol, 95 µg/mL spectinomycin, 15 µg/mL zeocin, and 10 µg/mL gentamycin), 0.5 mL of which was used to inoculate a 50 mL LB culture (in a 250 mL sterile Erlenmeyer flask) supplemented with antibiotics. Cultures were grown to an OD₆₀₀ of 0.5-0.6, followed by induction with a final concentration of 1 mM IPTG and 1 mM 5HTP. The cultures were expressed at 30 °C with shaking at 250 rpm for 16 h. The cultures were spun down at 5,000 x g for 10 minutes at 4 °C, and the supernatant was removed. Cell pellets were resuspended in a ratio of 1 mL lysis buffer to 10 mL expression culture volume. Lysis buffer: B-PER Bacterial Protein Extraction Reagent (Thermo Scientific), 1X Halt Protease Inhibitor Cocktail (Thermo Scientific), 0.01% Pierce Universal Nuclease (Thermo Scientific). Cells were nutated for 30 minutes at room temperature, then clarified by centrifugation at 18,000 x g for 10 minutes at 4 °C. The sfGFP reporter proteins contain a C-terminal polyhistidine tag and were purified using a HisPur Ni-NTA resin (Thermo Scientific) following the manufacturer's protocol. Protein purity was confirmed by SDS-PAGE analysis and protein molecular mass was characterized using HPLC-coupled ESI-MS of intact proteins (Agilent Technologies, 1260 Infinity ESI-TOF).

To express the sfGFP-3TGA-151TAG reporter, ATMW-BL21 cells were co-transformed with a pGTEV-3TGA-151TAG reporter plasmid⁶⁴ and pEvoltac EcW.h14-MbPylRS TAG suppressor plasmid encoding both EcTrp and the pyrrolysyl pairs.⁶⁴ The sfGFP reporter was expressed and analyzed as described above, except supplemented with 1 mM 5HTP and 1 mM AzK upon 1 mM IPTG induction, and a larger culture volume of 500 mL (grown and expressed in a 2 L sterile Erlenmeyer flask) was used.

Expression of sfGFP-151-CpK and sfGFP-151-AzK were performed as previously described.⁴

Trastuzumab expression in Expi293 suspension cells and mass spectrometry analysis:
Expi293 cells were grown and cultivated under standard conditions as recommended by the supplier (ThermoFisher). Cells were grown in Expi293 medium supplemented with 0.5x antibiotic-antimyotic (Thermo Fisher, 15240062) at 37 °C, 8% CO₂, in sterile plastic Erlenmeyer flasks with ventilated caps, and shaken using a CO₂-resistant orbital shaker at 125 rpm. Cultures were maintained by seeding at a 0.3 million viable cells/mL, then expanding cultures every 4^{th} day when they reached a density of 3-5 million viable cells/mL. Cell density and viability were measured using the trypan blue exclusion method with a Bio-Rad TC20^{™} automated cell counter.

An adaptation of a previously reported PEI-based transfection method was used for antibody expression.⁶⁵ After reaching 3-5 million viable cells/mL at a minimum of 95% viability, cells were centrifuged at 200 xg for 6 min and resuspended in fresh media to 20 million viable cells/mL. To the concentrated cultures was added a 1:1 ratio of pCDNA3.1-HC-121-TGA-Trastuzumab and pAcBac-EcWRS-H14-1x-EcWtR(TGA) at a final DNA concentration of 25 µg/mL. A concentrated stock solution of 40 kDa linear PEI (40 mg/mL) was added to a final concentration of 50 µg/mL. Cells were incubated with shaking under standard expansion conditions for 3 hours, then diluted to 3-4 million viable cells/mL, and sterile (0.22 µm filtered) 100 mM stocks of 5HTP and valproic acid were added to 1 mM and 2 mM final concentrations, respectively.

After 7-12 days of expression, Expi293 supernatant was harvested by pelleting cells at 800 xg for 8 minutes, then passing the decanted media through a sterile filter (0.22 µm). The media was adjusted to pH 5.4, 50 mM NaOAc using a 500 mM stock. After equilibrating 1 mL Pierce^{™} Protein G resin (Thermo Scientific, 20398) with 15 column volumes of wash buffer (pH 5.4, 50 mM NaOAc), the filtered media was passed through by gravity flow (300-600 mL). Protein G resin was washed with a volume of 50 mM NaOAc, pH 5.4 equal to approximately half the volume of added media (150-300 mL). Purified antibody was eluted by 16-20 stepwise additions of 1 mL pH 2.7, 100 mM glycine, collecting each elution in tubes containing 120 µL pH 7.4 1 M sodium phosphate for immediate neutralization. Eltited fractions were qualitatively tested for the presence of protein by adding 10 µL elution fraction to 10 µL Bradford reagent (Thermo Scientific 1856209). Fractions containing protein were pooled and was concentrated to ~ 1-2 mg/mL final protein concentration using Amicon^{®} Ultra 15 (Millipore Sigma UFC901024) centrifugal filters. Concentrated antibody was buffer-exchanged using a PD midiTrap G-25 column (GE Healthcare 28918008) pre-equilibrated with 20 column volumes of PBS. Protein concentration was determined by absorption measurement using a NanoDrop 2000 spectrophotometer (Fisher Scientific ND2000).

Protein purity and protein conjugation state was determined by HPLC-coupled ESI-MS of fully reduced antibodies (Agilent Technologies, 1260 Infinity ESI-TOF). First, 20 µg antibody sample was adjusted to 50 mM phosphate pH 7.0, 10 mM fresh TCEP, then heated to 55 °C for 10 minutes. After cooling to 37 °C, antibody was incubated 1 µL Remove-iT^{®} PNGase (New England Biolabs, P0706S) for 60 minutes. To remove PNGase, digested antibody was incubated with 25 µL of settled chitin resin (New England Biolabs, S6651L) on an orbital shaker for 15 minutes, then carefully removed as supernatant after centrifuging at 15,000 xg for 1 minute. 250 - 500 ng of protein was used for HPLC-coupled MS analysis as described above.

### Flow cytometry

After reaching confluence, adherent cultures of SK-BR-3 and HEK293T cells were detached from the plate surfaces using 0.25% trypsin (HyClone) for 2 minutes at 37 °C. Trypsin was quenched with two volumes of ice-cold DMEM + 10% FBS, centrifuged at 2,000 xg for 5 minutes, then resuspended in ice-cold PBS at 1-2 million cells/mL. Cells were treated with 10 nM Trastuzumab-HC-121 5HTP-fluorescein-aniline (**14)** conjugate and gently rocked for 15 minutes at 4 °C. Cells were washed once and resuspended in ice-cold PBS, then analyzed by flow cytometry on a BD Accuri C6 Plus flow cytometer (BD Biosciences) using the FITC settings (excitation wavelength: 488 nm, standard filter: 533/30). 10,000 events were collected in BD Accuri C6 Plus. version 1.0.23.1, and data were processed using FlowJo, version 10.8.1.

### Example 1: Small molecule synthesis and characterization

### General information

Unless mentioned otherwise, all reactions were conducted with anhydrous solvents (source. Acros Organics, Fisher Scientific or Alfa Aesar) under an atmosphere of N₂ in oven dried glassware. All work up and purifications were conducted with reagent grade solvents under ambient conditions. Room temperature refers to 22 °C. Thin-layer chromatography was performed using Merck silica gel 60 F₂₅₄ pre-coated plates (0.25 mm). NMR spectra were recorded on Varian INOVA 500 MHz or 600 MHz NMR spectrometers at 25 °C. NMR data were processed using Mnova (Mestrelab). Chemical shifts are reported in ppm from tetramethylsilane (δ 0.00) with the solvent resonance as the internal standard. For ¹H-NMR, data are reported as follows: chemical shift, multiplicity (s = singlet, d = doublet, dd = double doublet, q = quartet, t = triplet, m = multiplet), coupling constants (Hz) and integration. Unless mentioned otherwise, reactions were monitored by liquid chromatography (Agilent Technologies 1260 Infinity) coupled mass-spectrometry (Agilent Technologies 6230 TOF LC/MS) using a reverse phase analytical column (Phenomenex) with mobile phase comprises of water-acetonitrile-0.1% formic acid. Reverse phase HPLC (RP-HPLC) purifications were performed in a Waters HPLC system (fitted with a Waters 1525 binary pump and Waters 2489 UV-VIS detector) using a reverse phase semi-preparative column (Phenomenex, Jupiter^{®} 10 µm, C-18, 300 Å, 250 x 10 mm); mobile phase for the RP-HPLC purification comprised of solvent-A (water-MeCN-0.1% trifluoroacetic acid in 95:5:0.1 ratio) and solvent B (MeCN-water-0.1% trifluoroacetic acid in 95:5:0.1 ratio); flow rate 6 mL/min.

*N*-BOc methyl ester of 5-hydroxytryptophan 16 was prepared from commercially available 5-hydroxy-L-tryptophan (5HTP; Chem Impex International) following a reported procedure.⁶⁶ Electrochemical reactions were performed in an ElectraSyn 2.0 system (IKA) using SK-50 graphite electrodes. PBS buffer has the following composition: 10 mM Na₂HPO₄, 1.8 mM 2.7 mM KH₂PO₄, 137 mM NaCl and 1.8 mM KCl; pH 7.4. Borate buffer has the following composition: 100 mM boric acid, 25 mM sodium tetraborate and 75 mM NaCl; pH 8.

### Example 2: Modelling eCLIC reaction with N,N-dialkyl anilines

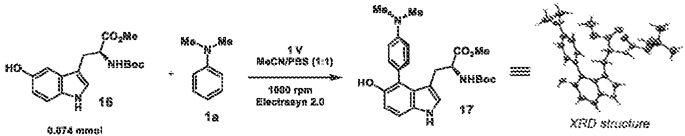

*N*-Boc methyl ester of 5-hydroxytryptophan **16** (25 mg, 0.074 mmol) and *N*,*N-*dimethylaniline **1a** (180 mg, 1.48 mmol, 20 eqv.) were dissolved in a 24 mL mixture of MeCN/PBS (1:1; pH 7.4). Resulting homogeneous mixture was split into four equal (each ~6 mL) parts. The ElectraSyn vial cap having a graphite anode and a graphite cathode were inserted into one part (6 mL) of the mixture in a ElectraSyn vial and subjected to electrolysis under a constant voltage (1 V) under ambient conditions while stirring vigorously (1000 rpm). After 1 h the ElectraSyn vial cap was removed and electrodes were rinsed with MeCN (5 mL); the washing layer was combined with the rest of the crude reaction mixture and preserved at 4 °C. Aforementioned protocol was followed for the rest three parts. Crude reaction mixtures from the four batches were pooled together and was diluted with 60 mL aqueous elution buffer (comprises of water-MeCN-0. 1% formic acid in 95:5:0.1 ratio) and poured into a column pre-packed with 8 g reverse phase C18 silica gel (particle size: 48-65 µm; Acros Organics; CAS no. 71889-02-6). After eluting the applied solution the column was washed with ~10000 mL aqueous elution buffer to remove all the unreacted *N,N*-dimethylaniline (judged by silica gel TLC). Next, a 1:1 mixture of organic (comprises of MeCN-water-0.1% formic acid in 95:5:0.1 ratio) and aqueous elution buffer was passed through the column and the eluted fractions were analyzed by LC-MS. The fractions having the expected product mass were pooled together, concentrated in a rotary evaporator followed by lyophilization. Further purification with RP-HPLC using a semi-preparative C18 column provided the analytically pure product (white solid, 6 mg). Following elution conditions was used for RP-HPLC: 0-10 min 100% A, 10-40 min 100% A to 100% B, 40-45 min 100% B, 45-48 min 100% B to 100% A, 48-50 min 100% A); retention time of the product = 21.03 min. **¹H-NMR (500 MHz, CD₃CN):** 9.06 (s; 1H), 7.37-7.41 (m; 2H), 7.32 (d, *J* = 5; 2H), 7.26 (d, *J* = 10 Hz; 1H), 6.99 (d, *J* = 5 Hz; 1H), 6.78 (d, *J* = 10; 1H), 5.14 (d; *J* = 5 Hz; 1H), 3.54-3.56 (m; 1H), 3.48 (s; 3H), 3.13 (s; 6H), 2.62 (dd, *J* = 5, 15 Hz; 1H), 2.40 (dd, *J* = 10, 15 Hz; 1H), 1.29 (s; 6H), 1.18 (s; 3H); **¹H-NMR (500 MHz, D₂O):** 7.74-7.77 (m; 2H), 7.67-7.69 (m; 2H), 7.47 (d, *J =* 5; 2H), 7.16 (s; 1H), 6.99 (d, *J =* 5 Hz; 1H), 3.54 (s; 3H), 3.39 (s; 6H), 2.67 (dd, *J* = 5, 15 Hz; 1H), 2.49 (dd, *J* = 10, 15 Hz; 1H), 1.30 (s; 6H), 1.15 (s; 3H); **¹³C-NMR (150 MHz, CD₃CN):** δ 173.79, 156.17, 147.98, 146.09, 133.28, 133.26, 132.92, 126.90, 126.59, 125.79, 118.66, 112.65, 112.48, 110.71, 79.72, 55.38, 52.28, 44.99, 29.47, 28.46, 28.23; **¹³C-NMR (150 MHz, D₂O):** δ 177.84, 159.70, 148.06, 144.30, 141.50, 135.41, 129.91, 127.14, 122.86, 120.31, 115.27, 114.59, 111.01, 83.91, 57.49, 55.05, 49.05, 30.95, 30.06, 29.74; ESI-TOF-MS (m/z): [M-+H]⁺ Calculated for C₂₅H₃₂N₃O₅ 454.2342, obtained 454.2170. The product structure was confirmed by X-ray diffraction analysis of a single crystal of the product grown at the room temperature (by slow evaporation of a solution in MeOH/diethylether). HPLC-MS profile of a solution of the crystal was found to be identical with that of the NMR characterized sample.

### Example 3: Modelling eCLIC reaction of primary anilines

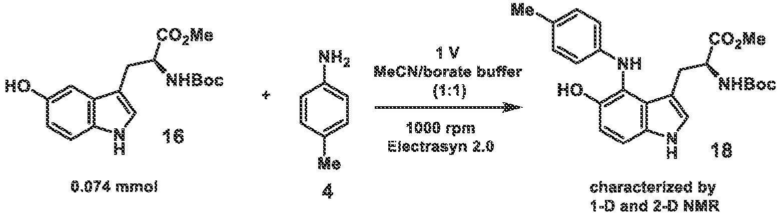

*N*-Boc methyl ester of 5-hydroxytryptophan **16** (25 mg, 0.074 mmol) and*p-*toludine **4** (80 mg, 0.74 mmol) were dissolved in a 12 mL mixture of MeCN/borate buffer (1:1; pH 8.0) and were split into two equal (each 6 mL) parts. The ElectraSyn vial cap having a graphite anode and a graphite cathode were inserted into one part (~6 mL) of the mixture and subjected to electrolysis under a constant voltage (1 V) under ambient conditions while stirring vigorously (1000 rpm). After 2 h the ElectraSyn vial cap was removed and electrodes were rinsed with MeCN (5 mL); the washing layer was combined with the rest of the reaction mixture and preserved at 4 °C. Aforementioned protocol was followed for the second part of the ~6 mL mixture. Crude reaction mixtures from the two batches were pooled together, diluted with 30 mL aqueous elution buffer (comprises of water-MeCN-0.1% formic acid in 95:5:0.1 ratio) and poured into a column pre-packed with 5 g reverse phase C18 silica gel (particle size: 48-65 µm; Acros Organics; CAS no. 71889-02-6). After the elution of the applied solution the column was washed with ~10000 mL aqueous elution buffer to remove all the unreacted *p*-toludine (judged by silica gel TLC). Next, a 1:1 mixture of organic (comprises of MeCN-water-0.1% formic acid in 95:5:0.1 ratio) and aqueous elution buffer was passed through the column and the eluted fractions were analyzed by LC-MS. Combined fractions with expected product were concentrated in a rotary evaporator followed by lyophilization. Repurification with a RP-HPLC utilizing a semi-preparative C-18 column provided the analytically pure product (white solid, 4 mg). Following elution conditions was used for RP-HPLC: 0-10 min 100% A, 10-40 min 100% A to 100% B, 40-45 min 100% B, 45-48 min 100% B to 100% A, 48-50 min 100% A); retention time of the product = 29.71 min. **¹H-NMR (500** MHz, **CD₃CN):** 9.12 (s; 1H), 7.20 (d, *J* = 10 Hz; 1H), 6.97 (d, *J* = 5; 1H), 6.94 (d, *J* = 10 Hz; 2H), 6.82 (d, *J* = 10 Hz; 1H), 6.44 (d, *J* = 10; 2H), 5.83 (s; 1H), 4.18-4.23 (m; 1H), 3.57 (s; 3H), 2.93 (dd, *J* = *5,* 10 Hz; 1H), 2.71-2.76 (m; 1H), 2.19 (s; 3H), 1.34 (s; 6H), 1.22 (s; 3H); **¹³C-NMR (150 MHz, CD₃CN):** δ 173.92, 157.38, 147.89, 146.96, 133.50, 130.52, 128.45, 126.15, 126.05, 119.07, 115.03, 112.32, 111.33, 110.37, 79.85, 56.53, 52.38, 29.18, 28.51, 20.50. ESI-TOF-MS (m/z): [M+H]⁺ Calculated for C₂₄H₃₀N₃O₅ 440.2185, obtained 440.2161.

### Example 4: Synthesis of the primary aniline FAM conjugate

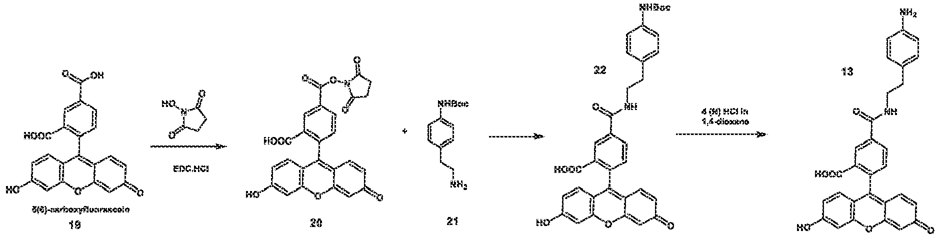

To a solution of 5(6)-carboxyfluorescein **19** (130 mg, 0.35 mmol; mixture of 5- and 6- isomer; Cayman Chemical) in anhydrous DMF (0.5 mL) was added 1-[3-(dimethylamino)propyl]-3-ethylcarbodiimide hydrochloride (EDC.HCL, 76 mg, 0.4 mmol; Chem Impex Int.) followed by the addition of *N*-hydroxysuccinimide (52 mg, 0.45 mmol) and the resulting mixture was stirred at the room temperature for 16 h. Solvent was evaporated off under reduced pressure and the residue was dissolved in a mixture of THF/EtOAc (50 mL, 1:9) and washed with water (4x15 mL). Combined organic layer was dried over anhydrous Na₂SO₄ and concentrated under reduced pressure to get the *N-*succinimidyl ester of 5(6)-carboxyfluorescein **20** (126 mg, 0.26 mmol, 74% yield). The purity of the product was confirmed by HPLC-MS analysis. ESI-TOF-MS (m/z): [M+H]⁺ Calculated for C₂₅H₁₆NO₉ 474.0825, obtained 474.0620. The intermediate was used directly in the next step without further purification.

*N*-Boc-protected phenethylamine (**21**) was prepared following a reported protocol from 4-aminophenethylamine (Combi-Blocks).⁶⁷

To a solution of *N*-succinimidyl ester of 5(6)-carboxyfluorescein **20** (120 mg, 0.25 mmol) in THF (5 mL) was added *N*-Boc-protected phenethylamine **21** (70 ing, 0.3 mmol) and the resulting mixture was stirred at the room temperature for 20 h to have complete conversion (LC-MS). The solvent was evaporated off under reduced pressure and the residue was partitioned between a biphasic mixture of water (25 mL) and organic solvents (EtOAc/THF 9:1, 40 mL). Organic layer was separated and aqueous layer was extracted with the EtOAc/THF mixture (3x15 mL). Combined organic layer was dried over anhydrous Na₂SO₄ and concentrated under reduced pressure to get the expected product **22** as a red semi-solid (118 mg, 0.2 mmol, 80% yield). The purity of the product was confirmed by HPLC-MS analysis. ESI-TOF-MS (m/z): [M+H]⁺ Calculated for C₃₄H₃₁N₂O₈ 595.2080, obtained 595.1850. The intermediate was used directly in the next step without further purification.

Anhydrous 4 (N) HCl solution in 1,4-dioxane (5 mL) was added to the intermediate **22** under an inert atmosphere. Resulting mixture was stirred for 4 h at the room temperature. LC-MS analysis of the crude mixture confirmed completion of the removal of the Boc protection. Solvent was evaporated off under reduced pressure to get an oil that was repeatedly diluted with 15 mL chloroform and dried under reduced pressure to get rid of dissolved hydrochloric acid. The expected product **13** was obtained as an orange-yellow solid (105 mg, quantitative yield). The purity of the product was confirmed by HPLC-MS analysis. ESI-TOF-MS (m/z): [M+H]⁺ Calculated for C₂₉H₂₃N₂O₆ 495.1556, obtained 495.1436.

### Example 5: Synthesis of 4-(methyl(phenyl)amino)butanoic acid (1b)

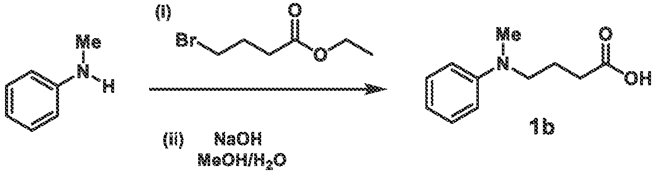

**1b** was prepared according to a reported procedure through a two-step sequence:⁶⁸

### Example 6: Synthesis of the dialkylanilne-FAM conjugate 14

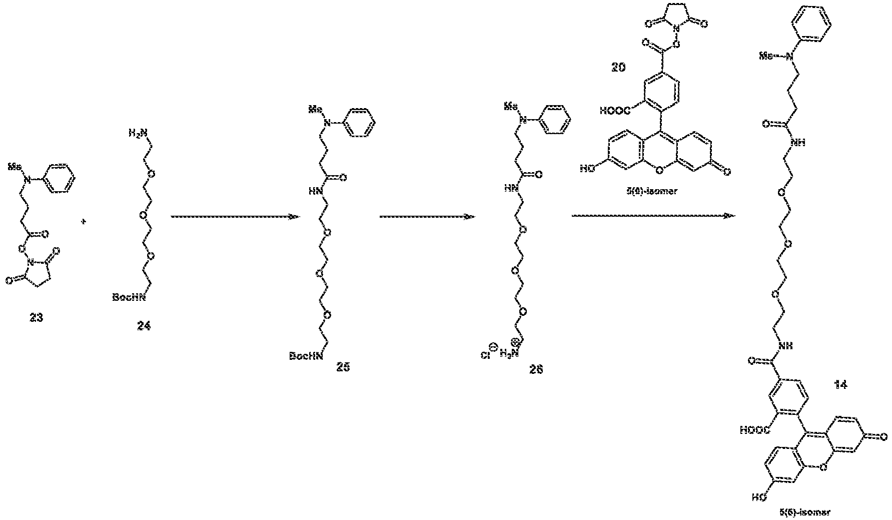

*N*-hydroxysuccinimide (1.07 g, 9.3 mmol) was added to a solution of 4-(methyl(phenyl)amino)butanoic acid **1b** (1.2 g, 6.2 mmol) in THF (30 mL), followed by the addition of EDC.HCl (1.78 g, 9.3 mmol). The resulting mixture was stirred at the room temperature for 16 h and then concentrated under reduced pressure. The residue was partitioned between a biphasic mixture of EtOAc (100 mL) and water (50 mL). Organic layer was separated washed sequentially with water (3x30 mL) and brine (30 mL). The organic layer was dried over anhydrous Na₂SO₄ and concentrated under reduced pressure to get the *N*-succinimidyl ester **23** (2.4 g, 8.3 mmol, 89% yield) as a white solid. **¹H-NMR (500 MHz, CDCl₃):** 7.21-7.25 (m; 2H), 6.69-6.74 (m; 3H), 3.43 (t, *J* = 10 Hz; 2H), 2.94 (s; 3H), 2.81 (s; 4H), 2.66 (t, *J* = 10 Hz; 2H), 1.99-2.06 (m; 2H); **¹³C-NMR (125 MHz, CDCl₃):** δ 169.26, 168.52, 149.15, 129.34, 116.11, 112.42, 51.45, 38.45, 28.43, 25.66, 22.04.

A mixture of the *N*-succinimidyl ester **23** (655 mg, 2.26 mmol) and Boc-amino-PEG₃-amine **24** (670 mg, 2.29 mmol; Ambeed, Inc.) in dichloromethane (5 mL) was stirred at the room temperature for 20 h. Solvent was evaporated off under reduced pressure to get an oil that was purified by silica gel (100-200 mesh) flash column chromatography (gradient elution: 50% EtOAc in hexane to EtOAc to 1% MeOH in EtOAc) to afford the product **25** as a yellow oil (875 mg, 1.87 mmol, 83% yield). **R_{f}**: 0.20 (1% MeOH in EtOAc); **¹H-NMR (600 MHz, CDCl₃):** 7.17-7.20 (m; 2H), 6.68-6.69 (m; 2H), 6.64 (t, *J* = 6 Hz; 1H), 6.20 (s; 1H), 5.07 (s; 1H), 3.56-3.60 (m; 9H), 3.51-3.53 (m; 2H), 3.49 (t, *J* = 6 Hz; 2H), 3.42 (q, *J* = 6 Hz; 2H), 3.32 (t, *J* = 6 Hz; 2H), 3.25-3.28 (m; 2H), 2.89 (s; 3H), 2.19 (t, *J* = 6 Hz; 2H), 1.87-1.92 (m; 2H), 1.42 (s; 9H), **¹³C-NMR (150 MHz, CDCl₃):** δ 172.57, 156.04, 149.40, 129.17, 116.17, 112.35, 112.27, 79.23, 70.44, 70.41, 70.17, 70.15, 69.88, 53.48, 51.99, 40.34, 39.21, 38.17, 33.54, 28.44, 22.80.

Anhydrous 4 (N) HCl solution in 1,4-dioxane (8 mL) was added to the oil (800 mg) under an inert atmosphere. Resulting mixture was stirred for 5 h at the room temperature. LC-MS analysis of the crude mixture confirmed complete removal of the Boc protection. The solvent was evaporated off under reduced pressure to get an oil that was repeatedly diluted with 10 mL methanol and dried under reduced pressure to get rid of dissolved hydrochloric acid. The hydrochloride salt of the expected product **26** was obtained as a yellow oil (750 mg, quantitative yield). The purity of the product was confirmed by HPLC-MS analysis. **¹H-NMR (600 MHz, CD₃OD):** 7.73-7.75 (m; 2H), 7.62-7.65 (m; 2H), 7.58-7.60 (m; 1H), 3.72-3.74 (m; 2H), 3.61-3.70 (m; 11H), 3.55 (t, *J* = 6 Hz; 2H), 3.38 (t, *J* = 6 Hz; 2H), 3.30 (s; 3H), 3.14 (t, *J* = 6 Hz; 2H), 1.83-1.85 (m; 2H); **¹³C-NMR (150 MHz, CD₃OD):** δ 174.57, 142.16, 131.84, 131.65, 122.65, 71.43, 71.13, 71.10, 70.40, 67.76, 60.21, 49.85, 46.37, 40.64, 40.40, 33.44, 22.22. ESI-TOF-MS (m/z): [M+H]⁺ Calculated for C₁₉H₄₃N₃O₄ 368.2549, obtained 368.2394.

Potassium carbonate (40 mg, 0.3 mmol) was added to a solution of the hydrochloride intermediate **26** (46 mg, 0.1 mmol) in DMF (0.2 mL) and stirred for 5 min before the addition of a solution of *N*-succinimidyl ester of 5(6)-carboxyfluorescein **20** (48 mg, 0.1 mmol) in THF (2 mL). Resulting mixture was stirred at the room temperature for 14 h to allow full conversion (LC-MS). Solvent was evaporated off under reduced pressure and the residue was partitioned between a biphasic mixture of water (10 mL) and organic solvents (EtOAc/THF 9:1, 20 mL). Organic layer was separated and aqueous layer was extracted with the EtOAc/THF mixture (4x15 mL). Combined organic layer was dried over anhydrous Na₂SO₄ and concentrated under reduced pressure to get 20 as an oil (72 mg, 0.1 mmol, 67% yield). The purity of the product was confirmed by HPLC-MS analysis. ESI-TOF-MS (m/z): [M+H]⁻ Calculated for C₄₀H₄₄N₃O₁₀ 726.3026, obtained 726.2658.

### Example 7: Synthesis of Biotin-PEG3-aniline (15)

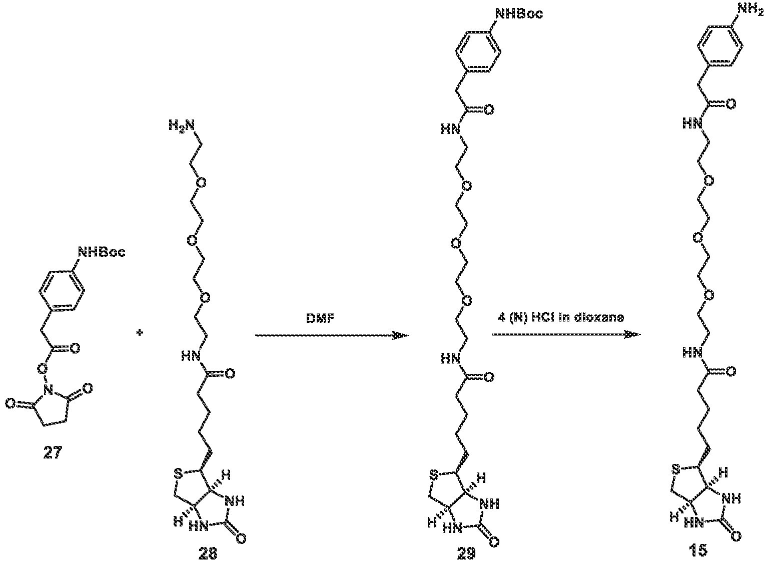

A mixture of biotin-PEG₃-amine **28** (420 mg, 1.0 mmol; BroadPharm), *N-*succinimidyl ester **27** (419 mg, 1.2 mmol) in anhydrous DMF (1 mL) was heated and stirred at 50 °C for 12 h. The solvent was evaporated off under reduced pressure and the residue was purified by silica gel (100-200 mesh) column chromatography (gradient elution: EtOAc to 2% MeOH in EtOAc to 10% MeOH in EtOAc and then to 25% MeOH in EtOAc) to afford the product. Silica gel was removed from the product by dissolving the product in warm chloroform and filtering. The product **29** was obtained as a yellow oil (508 mg, 0.78 mmol; 78% yield). **R_{f}**: 0.60 (50% MeOH in EtOAc); **¹H-NMR (500 MHz, CDCl₃):** 7.32-7.33 (m; 2H), 715-7.16 (m; 2H), 6.76 (s; 1H), 6.37-6.43 (m; 2H), 6.84 (s; 1H), 4.45-4.47 (m; 1H), 4.25-4.27 (m; 1H), 4.08-4.12 (m; 1H), 3.48-3.57 (m; 12H), 3.35-3.41 (m; 3H), 3.08-3.12 (m; 1H), 2.84-2.88 (m; 1H), 2.65-2.72 (m; 3H), 2.16-2.19 (m; 2H), 1.58-1.68 (m; 4H), 1.49 (s; 9H), 1.37-1.41 (m; 2H), 1.22-1.25 (m; 3H); **¹³C-NMR (125 MHz, CDCl₃):** δ 173.91, 171.98, 164.69, 153.34, 137.96, 130.16, 129.80, 119.34, 81.42, 70.66, 70.63, 70.45, 70.33, 70.14, 70.01, 62.24, 60.69, 55.83, 43.15, 40.73, 39.69, 39.47, 36.11, 28.67, 25.79, 25.73, 21.33. FSI-TOF-MS (m/z): [M+H]⁺ Calculated for C₃₁H₅₀N₅O₈S 652.3374, obtained 652.3164.

To the intermediate (**29**) a solution of 4 (N) HCl in 1,4-dioxane (10 mL) was added dropwise at 4 °C and the resulting mixture was allowed to come to the room temperature while stirred. A solid precipitate started forming. After 4 h stirring at the room temperature the mixture was concentrating under reduced pressure. To the resulting oil, chloroform (~15 mL) was added and concentrated to dryness to get rid of dissolved HCl. The oil was triturated with diethylether (~20 mL) and dried thoroughly. Resulting solid residue was dissolved in ~10 mL water and lyophilized to get the expected product (15) as a yellow solid (410 mg, 0.74 mmol, 95% yield). **¹H-NMR (600 MHz, D₂O):** δ 7.38 (d, *J* = 12 Hz; 2H), 7.33 (d, *J* = 12; 2H), 4.49-4.52 (m; 1H), 4.30-4.32 (m; 1H), 3.74-3.76 (m; 1H), 3.65-3.68 (m; 1H), 3.59-3.60 (m; 8H), 3.53-3.56 (m; 4H), 3.29-3.34 (m; 4H), 3.20-3.23 (m; 1H), 2.88-2.91 (m; 1H), 2.67-2.71 (m; 1H), 2.16-2.19 (m; 2H), 1.43-1.65 (m; 4H), 1.27-1.33 (m; 2H); **¹³C-NMR (150 MHz, D₂O):** δ 176.72, 173.75, 165.08, 136.27, 130.66, 128.62, 123.22, 71.49, 70.63, 69.56, 69.34, 68.75, 68.67, 62.00, 60.19, 55.25, 43.20, 41.62, 39.62, 39.07, 38.86, 35.33, 27.82, 27.60, 25.06 ESI-TOF-MS (m/z): [M+H]⁻ Calculated for C₂₆H₄₂N₅O₆S 552.2361, obtained 552.2388.

### Example 8: Synthesis of alkyne-PEG₃-aniline (12)

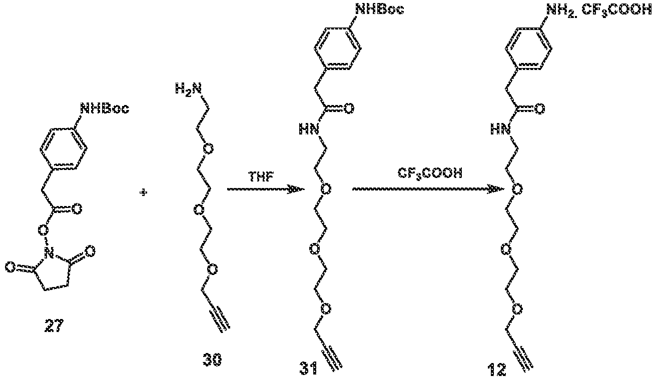

A mixture of alkyne-PEG₃-amine **30** (375 mg, 2.0 mmol; Lumiprobe), *N-*succinimidyl ester **27** (700 mg, 2.01 mmol) in anhydrous THF (1 mL) was heated and stirred at 60 °C for 8 h to allow full conversion of the starting *N*-succinimidyl ester (judged by LC-MS). The solvent was evaporated off under reduced pressure and the residue was purified by silica gel (100-200 mesh) column chromatography (gradient elution: 50% EtOAc in hexanes to EtOAc to 2% MeOH in EtOAc) to afford the product. Silica gel was removed from the product by dissolving the product in warm chloroform and filtering. Concentrating the filtrate provided the intermediate **31** as a yellow oil (722 mg, 1.72 mmol; 86% yield). **R_{f}:** 0.40 (1%) MeOH in EtOAc); **¹H-NMR (500 MHz, CDCl₃):** δ 7.33 (d, *J* = 10 Hz; 2H), 7.16 (d, *J* = 10; 2H), 6.76 (s; 1H), 6.04 (s; 1H), 4.17-4.18 (m; 2H), 3.61-3.68 (m; 4H), 3.52-3.56 (m; 4H), 3.47-3.49 (m; 4H), 3.37-3.40 (m; 2H), 2.42-2.43 (m; 1H), 1.50 (s; 9H), **¹³C-NMR (125 MHz, CDCl₃):** δ 171.55, 153.08, 137.88, 130.18, 129.74, 119.24, 80.75, 79.85, 74.97, 70.87, 70.77, 70.60, 70.50, 69.99, 60.66, 58.66, 43.29, 36.84, 28.62.

The intermediate **31** was dissolved in 5 mL CH₂Cl₂, and anhydrous trifluoroacetic acid (5 mL) was added dropwise at 4 °C, and the resulting mixture was stirred at 4 °C for 5 h to have complete removal of Boc protection (judged by LC-MS). The mixture was concentrated under reduced pressure; to fully remove TFA, additional CH₂Cl₂ (~10 mL) was added and the solution was evaporated four consecutive times. The expected product **12** was obtained as a yellow oil (545 mg, 1.7 mmol, quantitative yield). **¹H-NMR (500 MHz, CD₃OD):** δ 7.22 (d, *J =* 10 Hz; 2H), 7.10 (d, *J* = 10; 2H), 5.24 (s; 1H), 3.94 (d, *J* = 5; 2H), 3.35-3.44 (m; 9H), 3.29-3.31 (m; 2H), 3.07 (s; 1H), 2.60 (d, *J* = 5; 1H), **¹³C-NMR (125 MHz, CD₃OD):** δ 173.58, 138.74, 132.23, 130.98, 124.42, 80.82, 76.28, 71.83, 71.61, 71.50, 70.70, 70.36, 59.33, 43.23, 40.90. ESI-TOF-MS (m/z): [M+H]⁺ Calculated for C₁₇H₂₅N₂O₄ 321.1814, obtained 321.1799.

### Example 9: Mass spec and HPLC analysis of eCLIC reactions and conjugates

MS analysis of eCLIC reactions associated with FIG. 2C. Expected molecular weights: sfGFP-151-5HTP (27636 Da), conjugate with **1a** (27755 Da), conjugate with 1b (27827 Da), conjugate with 2 (27785 Da). (FIG. 8A-G).

MS analysis of eCLIC reactions of sfGFP-151-5HTP with various substrates associated with FIG. 3A. Expected molecular weight of unmodified sfGFP-151-5HTP is 27636 Da. Expected eCLIC adduct is highlighted with a green arrow in each case. (FIG. 9A-P).

FIG. 10A-E shows representative examples of MS analysis of eCLIC reactions of wild-type sfGFP with various substrates shown in FIG 3A. None of the substrates show detectable labeling, except for phenothiazine **(11),** which is known to label tyrosine residues. Expected molecular weight of unmodified wild-type sfGFP is 27618 Da.

FIG. 11A-B shows Myoglobin and 5F7 anti-HER2 nanobody control proteins lacking a 5HTP residue do not undergo eCLIC labeling with the 1b aniline, even though the 5HTP mutants of these proteins can be efficiently labeled (FIG. B-C).

FIG. 12 shows HPLC-MS analysis of the eCLIC reaction between N-Boc methyl ester of 5-hydroxytryptophan **17** (exact mass: 334.15) and N,N-dimethylaniline (exact mass: 121.09); A₂₈₀ represents absorbance at 280 nm in an arbitrary unit; N,N-dimethylaniline has little absorbance at 280 nm (elutes around 4.5 min); there was a single peak having the expected product mass (at 9.3 min, see below for the mass spec) in the crude reaction mixture, other peaks correspond to unreacted starting material (5HTP derivative), higher order homo- and hetero-coupling reactions between the starting materials having following m/z (protonated forms): peak (i) 786.35 (2A+B-4+H⁺), peak (ii) 1118.48 (3A+B-6+H⁺), peak (iii) 667.28 (2A-2+H⁺) etc. [A and B refers to **17** and **1a,** respectively]; expected product (A+B-2) having m/z 454.21 was structurally characterized by mass spectrometry, NMR and XRD analysis; dissolved crystal refers to a solution of the crystal of the product, the crystal having been retrieved from the same sample pool from which diffraction quality single crystal was picked for XRD structure analysis.

FIG. 13A-B shows HPLC-MS analysis of the eCLIC reaction modelling between *N*-Boc methyl ester of 5-hydroxytryptophan **17** (exact mass: 334.15) and*p-*toludine **4** (exact mass: 107.15); A₂₅₄ represents absorbance at 254 nm in an arbitrary unit; expected product (A+C-2) having m/z 440.21 (protonated form) was structurally characterized by mass spectrometry and co-relation NMR analysis; peak (i) had m/z (protonated form) of 438.21 (A+C-4+H⁺) is likely generated by over-oxidation of the expected product (A+C-2); peak (ii) had m/z (protonated forms) of 454.20 (A+C-4+16+H⁺) is suggestive of a further oxidized product, [A and C refers to the **17** and **4,** respectively] **(B)** chemical shifts of ¹H and ¹³C assigned through 1-D and 2-D NMR analysis (*vide infra*); carbons are numbered numerically and protons are designated alphabetically.

Fig. 14 shows a proposed mechanism of eCLIC.

FIG. 15 shows the results of experiments demonstrating the interchangeable roles of TEMPO and oxygen in the eCLIC reaction. sfGFP-151-5HTP was reacted with **1b** in 100 mM HEPES, pH 7.0 at 700 mV for 1 hour under oxygen-depleted conditions in the absence of TEMPO, oxygen-depleted conditions in the absence of TEMPO (250 µM), and optimized eCLIC conditions (aerobic + TEMPO). Expected molecular weight for sfGFP-151-TAG is 27636 Da, and its conjugation product with **1b** is 27827 Da.

### Example 10: Plasmid maps and primer information

FIG. 16 shows the plasmid map of pCBNA3.1-C-Her2+SbfI (containing Trastuzumab light chain and heavy chain fragments)

### Sequence: pCDNA3.1-C-Her2+SbfI (containing Trastuzumab light chain and heavy chain fragments) (SEQ ID NO:1)

CGACTTATCGCCACTGGCAGCAGCCACTGGTAACAGGATTAGCAGA
GCGAGGTATGTAGGCGGTGCTACAGAGTTCTTGAAGTGGTGGCCTAACTACG
GCTACACTAGAAGAACAGTATTTGGTATCTGCGCTCTGCTGAAGCCAGTTACC
TTCGGAAAAAGAGTTGGTAGCTCTTGATCCGGCAAACAAACCACCGCTGGTA
GCGGTTTTTTTGTTTGCAAGCAGCAGATTACGCGCAGAAAAAAAGGATCTCAA
GAAGATCCTTTGATCTTTTCTACGGGGTCTGACGCTCAGTGGAACGAAAACTC
ACGTTAAGGGATTTTGGTCATGAGATTATCAAAAAGGATCTTCACCTAGATCC
TTTTAAATTAAAAATGAAGTTTTAAATCAATCTAAAGTATATATGAGTAAACT
TGGTCTGACAGTTACCAATGCTTAATCAGTGAGGCACCTATCTCAGCGATCTG
TCTATTTCGTTCATCCATAGTTGCCTGACTCCCCGTCGTGTAGATAACTACGAT
ACGGGAGGGCTTACCATCTGGCCCCAGTGCTGCAATGATACCGCGAGACCCA
CGCTCACCGGCTCCAGATTTATCAGCAATAAACCAGCCAGCCGGAAGGGCCG
AGCGCAGAAGTGGTCCTGCAACTTTATCCGCCTCCATCCAGTCTATTAATTGTT
GCCGGGAAGCTAGAGTAAGTAGTTCGCCAGTTAATAGTTTGCGCAACGTTGTT
GCCATTGCTACAGGCATCGTGGTGTCACGCTCGTCGTTTGGTATGGCTTCATTC
AGCTCCGGTTCCCAACGATCAAGGCGAGTTACATGATCCCCCATGTTGTGCAA
AAAAGCGGTTAGCTCCTTCGGTCCTCCGATCGTTGTCAGAAGTAAGTTGGCCG
CAGTGTTATCACTCATGGTTATGGCAGCACTGCATAATTCTCTTACTGTCATGC
CATCCGTAAGATGCTTTTCTGTGACTGGTGAGTACTCAACCAAGTCATTCTGA
GAATAGTGTATGCGGCGACCGAGTTGCTCTTGCCCGGCGTCAATACGGGATAA
TACCGCGCCACATAGCAGAACTTTAAAAGTGCTCATCATTGGAAAACGTTCTT
CGGGGCGAAAACTCTCAAGGATCTTACCGCTGTTGAGATCCAGTTCGATGTAA
CCCACTCGTGCACCCAACTGATCTTCAGCATCTTTTACTTTCACCAGCGTTTCT
GGGTGAGCAAAAACAGGAAGGCAAAATGCCGCAAAAAAGGGAATAAGGGCG

ACACGGAAATGTTGAATACTCATACTCTTCCTTTTTCAATATTATTGAAGCATT
TATCAGGGTTATTGTCTCATGAGCGGATACATATTTGAATGTATTTAGAAAAA
TAAACAAATAGGGGTTCCGCGCACATTTCCCCGAAAAGTGCCACCTGACGTCG
ACGGATCGGGAGATCTcggccgcagcgatcgcatccatggcctccgcgaccggctgcagaacagcgggcagtt
cggtttcaggCaggtcttgcaacgtgacaccctgtgcacggcgggagatgcaataggtcaggctctcgctgaattccccaatgtc
aagcacttccggaatcgggagcgcggccgatgcaaagtgccgataaacataacgatctttgtagaaaccatcggcgcagctattt
acccgcaggacatatccacgccctcctacatcgaagctgaaagcacgagattcttcgccctccgagagctgcatcaggtcggag
acgctgtcgaacttttcgatcagaaacttctcgacagacgtcgcggtgagttcaggcttaattaagtacgggcctccaaaaaagcct
cctcactacttctggaatagctcagaggcagaggcggcctcggcctctgcataaataaaaaaaattagtcagccatggggcgga
gaatgggcggaactgggcggagttaggggcgggatgggcggagttaggggcgggactatggttgctgactaattgagatgcat
gctttgcatacttctgcctgctggggagcctggggactttccacacctggttgctgactaattgagatgcatgctttgcatacttctgc
ccgcggagttattaatagtaatcaattacggggtcattagttcatagcccatatatggagttccgcgttacataacttacggtaaatgg
cccgcctggctgaccgcccaacgacccccgcccattgacgtcaataatgacgtatgttcccatagtaacgccaatagggactttc
cattgacgtcaatgggtggagtatttacggtaaactgcccacttggcagtacatcaagtgtatcatatgccaagtacgccccctattg
acgtcaatgacggtaaatggcccgcctggcattatgcccagtacatgaccttatgggactttcctacttggcagtacatctacgtatt
agtcatcgctattaccatggtgatgcggttttggcagtacatcaatgggcgtggatagcggtttgactcacggggatttccaagtctc
caccccattgacgtcaatgggagtttgttttggcaccaaaatcaacgggactttccaaaatgtcgtaacaactccgccccattgacg
caaatgggcggtaggcgtgtacggtgggaggtctatataagcagagctgggtacgtgaaccgtcagatcgcctggagacgcca
tcacactagtcaccatgagggtccccgctcagctcctggggctcctgctgctctggctcccaggtgcacgatgtgacatccagat
gacccagtccccctcctccctgtctgcctccgtgggcgacagagtgaccatcacctgtcgggcctcccaggatgtgaacaccgc
cgtggcctggtatcagcagaagcctggcaaggcccctaagctgctgatctactccgcctccttcctgtactccggcgtgccctccc
ggttctccggctccagatccggcaccgacttcaccctgaccatctccagcctgcagcctgaggacttcgccacctactactgcca
gcagcactacaccacccctccaaccttcggccagggcaccaaggtggagatcaagcgtacggtggctgcaccatctgtcttcat
cttcccgccatctgatgagcagttgaaatctggaactgcctctgttgtgtgcctgctgaataacttctatcccagagaggccaaagta
cagtggaaggtggataacgccctccaatcgggtaactcccaggagagtgtcacagagcaggacagcaaggacagcacctaca
gcctcagcagcaccctgacgctgagcaaagcagactacgagaaacacaaagtctacgcctgcgaagtcacccatcagggcct
gagctcgcccgtcacaaagagcttcaacaggggagagtgttaataacctgcaggtcactgtgccttctagttgccagccatctgtt
gtttgcccctcccccgtgccttccttgaccctggaaggtgccactcccactgtcctttcctaataaaatgaggaaattgcatcgcatt
gtctgagtaggtgtcattctattctggggggtggggtggggcaggacagcaagggggaggattgggaagacaatagcaggcat
gctggggatgcggtggggtcgacatatgccaagtacgccccctattgacgtcaatgacggtaaatggcccgcctggcattatgcc
cagtacatgaccttatgggactttcctacttggcagtacatctacgtattagtcatcgctattaccatggtgatgcggttttggcagtac
atcaatgggcgtggatagcggtttgactcacggggatttccaagcctccaccccattgacgtcaatgggagtttgttttggcaccaa
aatcaacgggactttccaaaatgtcgtaacaactccgccccattgacgcaaatgggcggtaggcgtgtacggtgggaggtctata

taagcagagctgggtacgtcctcacattcagtgatcagcactgaacacaggatatccaccatgggttggagcctcatcttgctcttc
cttgtcgctgttgctacgcgtgtccactccgaagtgcagctggtggagtctggcggaggactggtgcagccaggcggatccctg
agactgtcttgtgccgcctccggcttcaacatcaaggacacctacatccactgggtccgccaggcaccaggcaagggactgga
atgggtggcccggatctaccctaccaacggctacacccgatacgccgactccgtgaagggccggttcaccatctccgccgaca
cctccaagaacaccgcctacctgcagatgaattccctgagggccgaggacaccgccgtgtactactgctccagatggggaggc
gacggcttctacgccatggactactggggccagggcaccctggtcacagtgtcctctgctagcaccaagggcccatcggtcttc
cccctggcaccctcctccaagagcacctctgggggcacagcggccctgggctgcctggtcaaggactacttccccgaaccggt
gacggtgtcgtggaactcaggcgccctgaccagcggcgtgcacaccttcccggctgtcctacagtcctcaggactctactccctc
agcagcgtggtgaccgtgccctccagcagcttgggcacccagacctacatctgcaacgtgaatcacaagcccagcaacaccaa
ggtggacaagaaagttgagcccaaatcttgtgacaaaactcacacatgcccaccgtgcccagcacctgaactcctggggggac
cgtcagtcttcctcttccccccaaaacccaaggacaccctcatgatctcccggacccctgaggtcacatgcgtggtggtggacgt
gagccacgaagaccctgaggtcaagttcaactggtacgtggacggcgtggaggtgcataatgccaagacaaagccgcgggag
gagcagtacaacagcacgtaccgtgtggtcagcgtcctcaccgtcctgcaccaggactggctgaatggcaaggagtacaagtg
caaggtctccaacaaagccctcccagcccccatcgagaaaaccatctccaaagccaaagggcagccccgagaaccacaggtg
tacaccctgcccccatcccgggatgagctgaccaagaaccaggtcagcctgacctgcctggtcaaaggcttctatcccagcgac
atcgccgtggagtgggagagcaatgggcagccggagaacaactacaagaccacgcctcccgtgctggactccgacggctcct
tcttcctctacagcaagctcaccgtggacaagagcaggtggcagcaggggaacgtcttctcatgctccgtgatgcatgaggctct
gcacaaccactacacgcagaagagcctctccctgtctccgggtTCTtaaGCGGCCGCTCGAGTCTAGAGG
GCCCGTTTAAACCCGCTGATCAGCCTCGACTGTGCCTTCTAGTTGCCAGCCAT
CTGTTGTTTGCCCCTCCCCCGTGCCTTCCTTGACCCTGGAAGGTGCCACTCCCA
CTGTCCTTTCCTAATAAAATGAGGAAATTGCATCGCATTGTCTGAGTAGGTGT
CATTCTATTCTGGGGGGTGGGGTGGGGCAGGACAGCAAGGGGGAGGATTGGG
AAGACAATAGCAGGCATGCTGGGGATGCGGTGGGCTCTATGGCTTCTGAGGC
GGAAAGAACCAGCTGGGGCTCTAGGGGGTATCCCCACGCGCCCTGTAGCGGC
GCATTAAGCGCGGCGGGTGTGGTGGTTACGCGCAGCGTGACCGCTACACTTGC
CAGCGCCCTAGCGCCCGCTCCTTTCGCTTTCTTCCCTTCCTTTCTCGCCACGTT
CGCCGGCTTTCCCCGTCAAGCTCTAAATCGGGGGCTCCCTTTAGGGTTCCGAT
TTAGTGCTTTACGGCACCTCGACCCCAAAAAACTTGATTAGGGTGATGGTTCA
CGTAGTGGGCCATCGCCCTGATAGACGGTTTTTCGCCCTTTGACGTTGGAGTC
CACGTTCTTTAATAGTGGACTCTTGTTCCAAACTGGAACAACACTCAACCCTA
TCTCGGTCTATTCTTTTGATTTATAAGGGATTTTGCCGATTTCGGCCTATTGGT
TAAAAAATGAGCTGATTTAACAAAAATTTAACGCGAATTAATTCTGTGGAATG
TGTGTCAGTTAGGGTGTGGAAAGTCCCCAGGCTCCCCAGCAGGCAGAAGTAT

GCAAAGCATGCATCTCAATTAGTCAGCAACCAGGTGTGGAAAGTCCCCAGGC
TCCCCAGCAGGCAGAAGTATGCAAAGCATGCATCTCAATTAGTCAGCAACCA
TAGTCCCGCCCCTAACTCCGCCCATCCCGCCCCTAACTCCGCCCAGTTCCGCC
CATTCTCCGCCCCATGGCTGACTAATTTTTTTTATTTATGCAGAGGCCGAGGCC
GCCTCTGCCTCTGAGCTATTCCAGAAGTAGTGAGGAGGCTTTTTTGGAGGCCT
AGGCTTTTGCAAAAAGCTCCCGGGAGCTTGTATATCCATTTTCGGATCTGATC
AAGAGACAGGATGAGGATCGTTTCGCATGATTGAACAAGATGGATTGCACGC
AGGTTCTCCGGCCGCTTGGGTGGAGAGGCTATTCGGCTATGACTGGGCACAAC
AGACAATCGGCTGCTCTGATGCCGCCGTGTTCCGGCTGTCAGCGCAGGGGCGC
CCGGTTCTTTTTGTCAAGACCGACCTGTCCGGTGCCCTGAATGAACTGCAGGA
CGAGGCAGCGCGGCTATCGTGGCTGGCCACGACGGGCGTTCCTTGCGCAGCT
GTGCTCGACGTTGTCACTGAAGCGGGAAGGGACTGGCTGCTATTGGGCGAAG
TGCCGGGGCAGGATCTCCTGTCATCTCACCTTGCTCCTGCCGAGAAAGTATCC
ATCATGGCTGATGCAATGCGGCGGCTGCATACGCTTGATCCGGCTACCTGCCC
ATTCGACCACCAAGCGAAACATCGCATCGAGCGAGCACGTACTCGGATGGAA
GCCGGTCTTGTCGATCAGGATGATCTGGACGAAGAGCATCAGGGGCTCGCGC
CAGCCGAACTGTTCGCCAGGCTCAAGGCGCGCATGCCCGACGGCGAGGATCT
CGTCGTGACCCATGGCGATGCCTGCTTGCCGAATATCATGGTGGAAAATGGCC
GCTTTTCTGGATTCATCGACTGTGGCCGGCTGGGTGTGGCGGACCGCTATCAG
GACATAGCGTTGGCTACCCGTGATATTGCTGAAGAGCTTGGCGGCGAATGGG
CTGACCGCTTCCTCGTGCTTTACGGTATCGCCGCTCCCGATTCGCAGCGCATC
GCCTTCTATCGCCTTCTTGACGAGTTCTTCTGAGCGGGACTCTGGGGTTCGAA
ATGACCGACCAAGCGACGCCCAACCTGCCATCACGAGATTTCGATTCCACCGC
CGCCTTCTATGAAAGGTTGGGCTTCGGAATCGTTTTCCGGGACGCCGGCTGGA
TGATCCTCCAGCGCGGGGATCTCATGCTGGAGTTCTTCGCCCACCCCAACTTG
TTTATTGCAGCTTATAATGGTTACAAATAAAGCAATAGCATCACAAATTTCAC
AAATAAAGCATTTTTTTCACTGCATTCTAGTTGTGGTTTGTCCAAACTCATCAA
TGTATCTTATCATGTCTGTATACCGTCGACCTCTAGCTAGAGCTTGGCGTAATC
ATGGTCATAGCTGTTTCCTGTGTGAAATTGTTATCCGCTCACAATTCCACACAA
CATACGAGCCGGAAGCATAAAGTGTAAAGCCTGGGGTGCCTAATGAGTGAGC
TAACTCACATTAATTGCGTTGCGCTCACTGCCCGCTTTCCAGTCGGGAAACCT
GTCGTGCCAGCTGCATTAATGAATCGGCCAACGCGCGGGGAGAGGCGGTTTG
CGTATTGGGCGCTCTTCCGCTTCCTCGCTCACTGACTCGCTGCGCTCGGTCGTT

CGGCTGCGGCGAGCGGTATCAGCTCACTCAAAGGCGGTAATACGGTTATCCA
CAGAATCAGGGGATAACGCAGGAAAGAACATGTGAGCAAAAGGCCAGCAAA
AGGCCAGGAACCGTAAAAAGGCCGCGTTGCTGGCGTTTTTCCATAGGCTCCGC
CCCCCTGACGAGCATCACAAAAATCGACGCTCAAGTCAGAGGTGGCGAAACC
CGACAGGACTATAAAGATACCAGGCGTTTCCCCCTGGAAGCTCCCTCGTGCGC
TCTCCTGTTCCGACCCTGCCGCTTACCGGATACCTGTCCGCCTTTCTCCCTTCG
GGAAGCGTGGCGCTTTCTCATAGCTCACGCTGTAGGTATCTCAGTTCGGTGTA
GGTCGTTCGCTCCAAGCTGGGCTGTGTGCACGAACCCCCCGTTCAGCCCGACC
GCTGCGCCTTATCCGGTAACTATCGTCTTGAGTCCAACCCGGTAAGACA

### Light chain of Trastuzumab

### Heavy chain of Trastuzumab (bolded and underlined GCT was mutated to TGA for 5HTP introduction)

FIG. 17 shows the plasmid map of pAcBac1_EcWRS1-5HTP-H14_RevU6EcW_TGA.

### Sequence: pAcBac1_EcWRS1-5HTP-H14_RevU6EcW_TGA (SEQ ID NO:2)

ttctctgtcacagaatgaaaatttttctgtcatctcttcgttattaatgtttgtaattgactgaatatcaacgcttatttgca
gcctgaatggcgaatgggacgcgccctgtagcggcgcattaagcgcggcgggtgtggtggttacgcgcagcgtgaccgctac
acttgccagcgccctagcgcccgctcctttcgctttcttcccttcctttctcgccacgttcgccggctttccccgtcaagctctaaatc
gggggctccctttagggttccgatttagtgctttacggcacctcgaccccaaaaaacttgattagggtgatggttcacgtagtgggc
catcgccctgatagacggtttttcgccctttgacgttggagtccacgttctttaatagtggactcttgttccaaactggaacaacactc
aaccctatctcggtctattcttttgatttataagggattttgccgatttcggcctattggttaaaaaatgagctgatttaacaaaaatttaa
cgcgaattttaacaaaatattaacgtttacaatttcaggtggcacttttcggggaaatgtgcgcggaacccctatttgtttatttttctaa
atacattcaaatatgtatccgctcatgagacaataaccctgataaatgcttcaataatattgaaaaaggaagagtatgagtattcaaca
tttccgtgtcgcccttattcccttttttgcggcattttgccttcctgtttttgctcacccagaaacgctggtgaaagtaaaagatgctgaa
gatcagttgggtgcacgagtgggttacatcgaactggatctcaacagcggtaagatccttgagagttttcgccccgaagaacgtttt
ccaatgatgagcacttttaaagttctgctatgtggcgcggtattatcccgtattgacgccgggcaagagcaactcggtcgccgcata
cactattctcagaatgacttggttgagtactcaccagtcacagaaaagcatcttacggatggcatgacagtaagagaattatgcagt
gctgccataaccatgagtgataacactgcggccaacttacttctgacaacgatcggaggaccgaaggagctaaccgcttttttgca
caacatgggggatcatgtaactcgccttgatcgttgggaaccggagctgaatgaagccataccaaacgacgagcgtgacaccac
gatgcctgtagcaatggcaacaacgttgcgcaaactattaactggcgaactacttactctagcttcccggcaacaattaatagactg
gatggaggcggataaagttgcaggaccacttctgcgctcggcccttccggctggctggtttattgctgataaatctggagccggtg

agcgtgggtctcgcggtatcattgcagcactggggccagatggtaagccctcccgtatcgtagttatctacacgacggggagtca
ggcaactatggatgaacgaaatagacagatcgctgagataggtgcctcactgattaagcattggtaactgtcagaccaagtttact
catatatactttagattgatttaaaacttcatttttaatttaaaaggatctaggtgaagatcctttttgataatctcatgaccaaaatccctta
acgtgagttttcgttccactgagcgtcagaccccgtagaaaagatcaaaggatcttcttgagatcctttttttctgcgcgtaatctgctg
cttgcaaacaaaaaaaccaccgctaccagcggtggtttgtttgccggatcaagagctaccaactctttttccgaaggtaactggctt
cagcagagcgcagataccaaatactgtccttctagtgtagccgtagttaggccaccacttcaagaactctgtagcaccgcctacat
acctcgctctgctaatcctgttaccagtggctgctgccagtggcgataagtcgtgtcttaccgggttggactcaagacgatagttac
cggataaggcgcagcggtcgggctgaacggggggttcgtgcacacagcccagcttggagcgaacgacctacaccgaactga
gatacctacagcgtgagcattgagaaagcgccacgcttcccgaagggagaaaggcggacaggtatccggtaagcggcagggt
cggaacaggagagcgcacgagggagcttccagggggaaacgcctggtatctttatagtcctgtcgggtttcgccacctctgactt
gagcgtcgatttttgtgatgctcgtcaggggggcggagcctatggaaaaacgccagcaacgcggcctttttacggttcctggcctt
ttgctggccttttgctcacatgttctttcctgcgttatcccctgattctgtggataaccgtattaccgcctttgagtgagctgataccgctc
gccgcagccgaacgaccgagcgcagcgagtcagtgagcgaggaagcggaagagcgcctgatgcggtattttctccttacgcat
ctgtgcggtatttcacaccgcagaccagccgcgtaacctggcaaaatcggttacggttgagtaataaatggatgccctgcgtaag
cgggtgtgggcggacaataaagtcttaaactgaacaaaatagatctaaactatgacaataaagtcttaaactagacagaatagttgt
aaactgaaatcagtccagttatgctgtgaaaaagcatactggacttttgttatggctaaagcaaactcttcattttctgaagtgcaaatt
gcccgtcgtattaaagaggggcgtggccaagggcatggtaaagactatattcgcggcgttgtgacaatttaccgaacaactccgc
ggccgggaagccgatctcggcttgaacgaattgttaggtggcggtacttgggtcgatatcaaagtgcatcacttcttcccgtatgcc
caactttgtatagagagccactgcgggatcgtcaccgtaatctgcttgcacgtagatcacataagcaccaagcgcgttggcctcat
gcttgaggagattgatgagcgcggtggcaatgccctgcctccggtgctcgccggagactgcgagatcatagatatagatctcact
acgcggctgctcaaacctgggcagaacgtaagccgcgagagcgccaacaaccgcttcttggtcgaaggcagcaagcgcgatg
aatgtcttactacggagcaagttcccgaggtaatcggagtccggctgatgttgggagtaggtggctacgtctccgaactcacgac
cgaaaagatcaagagcagcccgcatggatttgacttggtcagggccgagcctacatgtgcgaatgatgcccatacttgagccac
ctaactttgttttagggcgactgccctgctgcgtaacatcgttgctgctgcgtaacatcgttgctgctccataacatcaaacatcgacc
cacggcgtaacgcgcttgctgcttggatgcccgaggcatagactgtacaaaaaaacagtcataacaagccatgaaaaccgccac
tgcgccgttaccaccgctgcgttcggtcaaggttctggaccagttgcgtgagcgcatacgctacttgcattacagtttacgaaccga
acaggcttatgtcaactgggttcgtgccttcatccgtttccacggtgtgcgtcacccggcaaccttgggcagcagcgaagtcgag
gcatttctgtcctggctggcgaacgagcgcaaggtttcggtctccacgcatcgtcaggcattggcggccttgctgttcttctacggc
aaggtgctgtgcacggatctgccctggcttcaggagatcggtagacctcggccgtcgcggcgcttgccggtggtgctgaccccg
gatgaagtggttcgcatcctcggttttctggaaggcgagcatcgtttgttcgcccaggactctagctatagttctagtggttggctac
gtacccgtagtggctatggcagggcttgcgcttaatgcgccgctacagggcgcgtggggataccccctagagccccagctggtt
ctttccgcctcagaagccatagagcccaccgcatccccagcatgcctgctattgtcttcccaatcctcccccttgctgtcctgcccc
accccaccccccagaatagaatgacacctactcagacaatgcgatgcaatttcctcattttattaggaaaggacagtgggagtggc

accttccagggtcaaggaaggcacgggggaggggcaaacaacagatggctggcaactagaaggcacagtcgaggctgatca
gcgggtttaaacgggccctctagactcgagttaaagtcgacgcggggaggcggcccaaagggagatccgactcgtctgaggg
cgaaggcgaagacgcggaagaggccgcagagccggcagcaggccgcgggaaggaaggtccgctggattgagggccgaa
gggacgtagcagaaggacgtcccgcgcagaatccaggtggcaacacaggcgagcagccaaggaaaggacgatgatttcccc
gacaacaccacggaattgtcagtgcccaacagccgagcccctgtccagcagcgggcaaggcaggcggcgatgagttccgcc
gtggcaatagggagggggaaagcgaaagtcccggaaaggagctgacaggtggtggcaatgccccaaccagtgggggttgcg
tcagcaaacacagtgcacaccacgccacgttgcctgacaacgggccacaactcctcataaagagacagcaaccaggatttatac
aaggaggagaaaatgaaagccatacgggaagcaatagcatgatacaaaggcattaaagcagcgtatccacatagcgtaaaagg
agcaacatagttaagaataccagtcaatctttcacaaattttgtaatccagaggttgattgtcgacttaacgcgttgaattcTTACG
GCTTCGCCACAAAACCAATCGCTTCGTACACCGCTTTTAGCGTACGGGAAGCG
TGCGCGCTGGCTTTTTCCGCGCCATCTTTCATCACCTGTTGCAGGAAGGCTTCA
TCGTTGCGGAAACGGTGATAGCGTTCCTGCAATTCAGTCAGCATACCGGAAAC
GGCATCAGCCACTTCACCTTTCAGATGACCATACATCTTGCCTTCGAACTGTTT
TTCCAGTTCTGGGATGCTCTGGCCCGTTACCGCTGAAAGGATATCCAACAGGT
TGGAAACGCCCGCTTTGTTCTGCACATCGTAGCGAACTACCGGCGGCTCGTCG
GAGTCAGTGACCGCACGTTTGATTTTCTTCACTACCGATTTCGGATCTTCCAGC
AGGCCGATAACGTTATTGCGATTATCGTCAGACTTGGACATCTTCTTGGTCGG
CTCCAGCAGCGACATTACGCGCGCGCCAGATTTCGGAATAAACGGCTCCGGC
ACCTTAAAGATCTCGCCATACAGCGCGTTGAAACGCTGGGCAATATCGCGGCT
CAGTTCGAGGTGCTGTTTCTGGTCTTCACCacaaggaccCAGATTAGTTTGATACA
GCAGGATGTCCGCTGCCATCAGCACCGGATAGTCAAACAGACCAGCGTTGAT
GTTCTCGGCATAACGCGCAGATTTATCTTTAAACTGCGTCATGCGACTCAGTT
CGCCGAAGTAGGTATAGCAGTTCAGTGCCCAGCCTAACTGTGCATGTTCCGGC
ACGTGGGACTGAACAAAAATGGTGCTTTTCTCAGGATCGATACCACAAGCCA
GATACAAGGCCAGCGTATCCAGCGTCGCTTTACGCAGCTTCTGTGCATCCTGG
CGCACGGTGATCGCGTGTTGGTCAACGATACAGTAAATGCAATGGTAGTCATC
CTGCATGTTTACCCACTGACGCAGCGCACCCATGTAGTTACCAATGGTCAATT
CACCTGAGGGCTGTGCGCCagcAAAAACGATGGGCTTAGTCATggtggcgctagccagct
tgggtctccctatagtgagtcgtattaatttcgataagccagtaagcagtgggttctctagttagccagagagctctgcttatatagac
ctcccaccgtacacgcctaccgcccatttgcgtcaatggggcggagttgttacgacattttggaaagtcccgttgattttggtgcca
aaacaaactcccattgacgtcaatggggtggagacttggaaatccccgtgagtcaaaccgctatccacgcccattgatgtactgcc
aaaaccgcatcaccatggtaatagcgatgactaatacgtagatgtactgccaagtaggaaagtcccataaggtcatgtactgggc
ataatgccaggcgggccatttaccgtcattgacgtcaatagggggcgtacttggcatatgatacacttgatgtactgccaagtggg

cagtttaccgtaaatagtccacccattgacgtcaatggaaagtccctattggcgttactatgggaacatacgtcattattgacgtcaat
gggcgggggtcgttgggcggtcagccaggcgggccatttaccgtaagttatgtaacgcggaactccatatatgggctatgaacta
atgaccccgtaattgattactattaataactagtcaataatcaatgtcaacgcgtatatctggcccgtacatcgcgaagcagcgcaaa
acGGATCCtgcaggtatttGCGGCCGCggtccgtatactccggaatattaatagatcatggagataattaaaatgataa
ccatctcgcaaataaataagtattttactgttttcgtaacagttttgtaataaaaaaacctataaatattccggattattcataccgtccca
ccatcgggcgcgAACTCCTAAAAAACCGCCACCatgaagtgccttttgtacttagcctttttattcattggggtg
aattgcaagttcaccatagtttttccacacaaccaaaaaggaaactggaaaaatgttccttctaattaccattattgcccgtcaagctc
agatttaaattggcataatgacttaataggcacagccttacaagtcaaaatgcccaagagtcacaaggctattcaagcagacggtt
ggatgtgtcatgcttccaaatgggtcactacttgtgatttccgctggtatggaccgaagtatataacacattccatccgatccttcact
ccatctgtagaacaatgcaaggaaagcattgaacaaacgaaacaaggaacttggctgaatccaggcttccctcctcaaagttgtg
gatatgcaactgtgacggatgccgaagcagtgattgtccaggtgactcctcaccatgtgctggttgatgaatacacaggagaatg
ggttgattcacagttcatcaacggaaaatgcagcaattacatatgccccactgtccataactctacaacctggcattctgactataag
gtcaaagggctatgtgattctaacctcatttccatggacatcaccttcttctcagaggacggagagctatcatccctgggaaaggag
ggcacagggttcagaagtaactactttgcttatgaaactggaggcaaggcctgcaaaatgcaatactgcaagcattggggagtca
gactcccatcaggtgtctggttcgagatggctgataaggatctctttgctgcagccagattccctgaatgcccagaagggtcaagt
atctctgctccatctcagacctcagtggatgtaagtctaattcaggacgttgagaggatcttggattattccctctgccaagaaacctg
gagcaaaatcagagcgggtcttccaatctctccagtggatctcagctatcttgctcctaaaaacccaggaaccggtcctgctttcac
cataatcaatggtaccctaaaatactttgagaccagatacatcagagtcgatattgctgctccaatcctctcaagaatggtcggaatg
atcagtggaactaccacagaaagggaactgtgggatgactgggcaccatatgaagacgtggaaattggacccaatggagttctg
aggaccagttcaggatataagtttcctttatacatgattggacatggtatgttggactccgatcttcatcttagctcaaaggctcaggt
gttcgaacatcctcacattcaagacgctgcttcgcaacttcctgatgatgagagtttattttttggtgatactgggctatccaaaaatcc
aatcgagcttgtagaaggttggttcagtagttggaaaagctctattgcctcttttttctttatcatagggttaatcattggactattcttggt
tctccgagttggtatccatctttgcattaaattaaagcacaccaagaaaagacagatttatacagacatagagatgaaccgacttgg
aaagtgataaggccaggccggccaagcttgtcgagaagtactagaggatcataatcagccataccacatttgtagaggttttactt
gctttaaaaaacctcccacacctccccctgaacctgaaacataaaatgaatgcaattgttgttgttaacttgtttattgcagcttataatg
gttacaaataaagcaatagcatcacaaatttcacaaataaagcatttttttcactgcattctagttgtggtttgtccaaactcatcaatgt
atcttatcatgtctggatctgatcactgcttgagcctagcAAAAAATGGCAGGGGCGGAGAGACTCGA
ACTCCCAaCACCCGGTTTTgaAGACCGGTGCTCTACCAATTGAACTACGCCCCT
GGTGTTTCGTCCTTTCCACAAGATATATAAAGCCAAGAAATCGAAATACTTTC
AAGTTACGGTAAGCATATGATAGTCCATTTTAAAACATAATTTTAAAACTGCA
AACTACCCAAGAAATTATTACTTTCTACGTCACGTATTTTGTACTAATATCTTT
GTGTTTACAGTCAAATTAATTCTAATTATCTCTCTAACAGCCTTGTATCGTATA
TGCAAATATGAAGGAATCATGGGAAATAGGCCCTCTTCCTGCCCGACCTAGga

gatccgaaccagataagtgaaatctagttccaaactattttgtcatttttaattttcgtattagcttacgacgctacacccagttcccatct
attttgtcactcttccctaaataatccttaaaaactccatttccacccctcccagttcccaactattttgtccgcccacagcggggcattt
ttcttcctgttatgtttttaatcaaacatcctgccaactccatgtgacaaaccgtcatcttcggctacttt

### EcWRS-H14

### EcW tRNA UCA (TGA suppressor)

FIG. 18 shows the plasmid map of pET22b-T5-5F7.

### Sequence: pET22b-T5-5F7 sequence (SEQ ID NO:3)

tggcgaatgggacgcgccctgtagcggcgcattaagcgcggcgggtgtggtggttacgcgcagcgtgaccgc
tacacttgccagcgccctagcgcccgctcctttcgctttcttcccttcctttctcgccacgttcgccggctttccccgtcaagctctaaa
tcgggggctccctttagggttccgatttagtgctttacggcacctcgaccccaaaaaacttgattagggtgatggttcacgtagtgg
gccatcgccctgatagacggtttttcgccctttgacgttggagtccacgttctttaatagtggactcttgttccaaactggaacaacac
tcaaccctatctcggtctattcttttgatttataagggattttgccgatttcggcctattggttaaaaaatgagctgatttaacaaaaattta
acgcgaattttaacaaaatattaacgtttacaatttcaggtggcacttttcggggaaatgtgcgcggaacccctatttgtttatttttcta
aatacattcaaatatgtatccgctcatgagacaataaccctgataaatgcttcaataatattgaaaaaggaagagtatgagtattcaac
atttccgtgtcgcccttattcccttttttgcggcattttgccttcctgtttttgctcacccagaaacgctggtgaaagtaaaagatgctga
agatcagttgggtgcacgagtgggttacatcgaactggatctcaacagcggtaagatccttgagagttttcgccccgaagaacgtt
ttccaatgatgagcacttttaaagttctgctatgtggcgcggtattatcccgtattgacgccgggcaagagcaactcggtcgccgca
tacactattctcagaatgacttggttgagtactcaccagtcacagaaaagcatcttacggatggcatgacagtaagagaattatgca
gtgctgccataaccatgagtgataacactgcggccaacttacttctgacaacgatcggaggaccgaaggagctaaccgcttttttg
cacaacatgggggatcatgtaactcgccttgatcgttgggaaccggagctgaatgaagccataccaaacgacgagcgtgacacc
acgatgcctgcagcaatggcaacaacgttgcgcaaactattaactggcgaactacttactctagcttcccggcaacaattaataga
ctggatggaggcggataaagttgcaggaccacttctgcgctcggcccttccggctggctggtttattgctgataaatctggagccg
gtgagcgtgggtctcgcggtatcattgcagcactggggccagatggtaagccctcccgtatcgtagttatctacacgacggggag
tcaggcaactatggatgaacgaaatagacagatcgctgagataggtgcctcactgattaagcattggtaactgtcagaccaagttt
actcatatatactttagattgatttaaaacttcatttttaatttaaaaggatctaggtgaagatcctttttgataatctcatgaccaaaatcc
cttaacgtgagttttcgttccactgagcgtcagaccccgtagaaaagatcaaaggatcttcttgagatcctttttttctgcgcgtaatct
gctgcttgcaaacaaaaaaaccaccgctaccagcggtggtttgtttgccggatcaagagctaccaactctttttccgaaggtaactg
gcttcagcagagcgcagataccaaatactgtccttctagtgtagccgtagttaggccaccacttcaagaactctgtagcaccgcct
acatacctcgctctgctaatcctgttaccagtggctgctgccagtggcgataagtcgtgtcttaccgggttggactcaagacgatag
ttaccggataaggcgcagcggtcgggctgaacggggggttcgtgcacacagcccagcttggagcgaacgacctacaccgaac
tgagatacctacagcgtgagctatgagaaagcgccacgcttcccgaagggagaaaggcggacaggtatccggtaagcggcag
ggtcggaacaggagagcgcacgagggagcttccagggggaaacgcctggtatctttatagtcctgtcgggtttcgccacctctg

acttgagcgtcgatttttgtgatgctcgtcaggggggcggagcctatggaaaaacgccagcaacgcggcctttttacggttcctgg
ccttttgctggccttttgctcacatgttctttcctgcgttatcccctgattctgtggataaccgtattaccgcctttgagtgagctgatacc
gctcgccgcagccgaacgaccgagcgcagcgagtcagtgagcgaggaagcggaagagcgcctgatgcggtattttctccttac
gcatctgtgcggtatttcacaccgcatatatggtgcactctcagtacaatctgctctgatgccgcatagttaagccagtatacactcc
gctatcgctacgtgactgggtcatggctgcgccccgacacccgccaacacccgctgacgcgccctgacgggcttgtctgctccc
ggcatccgcttacagacaagctgtgaccgtctccgggagctgcatgtgtcagaggttttcaccgtcatcaccgaaacgcgcgag
gcagctgcggtaaagctcatcagcgtggtcgtgaagcgattcacagatgtctgcctgttcatccgcgtccagctcgttgagtttctc
cagaagcgttaatgtctggcttctgataaagcgggccatgttaagggcggttttttcctgtttggtcactgatgcctccgtgtaaggg
ggatttctgttcatgggggtaatgataccgatgaaacgagagaggatgctcacgatacgggttactgatgatgaacatgcccggtt
actggaacgttgtgagggtaaacaactggcggtatggatgcggcgggaccagagaaaaatcactcagggtcaatgccagcgct
tcgttaatacagatgtaggtgttccacagggtagccagcagcatcctgcgatgcagatccggaacataatggtgcagggcgctga
cttccgcgtttccagactttacgaaacacggaaaccgaagaccattcatgttgttgctcaggtcgcagacgttttgcagcagcagtc
gcttcacgttcgctcgcgtatcggtgattcattctgctaaccagtaaggcaaccccgccagcctagccgggtcctcaacgacagg
agcacgatcatgcgcacccgtggggccgccatgccggcgataatggcctgcttctcgccgaaacgtttggtggcgggaccagt
gacgaaggcttgagcgagggcgtgcaagattccgaataccgcaagcgacaggccgatcatcgtcgcgctccagcgaaagcg
gtcctcgccgaaaatgacccagagcgctgccggcacctgtcctacgagttgcatgataaagaagacagtcataagtgcggcgac
gatagtcatgccccgcgcccaccggaaggagctgactgggttgaaggctctcaagggcatcggtcgagatcccggtgcctaat
gagtgagctaacttacattaattgcgttgcgctcactgcccgctttccagtcgggaaacctgtcgtgccagctgcattaatgaatcg
gccaacgcgcggggagaggcggtttgcgtattgggcgccagggtggtttttcttttcaccagtgagacgggcaacagctgattgc
ccttcaccgcctggccctgagagagttgcagcaagcggtccacgctggtttgccccagcaggcgaaaatcctgtttgatggtggt
taacggcgggatataacatgagctgtcttcggtatcgtcgtatcccactaccgagatatccgcaccaacgcgcagcccggactcg
gtaatggcgcgcattgcgcccagcgccatctgatcgttggcaaccagcatcgcagtgggaacgatgccctcattcagcatttgca
tggtttgttgaaaaccggacatggcactccagtcgccttcccgttccgctatcggctgaatttgattgcgagtgagatatttatgcca
gccagccagacgcagacgcgccgagacagaacttaatgggcccgctaacagcgcgatttgctggtgacccaatgcgaccaga
tgctccacgcccagtcgcgtaccgtcttcatgggagaaaataatactgttgatgggtgtctggtcagagacatcaagaaataacgc
cggaacattagtgcaggcagcttccacagcaatggcatcctggtcatccagcggatagttaatgatcagcccactgacgcgttgc
gcgagaagattgtgcaccgccgctttacaggcttcgacgccgcttcgttctaccatcgacaccaccacgctggcacccagttgat
cggcgcgagatttaatcgccgcgacaatttgcgacggcgcgtgcagggccagactggaggtggcaacgccaatcagcaacga
ctgtttgcccgccagttgttgtgccacgcggttgggaatgtaattcagctccgccatcgccgcttccactttttcccgcgttttcgcag
aaacgtggctggcctggttcaccacgcgggaaacggtctgataagagacaccggcatactctgcgacatcgtataacgttactgg
tttcacattcaccaccctgaattgactctcttccgggcgctatcatgccataccgcgaaaggttttgcgccattcgatggtgtccggg
atctcgacgctctcccttatgcgactcctgcattaggaagcagcccagtagtaggttgaggccgttgagcaccgccgccgcaagg
aatggtgcatgcaaggagatggcgcccaacagtcccccggccacggggcctgccaccatacccacgccgaaacaagcgctc

atgagcccgaagtggcgagcccgatcttccccatcggtgatgtcggcgatataggcgccagcaaccgcacctgtggcgccggt
gatgccggccacgatgcgtccggcgtagaggatcgagatctcgatcccgcgaaattaatacgactcactataggggaattgtga
gcggataacaattcccctctagagtttgacagcattatcatcgatctcgagaaatcataaaaaatttatttgctttgtgagcggataac
aattataatagattcaattgtgagcggataacaatttcacacagaattcattaaagaggagaaattacatATGGAAGTGCA
GCTGGTGGAAAGCGGCGGCCTTGTGCAGGCGGGCGGCAGCCTGCGCCTG
AGCTGCGCGGCGAGCGGCATTACCTTTAGCATTAACACGATGGGCTGGTATCG
CCAGGCGCCGGGCAAACAGCGCGAACTGGTGGCGCTGATTAGCAGCATTGGC
GATACCTATTATGCGGATAGCGTGAAAGGCCGCTTTTggATTAGCCGCGATAAC
GCGAAAAACACCGTGTATCTGCAAATGAACAGCCTGAAACCGGAAGATACCG
CGGTGTATTATTGCAAACGCTTTCGCACCGCGGCGCAGGGCACCGATTATTGG
GGCCAGGGCACCcaaGTGACCGTGAGCagcCATCACCACCACCATCACTAAaagctt
aattagctgagcttggactcctgttgatagatccagtaatgacctcagaactccatctggatttgttcagaacgctcggttgccgccg
ggcgttttttattggtgagaatccaagctagcttggcggcggccgcactcgagcaccaccaccaccaccactgagatccggctgc
taacaaagcccgaaaggaagctgagttggctgctgccaccgctgagcaataactagcataaccccttggggcctctaaacgggt
cttgaggggttttttgctgaaaggaggaactatatccggat

### 5F7 (AntiHer2 nanobody)

FIG. 19 shows the plasmid map of pET22b-Myoglobin-K99>TAG.

### Sequence pET22b-Myoglobin-K99>TAG (SEQ ID NO:4)

agatacctacagcgtgagctatgagaaagcgccacgcttcccgaagggagaaaggcggacaggtatccggta
agcggcagggtcggaacaggagagcgcacgagggagcttccagggggaaacgcctggtatctttatagtcctgtcgggtttcg
ccacctctgacttgagcgtcgatttttgtgatgctcgtcaggggggcggagcctatggaaaaacgccagcaacgcggcctttttac
ggttcctggccttttgctggccttttgctcacatgttctttcctgcgttatcccctgattctgtggataaccgtattaccgcctttgagtga
gctgataccgctcgccgcagccgaacgaccgagcgcagcgagtcagtgagcgaggaagcggaagagcgcctgatgcggtat
tttctccttacgcatctgtgcggtatttcacaccgcatatatggtgcactctcagtacaatctgctctgatgccgcatagttaagccagt
atacactccgctatcgctacgtgactgggtcatggctgcgccccgacacccgccaacacccgctgacgcgccctgacgggcttg
tctgctcccggcatccgcttacagacaagctgtgaccgtctccgggagctgcatgtgtcagaggttttcaccgtcatcaccgaaac
gcgcgaggcagctgcggtaaagctcatcagcgtggtcgtgaagcgattcacagatgtctgcctgttcatccgcgtccagctcgtt
gagtttctccagaagcgttaatgtctggcttctgataaagcgggccatgttaagggcggttttttcctgtttggtcactgatgcctccgt
gtaagggggatttctgttcatgggggtaatgataccgatgaaacgagagaggatgctcacgatacgggttactgatgatgaacat
gcccggttactggaacgttgtgagggtaaacaactggcggtatggatgcggcgggaccagagaaaaatcactcagggtcaatg
ccagcgcttcgttaatacagatgtaggtgttccacagggtagccagcagcatcctgcgatgcagatccggaacataatggtgcag
ggcgctgacttccgcgtttccagactttacgaaacacggaaaccgaagaccattcatgttgttgctcaggtcgcagacgttttgcag

cagcagtcgcttcacgttcgctcgcgtatcggtgattcattctgctaaccagtaaggcaaccccgccagcctagccgggtcctcaa
cgacaggagcacgatcatgcgcacccgtggggccgccatgccggcgataatggcctgcttctcgccgaaacgtttggtggcgg
gaccagtgacgaaggcttgagcgagggcgtgcaagattccgaataccgcaagcgacaggccgatcatcgtcgcgctccagcg
aaagcggtcctcgccgaaaatgacccagagcgctgccggcacctgtcctacgagttgcatgataaagaagacagtcataagtgc
ggcgacgatagtcatgccccgcgcccaccggaaggagctgactgggttgaaggctctcaagggcatcggtcgagatcccggt
gcctaatgagtgagctaacttacattaattgcgttgcgctcactgcccgctttccagtcgggaaacctgtcgtgccagctgcattaat
gaatcggccaacgcgcggggagaggcggtttgcgtattgggcgccagggtggtttttcttttcaccagtgagacgggcaacagc
tgattgcccttcaccgcctggccctgagagagttgcagcaagcggtccacgctggtttgccccagcaggcgaaaatcctgtttgat
ggtggttaacggcgggatataacatgagctgtcttcggtatcgtcgtatcccactaccgagatatccgcaccaacgcgcagcccg
gactcggtaatggcgcgcattgcgcccagcgccatctgatcgttggcaaccagcatcgcagtgggaacgatgccctcattcagc
atttgcatggtttgttgaaaaccggacatggcactccagtcgccttcccgttccgctatcggctgaatttgattgcgagtgagatattt
atgccagccagccagacgcagacgcgccgagacagaacttaatgggcccgctaacagcgcgatttgctggtgacccaatgcg
accagatgctccacgcccagtcgcgtaccgtcttcatgggagaaaataatactgttgatgggtgtctggtcagagacatcaagaaa
taacgccggaacattagtgcaggcagcttccacagcaatggcatcctggtcatccagcggatagttaatgatcagcccactgacg
cgttgcgcgagaagattgtgcaccgccgctttacaggcttcgacgccgcttcgttctaccatcgacaccaccacgctggcaccca
gttgatcggcgcgagatttaatcgccgcgacaatttgcgacggcgcgtgcagggccagactggaggtggcaacgccaatcagc
aacgactgtttgcccgccagttgttgtgccacgcggttgggaatgtaattcagctccgccatcgccgcttccactttttcccgcgtttt
cgcagaaacgtggctggcctggttcaccacgcgggaaacggtctgataagagacaccggcatactctgcgacatcgtataacgt
tactggtttcacattcaccaccctgaattgactctcttccgggcgctatcatgccataccgcgaaaggttttgcgccattcgatggtgt
ccgggatctcgacgctctcccttatgcgactcctgcattaggaagcagcccagtagtaggttgaggccgttgagcaccgccgcc
gcaaggaatggtgcatgcaaggagatggcgcccaacagtcccccggccacggggcctgccaccatacccacgccgaaacaa
gcgctcatgagcccgaagtggcgagcccgatcttccccatcggtgatgtcggcgatataggcgccagcaaccgcacctgtggc
gccggtgatgccggccacgatgcgtccggcgtagaggatcgagatctcgatcccgcgaaattaatacgactcactataggggaa
ttgtgagcggataacaattcccctctagagtttgacagcattatcatcgatctcgagaaatcataaaaaatttatttgctttgtgagcgg
ataacaattataatagattcaattgtgagcggataacaatttcacacagaattcattaaagaggagaaattacatATGGTTCT
GTCTGAAGGTGAATGGCAGCTGGTTCTGCATGTTTGGGCTAAAGTTGAAGCTG
ACGTCGCTGGTCATGGTCAGGACATCTTGATTCGACTGTTCAAATCTCATCCG
GAAACTCTGGAAAAATTCGATCGTTTCAAACATCTGAAAACTGAAGCTGAAA
TGAAAGCTTCTGAAGATCTGAAAAAACATGGTGTTACCGTGTTAACTGCCCTA
GGTGCTATCCTTAAGAAAAAAGGGCATCATGAAGCTGAGCTCAAACCGCTTG
CACAATCGCATGCTACTAAACATaagATCCCGATCAAATACCTGGAATTCATCT
CTGAAGCGATCATCCATGTTCTGCATTCTAGACATCCAGGTGACTTCGGTGCT
GACGCTCAGGGTGCTATGAACAAAGCTCTCGAGCTGTTCCGTAAAGATATCGC

TGCTAAGTACAAAGAACTGGGTTAgCAGGGTGGCTCGGGACATCATCACCATC
ACCATTAAGGTCTCaagcttaattagctgagcttggactcctgttgatagatccagtaatgacctcagaactccatctg
gatttgttcagaacgctcggttgccgccgggcgttttttattggtgagaatccaagctagcttggcggccgcactcgagcacc
accaccaccaccactgagatccggctgctaacaaagcccgaaaggaagctgagttggctgctgccaccgctgagcaataacta
gcataaccccttggggcctctaaacgggtcttgaggggttttttgctgaaaggaggaactatatccggattggcgaatgggacgc
gccctgtagcggcgcattaagcgcggcgggtgtggtggttacgcgcagcgtgaccgctacacttgccagcgccctagcgcccg
ctcctttcgctttcttcccttcctttctcgccacgttcgccggctttccccgtcaagctctaaatcgggggctccctttagggttccgatt
tagtgctttacggcacctcgaccccaaaaaacttgattagggtgatggttcacgtagtgggccatcgccctgatagacggtttttcg
ccctttgacgttggagtccacgttctttaatagtggactcttgttccaaactggaacaacactcaaccctatctcggtctattcttttgatt
tataagggattttgccgatttcggcctattggttaaaaaatgagctgatttaacaaaaatttaacgcgaattttaacaaaatattaacgtt
tacaatttcaggtggcacttttcggggaaatgtgcgcggaacccctatttgtttatttttctaaatacattcaaatatgtatccgctcatg
agacaataaccctgataaatgcttcaataatattgaaaaaggaagagtatgagtattcaacatttccgtgtcgcccttattcccttttttg
cggcattttgccttcctgtttttgctcacccagaaacgctggtgaaagtaaaagatgctgaagatcagttgggtgcacgagtgggtt
acatcgaactggatctcaacagcggtaagatccttgagagttttcgccccgaagaacgttttccaatgatgagcacttttaaagttct
gctatgtggcgcggtattatcccgtattgacgccgggcaagagcaactcggtcgccgcatacactattctcagaatgacttggttg
agtactcaccagtcacagaaaagcatcttacggatggcatgacagtaagagaattatgcagtgctgccataaccatgagtgataac
actgcggccaacttacttctgacaacgatcggaggaccgaaggagctaaccgcttttttgcacaacatgggggatcatgtaactcg
ccttgatcgttgggaaccggagctgaatgaagccataccaaacgacgagcgtgacaccacgatgcctgcagcaatggcaacaa
cgttgcgcaaactattaactggcgaactacttactctagcttcccggcaacaattaatagactggatggaggcggataaagttgca
ggaccacttctgcgctcggcccttccggctggctggtttattgctgataaatctggagccggtgagcgtgggtctcgcggtatcatt
gcagcactggggccagatggtaagccctcccgtatcgtagttatctacacgacggggagtcaggcaactatggatgaacgaaat
agacagatcgctgagataggtgcctcactgattaagcattggtaactgtcagaccaagtttactcatatatactttagattgatttaaaa
cttcatttttaatttaaaaggatctaggtgaagatcctttttgataatctcatgaccaaaatcccttaacgtgagttttcgttccactgagc
gtcagaccccgtagaaaagatcaaaggatcttcttgagatcctttttttctgcgcgtaatctgctgcttgcaaacaaaaaaaccaccg
ctaccagcggtggtttgtttgccggatcaagagctaccaactctttttccgaaggtaactggcttcagcagagcgcagataccaaat
actgtccttctagtgtagccgtagttaggccaccacttcaagaactctgtagcaccgcctacatacctcgctctgctaatcctgttacc
agtggctgctgccagtggcgataagtcgtgtcttaccgggttggactcaagacgatagttaccggataaggcgcagcggtcggg
ctgaacggggggttcgtgcacacagcccagcttggagcgaacgacctacaccgaactg

### Myoglobin

Thus, the present disclosure establishes a unique electrochemical chemoselective conjugation reaction to site-specifically modify proteins expressed in both bacteria and eukaryotes. The electrocatalysis reactions are highly scalable, rapid (taking less than about 30 minutes to about 2 hours, and highly selective for the targeted unnatural amino acid.

While this invention has been particularly shown and described with references to preferred embodiments thereof, it will be understood by those skilled in the art that various changes in form and details may be made therein without departing from the scope of the invention encompassed by the appended claims.

### References:

The following references are herein incorporated by reference in their entirety.
1. Faraday, M., Siebente Reihe von Experimental-Untersuchungen über Elektricität. Annalen der Physik 1834, 109 (31-34), 481-520.
2. Kolbe, H., Beobachtungen über die oxydirende Wirkung des Sauerstoffs, wenn derselbe mit Hülfe einer elektrischen Säule entwickelt wird. Journal für praktische Chemie 1847, 41 (1), 137-139.
3. Horn, E. J.; Rosen, B. R.; Baran, P. S., Synthetic Organic Electrochemistry: An Enabling and Innately Sustainable Method. ACS Central Science 2016, 2 (5), 302-308.
4. Yan, M.; Kawamata, Y.; Baran, P. S., Synthetic Organic Electrochemical Methods Since 2000: On the Verge of a Renaissance. Chemical Reviews 2017, 117 (21), 13230-13319.
5. Little, R. D., A Perspective on Organic Electrochemistry. The Journal of Organic Chemistry 2020, 85 (21), 13375-13390.
6. Zhu, C.; Ang, N. W. J.; Meyer, T. H.; Qiu, Y.; Ackermann, L., Organic Electrochemistry: Molecular Syntheses with Potential ACS Central Science 2021, 7 (3), 415-431.
7. Spicer, C. D.; Davis, B. G., Selective chemical protein modification. Nature communications 2014, 5, ncomms5740.
8. Stephanopoulos, N.; Francis, M. B., Choosing an effective protein bioconjugation strategy. Nature chemical biology 2011, 7 (12), 876-84.
9. Boutureira, O.; Bernardes, G. a. J., Advances in chemical protein modification. Chemical reviews 2015, 115 (5), 2174-2195.
10. deGruyter, J. N.; Malins, L. R.; Baran, P. S., Residue-Specific Peptide Modification: A Chemist's Guide. Biochemistry 2017, 56 (30), 3863-3873.
11. Lang, K.; Chin, J. W., Bioorthogonal reactions for labeling proteins. ACS chemical biology 2014, 9 (1), 16-20.
12. McKay, C. S.; Finn, M. G., Click chemistry in complex mixtures: bioorthogonal bioconjugation. Chemistry & biology 2014, 21 (9), 1075-101.
13. Shih, H. W.; Kamber, D. N.; Prescher, J. A., Building better bioorthogonal reactions. Current opinion in chemical biology 2014, 21, 103-11.
14. Sletten, E. M.; Bertozzi, C. R., Bioorthogonal chemistry: fishing for selectivity in a sea of functionality. Angewandte Chemie (International ed. in English) 2009, 48 (38), 6974-98.
15. Kim, C. H.; Axup, J. Y.; Schultz, P. G., Protein conjugation with genetically encoded unnatural amino acids. Current opinion in chemical biology 2013, 17 (3), 412-9.
16. Scinto, S. L.; Bilodeau, D. A.; Hincapie, R.; Lee, W.; Nguyen, S. S.; Xu, M.; am Ende, C. W.; Finn, M. G.; Lang, K.; Lin, Q.; Pezacki, J. P.; Prescher, J. A.; Robillard, M. S.; Fox, J. M., Bioorthogonal chemistry. Nature Reviews Methods Primers 2021, 1 (1), 30.
17. Bloom, S.; Liu, C.; Kölmel, D. K.; Qiao, J. X.; Zhang, Y.; Poss, M. A.; Ewing, W. R.; MacMillan, D. W., Decarboxylative alkylation for site-selective bioconjugation of native proteins via oxidation potentials. Nature chemistry 2018, 10 (2), 205.
18. Lin, S.; Yang, X.; Jia, S.; Weeks, A. M.; Hornsby, M.; Lee, P. S.; Nichiporuk, R. V.; Iavarone, A. T.; Wells, J. A.; Toste, F. D.; Chang, C. J., Redox-based reagents for chemoselective methionine bioconjugation. Science 2017, 355 (6325), 597-602.
19. Patterson, D. M.; Prescher, J. A., Orthogonal bioorthogonal chemistries. Current opinion in chemical biology 2015, 28, 141-9.
20. Italia, J. S.; Addy, P. S.; Erickson, S. B.; Peeler, J. C.; Weerapana, E.; Chatterjee, A., Mutually Orthogonal Nonsense-Suppression Systems and Conjugation Chemistries for Precise Protein Labeling at up to Three Distinct Sites. Journal of the American Chemical Society 2019, 141 (15), 6204-6212.
21. Mackay, A. S.; Payne, R. J.; Malins, L. R., Electrochemistry for the Chemoselective Modification of Peptides and Proteins. Journal of the American Chemical Society 2012, 144 (1), 23-41.
22. Song, C.; Liu, K.; Wang, Z.; Ding, B.; Wang, S.; Weng, Y.; Chiang, C.-W.; Lei, A., Electrochemical oxidation induced selective tyrosine bioconjugation for the modification of biomolecules. Chemical science 2019, 10 (34), 7982-7987.
23. Alvarez-Dorta, D.; Thobie-Gautier, C.; Croyal, M.; Bouzelha, M.; Mével, M.; Deniaud, D.; Boujtita, M.; Gouin, S. G., Electrochemically Promoted Tyrosine-Click-Chemistry for Protein Labeling. Journal of the American Chemical Society 2018, 140 (49), 17120-17126.
24. Sato, S.; Matsumura, M.; Kadonosono, T.; Abe, S.; Ueno, T.; Ueda, H.; Nakamura, H., Site-Selective Protein Chemical Modification of Exposed Tyrosine Residues Using Tyrosine Click Reaction. Bioconjugate Chemistry 2020, 31 (5), 1417-1424.
25. Depienne, S.; Alvarez-Dorta, D.; Croyal, M.; Temgoua, R. C. T.; Charlier, C.; Deniaud, D.; Mével, M.; Boujtita, M.; Gouin, S. G., Luminol anchors improve the electrochemical-tyrosine-click labelling of proteins. Chem Sci 2021, 12 (46), 15374-15381.
26. Seki, Y.; Ishiyama, T.; Sasaki, D.; Abe, J.; Sohma, Y.; Oisaki, K.; Kanai, M., Transition Metal-Free Tryptophan-Selective Bioconjugation of Proteins. Journal of the American Chemical Society 2016, 138 (34), 10798-10801.
27. Toyama, E.; Maruyama, K.; Sugai, T.; Kondo, M.; Masaoka, S.; Saitoh, T.; Oisaki, K.; Kanai, M., Electrochemical Tryptophan-Selective Bioconjugation. ChemRxiv 2019, 10.26434/chemrxiv.7795484.v1.
28. Kendall, G.; Cooper, H. J; Heptinstall, J.; Derrick, P. J.; Walton, D. J.; Peterson, I. R., Specific electrochemical nitration of horse heart myoglobin. Arch Biochem Biophys 2001, 392 (2), 169-79.
29. Iniesta, J.; Cooper, H. J.; Marshall, A. G.; Heptinstall, J.; Walton, D. J.; Peterson, I. R., Specific electrochemical iodination of horse heart myoglobin at tyrosine 103 as determined by Fourier transform ion cyclotron resonance mass spectrometry. Archives of biochemistry and biophysics 2008, 474 (1), 1-7.
30. Baran, P. S.; Guerrero, C. A.; Corey, E. J., The First Method for Protection---Deprotection of the Indole 2,3-π Bond. Organic Letters 2003, 5 (11), 1999-2001.
31. Decoene, K.; Unal, K.; Staes, A.; Gevaert, K.; Winne, J. M.; Madder, A., Protein Conjugation with Triazolinediones: Switching from a General Tyrosine-Selective Labeling Method to a Highly Specific Tryptophan Bioconjugation Strategy. ChemRxiv 2021, 10.26434/chemrxiv.13739320.v1*.*
32. Italia, J. S.; Addy, P. S.; Wrobel, C. J.; Crawford, L. A.; Lajoie, M. J.; Zheng, Y.; Chatterjee, A., An orthogonalized platform for genetic code expansion in both bacteria and eukaryotes. Nature chemical biology 2017, 13 (4), 446-450.
33. White, A. M.; Palombi, I. R.; Malins, L. R., Umpolung strategies for the functionalization of peptides and proteins. Chem Sci 2022, 13 (10), 2809-2823.
34. Jewett, J. C.; Bertozzi, C. R., Cu-free click cycloaddition reactions in chemical biology. Chemical Society Reviews 2010, 39 (4), 1272-1279.
35. Devaraj, N. K.; Weissleder, R., Biomedical applications of tetrazine cycloadditions. Accounts of chemicalresearch 2011, 44 (9), 816-827.
36. Chin, J. W., Expanding and reprogramming the genetic code. Nature 2017, 550 (7674), 53.
37. Italia, J. S.; Zheng, Y.; Kelemen, R. E.; Erickson, S. B.; Addy, P. S.; Chatterjee, A., Expanding the genetic code of mammalian cells. Biochemical Society transactions 2017, 45 (2), 555-562.
38. Young, D. D.; Schultz, P. G., Playing with the molecules of life. ACS chemical biology 2018**.**
39. Addy, P. S.; Erickson, S. B.; Italia, J. S.; Chatterjee, A., A chemoselective rapid azo-coupling reaction (CRACR) for unclickable bioconjugation. Journal of the American Chemical Society 2017, 139 (34), 11670-11673.
40. Sarathi Addy, P.; Italia, J. S.; Chatterjee, A., An Oxidative Bioconjugation Strategy Targeted to a Genetically Encoded 5-Hydroxytryptophan. Chembiochem : a European journal of chemical biology 2018, 19 (13), 1375-1378.
41. Ficaretta, E. D.; Wrobel, C. J. J.; Roy, S. J. S.; Erickson, S. B.; Italia, J. S.; Chatterjee, A., A Robust Platform for Unnatural Amino Acid Mutagenesis in E. coli Using the Bacterial Tryptophanyl-tRNA synthetase/tRNA pair. Journal of molecular biology 2022, 434 (8), 167304.
42. Addy, P. S.; Zheng, Y; Italia, J. S.; Chatterjee, A., A "Quenchergenic" Chemoselective Protein Labeling Strategy. Chembiochem : a European journal of chemical biology 2019, 20 (13), 1659-1663.
43. Addy, P. S.; Italia, J. S.; Chatterjee, A., An Oxidative Bioconjugation Strategy Targeted to a Genetically Encoded 5-Hydroxytryptophan. Chembiochem : a European journal of chemical biology 2018, 19 (13), 1375-1378.
44. Napolitano, A.; d'Ischia, M.; Prota, G., A profile of the oxidation chemistry of 5-hydroxyindole under biomimetic conditions. Tetrahedron 1988, 44 (23), 7265-7270.
45. Wrona, M. Z.; Dryhurst, G., Oxidation of serotonin by superoxide radical: implications to neurodegenerative brain disorders. Chemical research in toxicology 1998, 11 (6), 639-650.
46. Yamagishi, Y.; Ashigai, H.; Goto, Y.; Murakami, H.; Suga, H., Ribosomal synthesis of cyclic peptides with a fluorogenic oxidative coupling reaction. Chembiochem : a European journal of chemical biology 2009, 10 (9), 1469-72.
47. Humphries, K. A.; Wrona, M. Z.; Dryburst, G., Electrochemical and enzymatic oxidation of 5-hydroxytryptophan. Journal of Electroanalytical Chemistry 1993, 346 (1), 377-403.
48. Ferguson, W. J., Braunschweiger, K. I.; Braunschweiger, W. R.; Smith, J. R.; McCormick, J. J.; Wasmann, C. C.; Jarvis, N. P.; Bell, D. H.; Good, N. E., Hydrogen ion buffers for biological research. Analytical biochemistry 1980, 104 (2), 300-10.
49. Grady, J. K.; Chasteen, N. D.; Harris, D. C., Radicals from "Good's" buffers. Analytical biochemistry 1988, 173 (1), 111-5.
50. Nutting, J. E.; Rafiee, M.; Stahl, S. S., Tetramethylpiperidine N-Oxyl (TEMPO), Phthalimide N-Oxyl (PINO), and Related N-Oxyl Species: Electrochemical Properties and Their Use in Electrocatalytic Reactions. Chem Rev 2018, 118 (9), 483-4-4885.
51. Sheikholeslami, F.; Rasaee, M. J.; Shokrgozar, M. A.; Dizaji, M. M.; Rahbarizadeh, F.; Ahmadvande, D., Isolation of a Novel Nanobody Against HER-2/neu Using Phage Displays Technology. Laboratory Medicine 2010, 41 (2), 69-76.
52. Drago, J. Z.; Modi, S.; Chandarlapaty, S., Unlocking the potential of antibody-drug conjugates for cancer therapy. Nature reviews. Clinical oncology 2021, 18 (6), 327-344.
53. Beck, A.; Goetsch, L.; Dumontet, C.; Corvaïa, N., Strategies and challenges for the next generation of antibody-drug conjugates. Nature reviews. Drug discovery 2017, 16 (5), 315-337.
54. Axup, J. Y.; Bajjuri, K. M.; Ritland, M.; Hutchins, B. M.; Kim, C. H.; Kazane, S. A.; Halder, R.; Forsyth, J. S.; Santidrian, A. F.; Stafin, K.; Lu, Y.; Tran, H.; Seller, A. J.; Biroc, S. L.; Szydlik, A.; Pinkstaff, J. K.; Tian, F.; Sinha, S. C.; Felding-Habermann, B.; Smider, V. V.; Schultz, P. G., Synthesis of site-specific antibody-drug conjugates using unnatural amino acids. Proceedings of the National Academy of Sciences of the United States of America 2012, 109 (40), 16101-6.
55. VanBrunt, M. P.; Shanebeck, K.; Caldwell, Z.; Johnson, J.; Thompson, P.; Martin, T.; Dong, H.; Li, G.; Xu, H.; D'Hooge, F.; Masterson, L.; Bariola, P.; Tiberghien, A.; Ezeadi, E.; Williams, D. G.; Hartley, J. A; Howard, P. W.; Grabstein, K. H.; Bowen, M. A.; Marelli, M., Genetically Encoded Azide Containing Amino Acid in Mammalian Cells Enables Site-Specific Antibody-Drug Conjugates Using Click Cycloaddition Chemistry. Bioconjugate Chemistry 2015, 26 (11), 2249-2260.
56. Agarwal, P.; Bertozzi, C. R., Site-specific antibody-drug conjugates: the nexus of bioorthogonal chemistry, protein engineering, and drug development. Bioconjugate chemistry 2015, 26 (2), 176-192.
57. Tian, F.; Lu, Y.; Manibusan, A.; Sellers, A.; Tran, H.; Sun, Y.; Phuong, T.; Barnett, R.; Hehli, B.; Song, F.; DeGuzman, M. J.; Ensari, S.; Pinkstaff, J. K.; Sullivan, L. M.; Biroc, S. L.; Cho, H.; Schultz, P. G.; DiJoseph, J.; Dougher, M.; Ma, D.; Dushin, R.; Leal, M.; Tchistiakova, L.; Feyfant, E.; Gerber, H. P.; Sapra, P., A general approach to site-specific antibody drug conjugates. Proceedings of the National Academy of Sciences of the United States of America 2014, 111 (5), 1766-71.
58. Chen, Y.; Tang, J.; Wang, L.; Tian, Z.; Cardenas, A.; Fang, X.; Chatterjee, A.; Xiao, H., Creation of Bacterial cells with 5-Hydroxytryptophan as a 21(st) Amino Acid Building Block. Chem 2020, 6 (10), 2717-2727.
59. Roy, G.; Reier, J.; Garcia, A.; Martin, T.; Rice, M.; Wang, J.; Prophet, M.; Christie, R.; Dall'Acqua, W.; Ahuja, S.; Bowen, M. A.; Marelli, M., Development of a high yielding expression platform for the introduction of non-natural amino acids in protein sequences. mAbs 2020, 12 (1), 1684749.
60. Lang, K.; Davis, L.; Torres-Kolbus, J.; Chou, C.; Deiters, A.; Chin, J. W., Genetically encoded norbornene directs site-specific cellular protein labelling via a rapid bioorthogonal reaction. Nat Chem 2012, 4 (4), 298-304.
61. Zheng, Y.; Addy, P. S.; Mukherjee, R.; Chatterjee, A., Defining the current scope and limitations of dual noncanonical amino acid mutagenesis in mammalian cells. Chem Sci 2017, 8 (10), 7211-7217.
62. Ficaretta, E. D.; Wrobel, C. J. J.; Roy, S. J. S.; Erickson, S. B.; Italia, J. S.; Chatterjee, A., A Robust Platform for Unnatural Amino Acid Mutagenesis in E. coli Using the Bacterial Tryptophanyl-tRNA synthetase/tRNA pair. Journal of molecular biology 2022, 434 (8), 167304.
63. Italia, J. S.; Addy, P. S.; Erickson, S. B.; Peeler, J. C.; Weerapana, E.; Chatterjee, A., Mutually Orthogonal Nonsense-Suppression Systems and Conjugation Chemistries for Precise Protein Labeling at up to Three Distinct Sites. Journal of the American Chemical Society 2019, 141 (15), 6204-6212.
64. Backliwal, G ; Hildinger, M.; Chenuet, S.; Wulhfard, S.; De Jesus, M.; Wurm, F. M., Rational vector design and multi-pathway modulation of HEK 293E cells yield recombinant antibody titers exceeding 1 g/l by transient transfection under serum-free conditions. Nucleic acids research 2008, 36 (15), e96.
65. Zhu, B.; Ge, J.; Yao, S. Q., Developing new chemical tools for DNA methyltransferase 1 (DNMT 1): A small-molecule activity-based probe and novel tetrazole-containing inhibitors. Bioorganic & Medicinal Chemistry 2015, 23 (12), 2917-2927.
66. Perron, V.; Abbott, S.; Moreau, N.; Lee, D.; Penney, C.; Zacharie, B., A Method for the Selective Protection of Aromatic Amines in the Presence of Aliphatic Amines. Synthesis 2009, 2009 (02), 283-289_{.}
67. Pheeney, C. G.; Barton, J. K., DNA Electrochemistry with Tethered Methylene Blue. Langmuir 2012, 28 (17), 7063-7070.

## Claims

1. A method of producing a chemoselectively modified protein bioconjugate, comprising the steps of:
a.) providing a recombinantly-expressed protein comprising one, or more non-canonical amino acid (ncAA) residues at specific sites of the protein, wherein one, or more, of the ncAA residues is a 5-hydroxytryptophan (5-HTP);
b.) selectively oxidizing the one, or more, 5-HTP amino acid residues of the protein under suitable conditions and at low voltage, wherein the voltage is sufficient to generate an electrophilic 5-HTP intermediate; and
c.) reacting the 5-HTP intermediate with a coupling partner comprising a nucleophilic group under conditions suitable for the coupling of the nucleophilic group to the one, or more, 5-HTP residues in the protein, thereby producing a chemoselectively modified protein bioconjugate.

2. The method of claim 1, wherein the protein is selectively modified at two sites, and wherein the other one, or more, non-canonical amino acid residues is an azide ncAA residue, preferably wherein the method further comprises coupling the azide ncAA residue with a coupling partner under conditions suitable for the coupling of the coupling partner to the azide ncAA, thereby producing a chemoselectively modified protein bioconjugate modified at two specific sites of the protein, more preferably wherein the coupling partner further comprises a biochemical or chemical cargo entity.

3. A method of producing a chemoselectively modified protein bioconjugate wherein the protein is selectively modified at two or more sites, the method comprising the steps of:
a.) providing a recombinantly-expressed protein comprising one, or more, non-canonical amino acid (ncAA) residues at specific sites of the protein, wherein one of the ncAA residues is a 5-hydroxytryptophan (5-HTP) and wherein one, or more, of the ncAA residues is an azide ncAA residue;
b.) selectively oxidizing the 5-HTP amino acid residue of the protein under suitable conditions and at low voltage, wherein the voltage is sufficient to generate an electrophilic 5-HTP intermediate;
c.) reacting the 5-HTP intermediate with a coupling partner comprising a nucleophilic group under conditions suitable for the coupling of the aromatic amine to the 5-HTP residues in the protein; and
d.) selectively coupling the azide ncAA residue with a coupling partner under conditions suitable for the coupling of the coupling partner to the azide ncAA residue,
thereby producing a chemoselectively modified protein bioconjugate, wherein the protein is selectively modified at two sites.

4. The method of any of claims 1 to 3, wherein the one, or more, ncAA residues are exposed on the surface of the protein.

5. The method of any of claims 1 to 3, wherein the protein is an antibody or antibody fragment.

6. The method of any of claims 1 to 3, wherein the oxidation voltage is between about 0.4 volts and about 1.0 volts, preferably wherein the oxidation voltage is between about 0.4 volts and about 0.8 volts, more preferably wherein the oxidation voltage is between about 0.7 and 0.8 volts.

7. The method of any of claims 1 to 3, wherein the oxidation step b) is facilitated by the addition of an electron transfer mediator, preferably wherein the electron transfer mediator is 2,2,6,6-tetramethylpiperidine-1-oxyl (TEMPO).

8. The method of any of claims 1 to 3, wherein the nucleophilic group of step c) is selected from the group consisting of: aromatic amines, anilines, indoles, and thiazoles

9. The method of any of claims 1 to 3, wherein the nucleophilic group of step c) further comprises a biochemical of chemical cargo entity, preferably wherein the cargo entity is selected from the group consisting of one, or more, of the following: small molecule, therapeutic drug, detectable label, detectable probe, polymer such as PEG, peptide, or nucleic acid, more preferably wherein the cargo entity comprises means to immobilize the bioconjugate to a solid support.

10. A method of electrochemically labeling a protein, comprising the steps of:
a.) providing a recombinantly-expressed protein comprising one, or more, non-canonical amino acid (ncAA) residues at specific sites of the protein, wherein one, or more, of the ncAA residues is a 5-hydroxytryptophan (5-HTP);
b.) selectively oxidizing the one, or more, 5-HTP amino acid residues of the protein under suitable conditions and at low voltage, wherein the voltage is sufficient to generate an electrophilic 5-HTP intermediate; and
c.) reacting the 5-HTP intermediate with a coupling partner comprising a nucleophilic group with a detectably-labeled cargo entity, under conditions suitable for the coupling of the aromatic amine to the one, or more, 5-HTP residues in the protein,
thereby producing a labelled protein.

11. The method of claim 10, wherein the one, or more, ncAA residues are exposed on the surface of the protein, preferably wherein the protein is an antibody or antibody fragment.

12. The method of claim 11, wherein the oxidation voltage is between about 0.4 volts and about 1.0 volts, preferably wherein the oxidation voltage is between about 0.4 volts and about 0.8 volts, more preferably wherein the oxidation voltage is between about 0.7 and 0.8 volts.

13. The method of claim 12, wherein the oxidation step comprises the addition of an electron transfer mediator, preferably wherein the electron transfer mediator is 2,2,6,6-tetramethylpiperidine-1-oxyl (TEMPO).

14. The method of claim 13, wherein the nucleophilic group is selected from the group consisting of: aromatic amines, anilines, indoles, and thiazoles.

15. The methods of any of claims 10 through 14, wherein the protein is selectively modified at two sites, and the other one, or more, non-canonical amino acid residues is an azide ncAA residue, preferably wherein the method further comprises coupling the azide ncAA residue with a coupling partner under conditions suitable for the coupling of the coupling partner to the azide ncAA, thereby producing a chemoselectively modified protein bioconjugate modified at two specific sites of the protein, more preferably wherein the coupling partner further comprises a biochemical or chemical cargo entity.

## Patentansprüche

1. Verfahren zum Herstellen eines chemoselektiv modifizierten Protein-Biokonjugats, umfassend die folgenden Schritte:
a.) Bereitstellen eines rekombinant exprimierten Proteins, umfassend einen oder mehrere Reste nicht-kanonischer Aminosäuren (ncAA) an spezifischen Stellen des Proteins, wobei einer oder mehrere der ncAA-Reste ein 5-Hydroxytryptophan (5-HTP) ist;
b.) selektives Oxidieren des einen oder der mehreren 5-HTP-Aminosäurereste des Proteins unter geeigneten Bedingungen und bei niedriger Spannung, wobei die Spannung ausreichend ist, um ein elektrophiles 5-HTP-Zwischenprodukts zu erzeugen; und
c.) Umsetzen des 5-HTP-Zwischenprodukts mit einem Kopplungspartner, der eine nucleophile Gruppe umfasst, unter für das Koppeln der nucleophilen Gruppe an den einen oder die mehreren 5-HTP-Reste in dem Protein geeigneten Bedingungen,
wodurch ein chemoselektiv modifiziertes Protein-Biokonjugat hergestellt wird.

2. Verfahren nach Anspruch 1, wobei das Protein selektiv an zwei Stellen modifiziert ist und wobei der eine andere oder die mehreren anderen nicht-kanonischen Aminosäurereste ein Azid-ncAA-Rest sind, wobei vorzugsweise das Verfahren ferner das Koppeln des Azid-ncAA-Rests mit einem Kopplungspartner unter für das Koppeln des Kopplungspartners an das Azid-ncAA geeigneten Bedingungen umfasst, wodurch ein chemoselektiv modifiziertes Protein-Biokonjugat hergestellt wird, das an zwei spezifischen Stellen des Proteins modifiziert ist, wobei noch bevorzugter der Kupplungspartner ferner eine biochemische oder chemische Ladungseinheit umfasst.

3. Verfahren zum Herstellen eines chemoselektiv modifizierten Protein-Biokonjugats, wobei das Protein selektiv an zwei oder mehr Stellen modifiziert ist, wobei das Verfahren die folgenden Schritte umfasst:
a.) Bereitstellen eines rekombinant exprimierten Proteins, umfassend einen oder mehrere Reste nicht-kanonischer Aminosäuren (ncAA) an spezifischen Stellen des Proteins, wobei einer der ncAA-Reste ein 5-Hydroxytryptophan (5-HTP) ist und wobei einer oder mehrere der ncAA-Reste ein Azid-ncAA-Rest sind;
b.) selektives Oxidieren des 5-HTP-Aminosäurerestes des Proteins unter geeigneten Bedingungen und bei niedriger Spannung, wobei die Spannung ausreichend ist, um ein elektrophiles 5-HTP-Zwischenprodukts zu erzeugen;
c.) Umsetzen des 5-HTP-Zwischenprodukts mit einem Kopplungspartner, der eine nukleophile Gruppe umfasst, unter für das Koppeln des aromatischen Amins an die 5-HTP-Reste in dem Protein geeigneten Bedingungen; und
d.) selektives Koppeln des Azid-ncAA-Restes mit einem Kopplungspartner unter für das Koppeln des Kopplungspartners an den Azid-ncAA-Rest geeigneten Bedingungen,
wodurch ein chemoselektiv modifiziertes Protein-Biokonjugat hergestellt wird, wobei das Protein selektiv an zwei Stellen modifiziert ist.

4. Verfahren nach einem der Ansprüche 1 bis 3, wobei der eine oder die mehreren ncAA-Reste auf der Oberfläche des Proteins freiliegen.

5. Verfahren nach einem der Ansprüche 1 bis 3, wobei das Protein ein Antikörper oder ein Antikörperfragment ist.

6. Verfahren nach einem der Ansprüche 1 bis 3, wobei die Oxidationsspannung zwischen etwa 0,4 Volt und etwa 1,0 Volt liegt, vorzugsweise wobei die Oxidationsspannung zwischen etwa 0,4 Volt und etwa 0,8 Volt liegt, noch bevorzugter, wobei die Oxidationsspannung zwischen etwa 0,7 und 0,8 Volt liegt.

7. Verfahren nach einem der Ansprüche 1 bis 3, wobei der Oxidationsschritt b) durch die Zugabe eines Elektronentransfervermittlers erleichtert wird, wobei der Elektronentransfervermittler vorzugsweise 2,2,6,6-Tetramethylpiperidin-1-oxyl (TEMPO) ist.

8. Verfahren nach einem der Ansprüche 1 bis 3, wobei die nukleophile Gruppe von Schritt c) ausgewählt ist aus der Gruppe bestehend aus: aromatischen Aminen, Anilinen, Indolen und Thiazolen.

9. Verfahren nach einem der Ansprüche 1 bis 3, wobei die nukleophile Gruppe von Schritt c) ferner ein biochemisches Element einer chemischen Ladungseinheit umfasst, vorzugsweise wobei die Ladungseinheit ausgewählt ist aus der Gruppe bestehend aus einem oder mehreren der folgenden: kleines Molekül, therapeutischer Wirkstoff, detektierbarer Marker, detektierbare Sonde, Polymer wie PEG, Peptid oder Nukleinsäure, noch bevorzugter, wobei die Ladungseinheit Mittel zum Immobilisieren des Biokonjugats an einem festen Träger umfasst.

10. Verfahren zum elektrochemischen Markieren eines Proteins, umfassend die folgenden Schritte:
a.) Bereitstellen eines rekombinant exprimierten Proteins, umfassend einen oder mehrere Reste nicht-kanonischer Aminosäuren (ncAA) an spezifischen Stellen des Proteins, wobei einer oder mehrere der ncAA-Reste ein 5-Hydroxytryptophan (5-HTP) sind;
b.) selektives Oxidieren des einen oder der mehreren 5-HTP-Aminosäurereste des Proteins unter geeigneten Bedingungen und bei niedriger Spannung, wobei die Spannung ausreichend ist, um ein elektrophiles 5-HTP-Zwischenprodukt zu erzeugen; und
c.) Umsetzen des 5-HTP-Zwischenprodukts mit einem Kopplungspartner, der eine nukleophile Gruppe mit einer nachweisbar markierten Ladungseinheit umfasst, unter für das Koppeln des aromatischen Amins an den einen oder die mehreren 5-HTP-Reste in dem Protein geeigneten Bedingungen,
wodurch ein markiertes Protein hergestellt wird.

11. Verfahren nach Anspruch 10, wobei der eine oder die mehreren ncAA-Reste auf der Oberfläche des Proteins freiliegen, wobei das Protein vorzugsweise ein Antikörper oder Antikörperfragment ist.

12. Verfahren nach Anspruch 11, wobei die Oxidationsspannung zwischen etwa 0,4 Volt und etwa 1,0 Volt liegt, vorzugsweise wobei die Oxidationsspannung zwischen etwa 0,4 Volt und etwa 0,8 Volt liegt, noch bevorzugter, wobei die Oxidationsspannung zwischen etwa 0,7 und 0,8 Volt liegt.

13. Verfahren nach Anspruch 12, wobei der Oxidationsschritt die Zugabe eines Elektronentransfervermittlers umfasst, wobei der Elektronentransfervermittler vorzugsweise 2,2,6,6-Tetramethylpiperidin-1-oxyl (TEMPO) ist.

14. Verfahren nach Anspruch 13, wobei die nucleophile Gruppe ausgewählt ist aus der Gruppe bestehend aus: aromatischen Aminen, Anilinen, Indolen und Thiazolen.

15. Verfahren nach einem der Ansprüche 10 bis 14, wobei das Protein selektiv an zwei Stellen modifiziert ist und der eine andere oder die mehreren anderen nicht-kanonischen Aminosäurereste ein Azid ncAA-Rest sind, wobei vorzugsweise das Verfahren ferner das Koppeln des Azid-ncAA-Rests mit einem Kopplungspartner unter für das Koppeln des Kopplungspartners an das Azid-ncAA geeigneten Bedingungen umfasst, wodurch ein chemoselektiv modifiziertes Protein-Biokonjugat hergestellt wird, das an zwei spezifischen Stellen des Proteins modifiziert ist, wobei noch bevorzugter der Kupplungspartner ferner eine biochemische oder chemische Ladungseinheit umfasst.

## Revendications

1. Procédé de production d'un bioconjugué de protéine modifiée de manière chimiosélective, comprenant les étapes consistant à :
a.) fournir une protéine exprimée de manière recombinante comprenant un, ou plusieurs résidus d'acide aminé non-canonique (ncAA) sur des sites spécifiques de la protéine, dans lequel un ou plusieurs des résidus ncAA est un 5-hydroxytryptophane (5-HTP) ;
b.) oxyder sélectivement les un ou plusieurs résidus d'acide aminé 5-HTP de la protéine dans des conditions adaptées et à basse tension, dans lequel la tension est suffisante pour générer un intermédiaire 5-HTP électrophile ; et
c.) faire réagir l'intermédiaire 5-HTP avec un partenaire de couplage comprenant un groupe nucléophile dans des conditions adaptées pour le couplage du groupe nucléophile aux un ou plusieurs résidus 5-HTP dans la protéine,
en produisant ainsi un bioconjugué de protéine modifiée de manière chimiosélective.

2. Procédé selon la revendication 1, dans lequel la protéine est sélectivement modifiée en deux sites, et dans lequel les un ou plusieurs autres résidus d'acide aminé non-canonique sont un résidu ncAA d'azide, de préférence dans lequel le procédé comprend en outre le couplage du résidu ncAA d'azide avec un partenaire de couplage dans des conditions adaptées pour le couplage du partenaire de couplage au ncAA d'azide, produisant ainsi un bioconjugué de protéine modifiée de manière chimiosélective, modifiée en deux sites spécifiques de la protéine, plus préférablement dans lequel le partenaire de couplage comprend en outre une entité de charge chimique ou biochimique.

3. Procédé de production d'un bioconjugué de protéine modifiée de manière chimiosélective dans lequel la protéine est sélectivement modifiée en deux sites ou plus, le procédé comprenant les étapes consistant à :
a.) fournir une protéine exprimée de manière recombinante comprenant un ou plusieurs résidus d'acide aminé non-canonique (ncAA) sur des sites spécifiques de la protéine, dans lequel un des résidus ncAA est un 5-hydroxytryptophane (5-HTP) et dans lequel un ou plusieurs des résidus ncAA sont un résidu ncAA d'azide ;
b.) oxyder de manière sélective le résidu d'acide aminé 5-HTP de la protéine dans des conditions adaptées et à basse tension, dans lequel la tension est suffisante pour générer un intermédiaire 5-HTP électrophile ;
c.) faire réagir l'intermédiaire 5-HTP avec un partenaire de couplage comprenant un groupe nucléophile dans des conditions adaptées pour le couplage de l'amine aromatique aux résidus 5-HTP dans la protéine ; et
d.) coupler sélectivement le résidu ncAA d'azide avec un partenaire de couplage dans des conditions adaptées pour le couplage du partenaire de couplage au résidu ncAA d'azide,
fournissant ainsi un bioconjugué de protéine modifiée de manière chimiosélective, dans lequel la protéine est modifiée sélectivement en deux sites.

4. Procédé selon l'une quelconque des revendications 1 à 3, dans lequel les un ou plusieurs résidus ncAA sont exposés sur la surface de la protéine.

5. Procédé selon l'une quelconque des revendications 1 à 3, dans lequel la protéine est un anticorps ou un fragment d'anticorps.

6. Procédé selon l'une quelconque des revendications 1 à 3, dans lequel la tension d'oxydation est comprise entre environ 0,4 volt et environ 1,0 volt, préférablement dans lequel la tension d'oxydation est comprise entre environ 0,4 volt et environ 0,8 volt, plus préférablement dans lequel la tension d'oxydation est comprise entre environ 0,7 volt et 0,8 volt.

7. Procédé selon l'une quelconque des revendications 1 à 3, dans lequel l'étape d'oxydation b) est facilitée par l'ajout d'un médiateur de transfert d'électrons, de préférence dans lequel le médiateur de transfert d'électrons est un 2,2,6,6-tétraméthylpipéridine-1-oxyle (TEMPO).

8. Procédé selon l'une quelconque des revendications 1 à 3, dans lequel le groupe nucléophile de l'étape c) est sélectionné dans le groupe constitué d'amines aromatiques, d'anilines, d'indoles, et de thiazoles.

9. Procédé selon l'une quelconque des revendications 1 à 3, dans lequel le groupe nucléophile de l'étape c) comprend en outre un élément biochimique d'une entité de charge chimique, préférablement dans lequel l'entité de charge est sélectionnée dans le groupe constitué d'un ou de plusieurs des éléments suivants : petite molécule, médicament thérapeutique, marqueur détectable, sonde détectable, polymère tel que le PEG, peptide, ou un acide nucléique et, plus préférablement, dans lequel l'entité de charge comprend des moyens pour immobiliser le bioconjugué sur un support solide.

10. Procédé de marquage électrochimique d'une protéine, comprenant les étapes consistant à :
a.) fournir une protéine exprimée de manière recombinante comprenant un ou plusieurs résidus d'acide aminé non canonique (ncAA) sur des sites spécifiques de la protéine, dans lequel un ou plusieurs des résidus ncAA sont un 5-hydroxytryptophane (5-HTP) ;
b.) oxyder sélectivement les un ou plusieurs résidus d'acide aminé 5-HTP de la protéine dans des conditions adaptées et à basse tension, dans lequel la tension est suffisante pour générer un intermédiaire 5- HTP électrophile ; et
c.) faire réagir l'intermédiaire 5-HTP avec un partenaire de couplage comprenant un groupe nucléophile avec une entité de charge marquée de manière détectable, dans des conditions adaptées pour le couplage de l'amine aromatique aux un ou plusieurs résidus 5-HTP dans la protéine,
produisant ainsi une protéine marquée.

11. Procédé selon la revendication 10, dans lequel les un ou plusieurs résidus ncAA sont exposés sur la surface de la protéine, préférablement dans lequel la protéine est un anticorps ou un fragment d'anticorps.

12. Procédé selon la revendication 11, dans lequel la tension d'oxydation est comprise entre environ 0,4 volt et environ 1,0 volt, préférablement dans lequel la tension d'oxydation est comprise entre environ 0,4 volt et environ 0,8 volt, plus préférablement dans lequel la tension d'oxydation est comprise entre environ 0,7 et environ 0,8 volt.

13. Procédé selon la revendication 12, dans lequel l'étape d'oxydation comprend l'ajout d'un médiateur de transfert d'électron, préférablement dans lequel le médiateur de transfert d'électron est un 2,2,6,6-tétraméthylpipéridine-1-oxyle (TEMPO).

14. Procédé selon la revendication 13, dans lequel le groupe nucléophile est sélectionné dans le groupe constitué d'amines aromatiques, d'anilines, d'indoles et de thiazoles.

15. Procédés selon l'une quelconque des revendications 10 à 14, dans lesquels la protéine est sélectivement modifiée en deux sites, et les un ou plusieurs autres résidus d'acide aminé non-canonique sont un résidu ncAA d'azide, de préférence dans lequel le procédé comprend en outre le couplage du résidu ncAA d'azide avec un partenaire de couplage dans des conditions adaptées pour le couplage du partenaire de couplage au ncAA d'azide, produisant ainsi un bioconjugué de protéine modifiée de manière chimiosélective, modifiée en deux sites spécifiques de la protéine, plus préférablement dans lesquels le partenaire de couplage comprend en outre une entité de charge chimique ou biochimique.
